# EUROPEAN PATENT SPECIFICATION

(11) **EP 2 419 403 B1**
(45) Date of publication and mention of the grant of the patent: **07.09.2016**
(21) Application number: 10714143.4
(22) Date of filing: 12.04.2010
(51) Int. Cl.: C07C 401/00, A61K 31/59, A61P 7/00, A61P 9/00

(54) **VITAMIN D RECEPTOR AGONISTS AND USES THEREOF**
AGONISTEN DES VITAMIN-D-REZEPTORS UND ANWENDUNGEN DAVON
AGONISTES DU RÉCEPTEUR DE LA VITAMINE D ET UTILISATIONS CORRESPONDANTES

(30) Priority: 17.04.2009 US 170160 P
(43) Date of publication of application: 22.02.2012
(73) Proprietor: Vidasym, Inc., Libertyville, IL 60048 (US)
(72) Inventor: KAWAI, Megumi, Libertyville Illinois 60048-3344 (US)
(74) Representative: Clegg, Richard Ian
(86) International application number: PCT/US2010/030777
(87) International publication number: WO 2010/120698

(56) References cited:
- EP-A1- 1 559 708
- WO-A2-2009/067578
- US-A1- 2005 004 085
- SHIMIZU M ET AL: "Synthesis and biological activities of new 1alpha,25-dihydroxy-19-nor vitamin D3 analogs with modifications in both the A-ring and the side chain" BIOORGANIC & MEDICINAL CHEMISTRY, PERGAMON, GB LNKD- DOI:10.1016/J.BMC.2006.01.061, vol. 14, no. 12, 15 June 2006 (2006-06-15) , pages 4277-4294, XP025133326 ISSN: 0968-0896 [retrieved on 2006-06-15]
- MIKAMI K ET AL: "BINOL-TI-CATALYZED CARBONYL-ENE CYCLIZATION BY TUNING THE 6-BR-LIGAND FOR THE SYNTHESIS OF 2-METHYL-19-NOR-22-OXA VITAMIN D ANALOGUE WITH SIGNIFICANT DIFFERENTIATION ACTIVITY" SYNLETT, GEORG THIEME VERLAG, DE LNKD- DOI:10.1055/S-1999-2989, no. 12, 1 January 1999 (1999-01-01), pages 1899-1902, XP001002275 ISSN: 0936-5214
- MAKOTO NAKABAYASHI ET AL: "Crystal Structures of Rat Vitamin D Receptor Bound to Adamantyl Vitamin D Analogs: Structural Basis for Vitamin D Receptor Antagonism and Partial Agonism" JOURNAL OF MEDICINAL CHEMISTRY, AMERICAN CHEMICAL SOCIETY, WASHINGTON, US LNKD- DOI:10.1021/JM8004477, vol. 51, 1 January 2008 (2008-01-01), pages 5320-5329, XP007914913 ISSN: 0022-2623 [retrieved on 2008-08-19]
- BURY Y ET AL: "Molecular evaluation of vitamin D3 receptor agonists designed for topical treatment of skin diseases" JOURNAL OF INVESTIGATIVE DERMATOLOGY, NATURE PUBLISHING GROUP, GB LNKD- DOI:10.1046/J.1523-1747.2001.01332.X, vol. 116, no. 5, 1 May 2001 (2001-05-01), pages 785-792, XP002526323 ISSN: 0022-202X

## Description

Vitamin D₃ is made from 7-dehydrocholesterol in the skin after exposure to ultraviolet light, modified by vitamin D₃-25-hydroxylase in the liver to form 25(OH)D₃, and then by 25-hydroxyvitamin D₃-1α-hydroxylase in the kidney to form the active hormone, 1,25-dihydroxyvitamin D₃ (calcitriol). Calcitriol functions by activating the vitamin D receptor ("VDR"), a nuclear receptor. The activated VDR recruits cofactors to form a complex that binds to vitamin D response elements in the promoter region of target genes to regulate gene transcription. One of the target genes for VDR is CYP24A1, the gene that encodes 25-hydroxyvitamin D-24-hydroxylase, which is an enzyme responsible for the further metabolism of calcitriol (Meyer et al., 2007, J Biol Chem, 282: 22344-22352). In addition, calcitriol is known to induce VDR expression (Solvsten et al., 1997, Arch Dermatol Res, 289: 367-372; Hulla et al., 1995, Int J Cancer, 62: 711-716).

Calcitriol (the endogenous VDR agonist) and certain analogs thereof have been considered for treating various diseases, such as hypocalcemia and secondary hyperparathyroidism associated with chronic kidney disease (CKD), osteoporosis, proteinuria, viral infection, baceterial infection, musculoskeletal disorders, and psoriasis.

However, there is a desire for new vitamin D receptor agonists for targeting physiological systems, such as the cardiovascular system, the immune system, the renal system, or the skeletal system, and treating diseases associated therewith. Further examples of Vitamin D analogues proposed as VDR activators are provided in the following: A-ring modifications (Mikami et al., 1999, Synlett, 12, 1899-1902; Hector F. DeLuca et al., US2005/0004085 A1), side chain modifications (Bury et. al., 2001, J. Investig. Derm, 116, 785-792), and both A-ring and side chain modifications (Nakabayashi et. al., 2008, J. Med. Chem, 51, 5320-5329; Shimizu et al., 2006, Bioorganic & Medicinal Chem, 14, 4277-4294; Shimizu M. 2005, EP 1 559 708 A1; Von Geldern et. al., WO2009/067578 A2).

The invention provides compounds that activate the VDR and are useful for treating disorders that are affected by modulating the VDR. More particularly, the invention provides compounds with potencies targeting endothelial dysfunction, left ventricular hypertrophy and proteinuria, which can be useful for the treatment of cardiovascular complications and disease progression in the CKD patient population.

The invention provides a compound of Formula (I) as defined in the accompanying claim 1 and pharmaceutically acceptable salts thereof. Thus, the invention provides a compound of Formula (I) 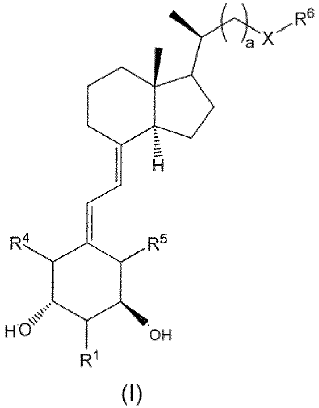 wherein
R¹ is =CH₂; or
R¹ and the carbon to which it is bonded together form a cyclopropyl group;
R⁴ and R⁵ are the same or different and each is selected from the group consisting of H, optionally substituted C₁₋₁₂ alkyl, hydroxyl, optionally substituted C₁₋₁₂ alkoxy, and halo;
R⁶ is optionally substituted C₁₋₁₂ alkyl or optionally substituted aryl;
X is oxygen or sulfur; and
a is 0-5 (i.e., 0, 1, 2, 3, 4, or 5);
provided that when a is 1-5, then R⁶ is optionally substituted aryl; and
when a is 0, R⁶ is optionally substituted C₁₋₁₂ alkyl;
or a pharmaceutically acceptable salt thereof. The optional substituents for a C₁₋₁₂ alkyl, a C₁₋₁₂ alkoxy and an aryl group are shown below.

In addition, the invention provides a pharmaceutical composition comprising (i) a compound or a pharmaceutically acceptable salt thereof of Formula (I) and (ii) a pharmaceutically acceptable carrier.

The invention further provides a compound or salt of Formula (I) for use in a method of treating a disease which benefits from a modulation of the vitamin D receptor.

### BRIEF DESCRIPTION OF THE SEVERAL VIEWS OF THE DRAWING(S)

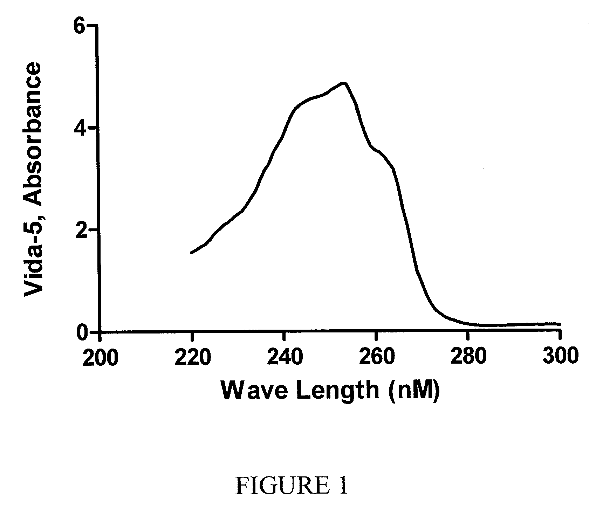

Figure 1 is a graph illustrating the absorbance profile for Vida-5. The maximal absorbance wavelength (ODₘₐₓ) for Vida-5 is at 253 nm and the the extinction coefficient is 48360.

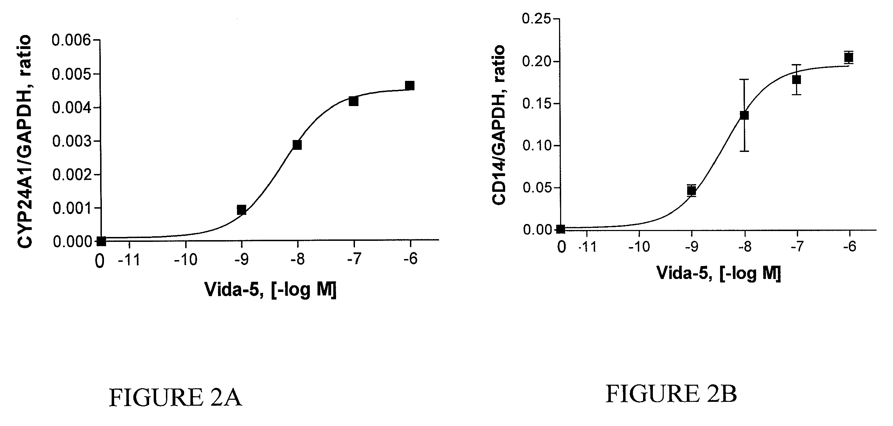

Figures 2A and 2B are graphs illustrating that the effects of Vida-5 on inducing the expression of CYP24A1 (Fig. 2A) and CD14 (Fig. 2B) in the HL-60 promyelocytic leukemia cells. The EC₅₀ (the half maximal effective concentration) of Vida-5 for CYP24A1 and CD 14 are 5.6 and 4.1 nM, respectively.

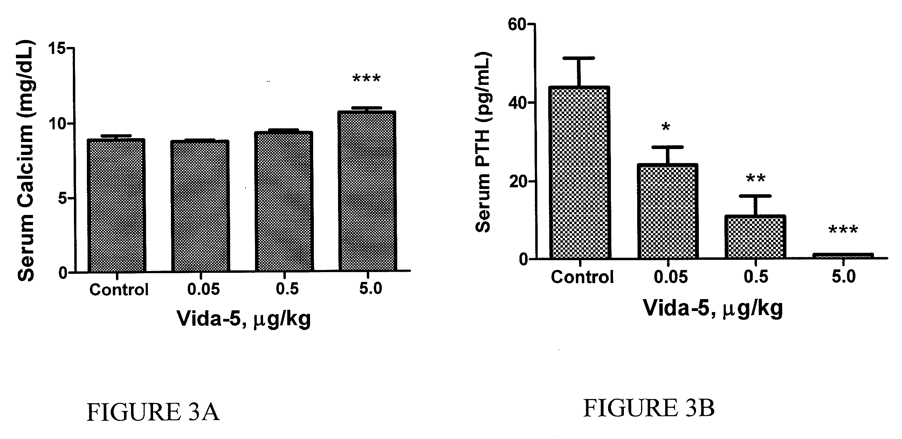

Figures 3A and 3B are graphs illustrating the effects of Vida-5 on serum Ca (Fig. 3A) and serum PTH (Fig. 3B) in normal mice. *p<0.05, **p<0.01, ***p<0.001 vs. Control (no drug).

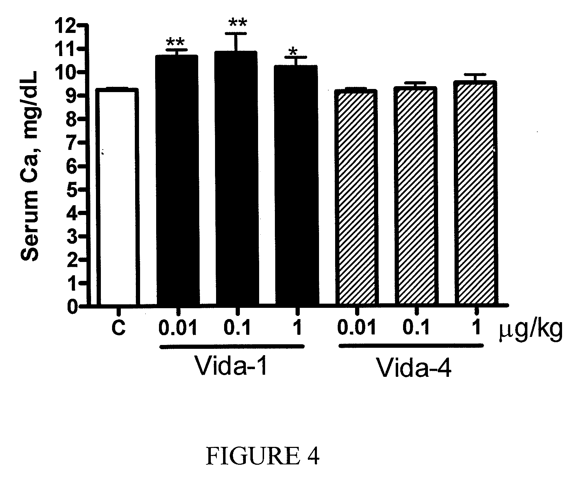

Figure 4 is a graph illustrating the effects of Vida-1 versus Vida-4 on serum calcium in normal mice. *p<0.05, **p<0.01 vs. Control (no drug). White bar: control; black bar: Vida-1; and striped bar: Vida-4.

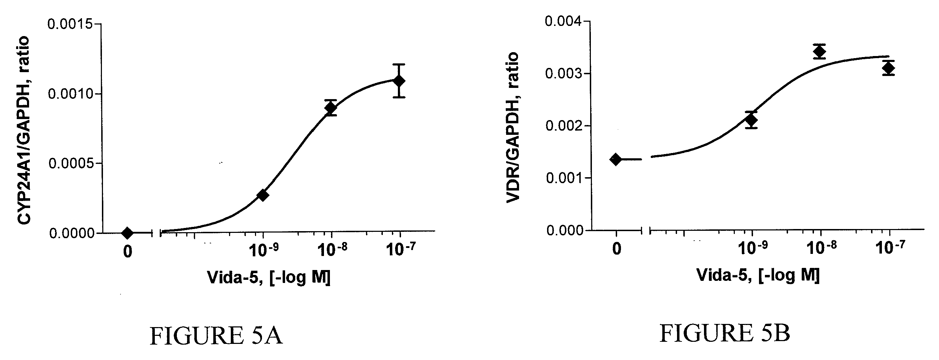

Figure 5A and 5B are graphs illustrating the effects of Vida-5 on inducing CYP24A1 and VDR expression in primary culture of human coronary artery smooth muscle cells. The EC₅₀ of Vida-5 for CYP24A1 and VDR are 2.9 and 1.3 nM, respectively.

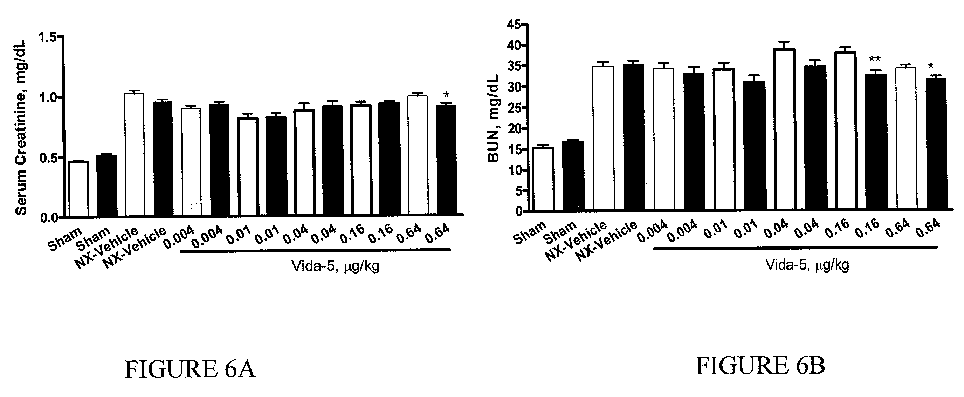

Figures 6A and 6B are graphs illustrating the effects of Vida-5 on serum creatinine (Fig. 6A) and serum BUN (Fig. 6B) in the 5/6 nephrectomized uremic rats. White bar: week 6 after surgery, before treatment. Black bar: week 8 after surgery, after drug treatment. *p<0.05, **p<0.01 vs. same group before treatment.

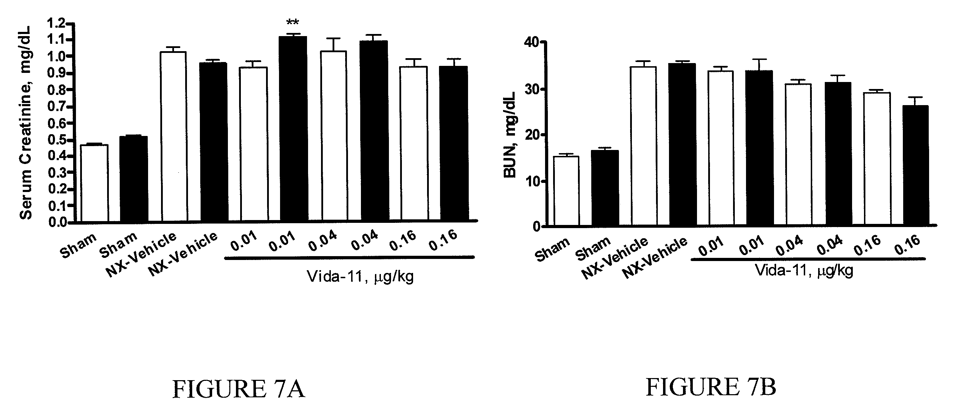

Figures 7A and 7B are graphs illustrating the effects of Vida-11 on serum creatinine (Fig. 7A) and serum BUN (Fig. 7B) in the 5/6 nephrectomized uremic rats. White bar: week 6 after surgery, before treatment. Black bar: week 8 after surgery, after drug treatment. **p<0.01 vs. same group before treatment.

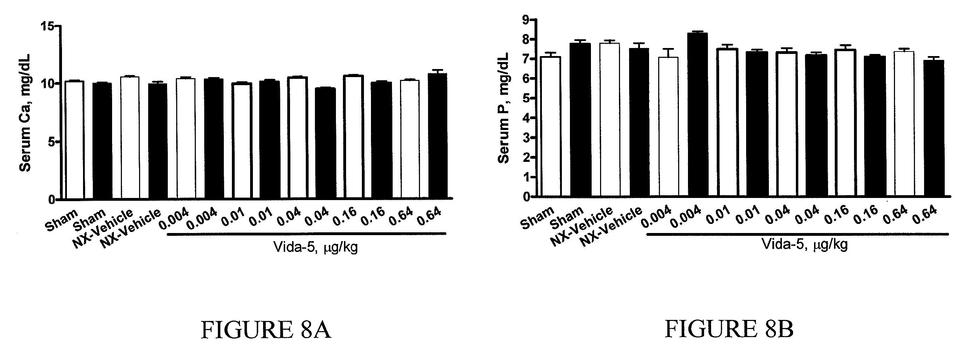

Figures 8A and 8B are graphs illustrating the effects of Vida-5 on serum calcium (Fig. 8A) and serum phosphorus (Fig. 8B) in the 5/6 nephrectomized uremic rats. White bar: week 6 after surgery, before treatment. Black bar: week 8 after surgery, after drug treatment.

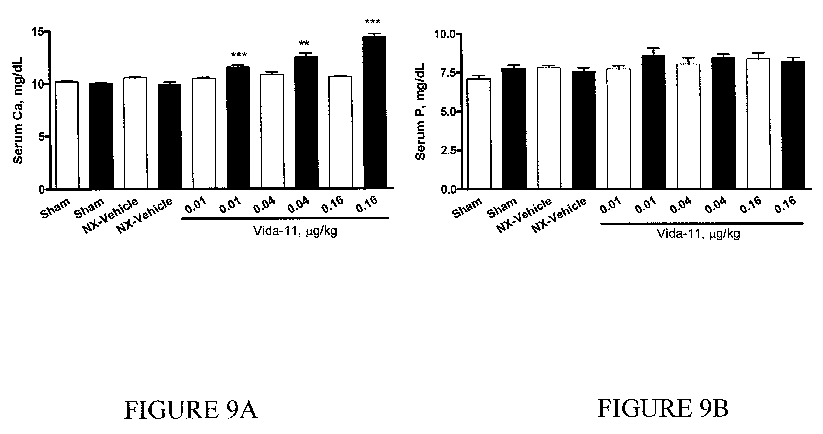

Figures 9A and 9B are graphs illustrating the effects of Vida-11 on serum calcium (Fig. 9A) and serum phosphorus (Fig. 9B) in the 5/6 nephrectomized uremic rats. White bar: week 6 after surgery, before treatment. Black bar: week 8 after surgery, after drug treatment. **p<0.01, ***p<0.001 vs. same group before treatment.

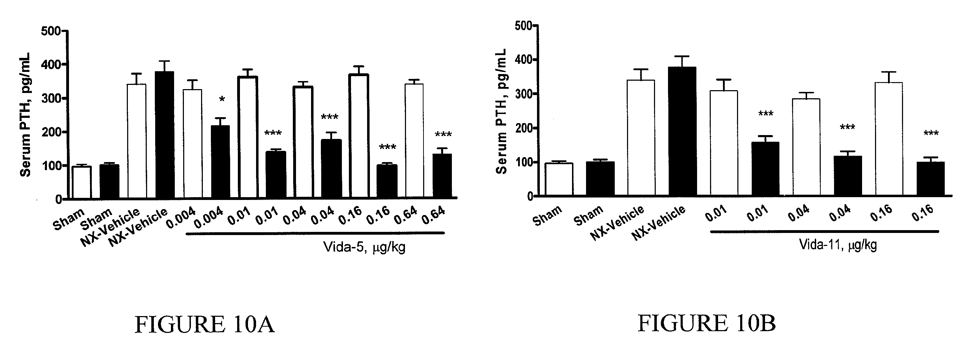

Figures 10A and 10B are graphs illustrating the effects of Vida-5 (Fig. 10A) and Vida-11 (Fig. 10B) on serum PTH in the 5/6 nephrectomized uremic rats. White bar: week 6 after surgery, before treatment. Black bar: week 8 after surgery, after drug treatment. *p<0.05, ***p<0.001 vs. same group before treatment.

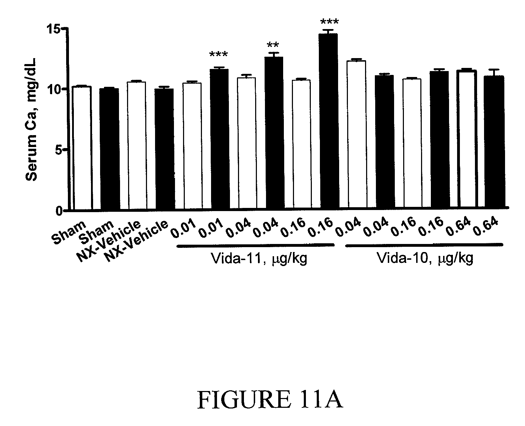

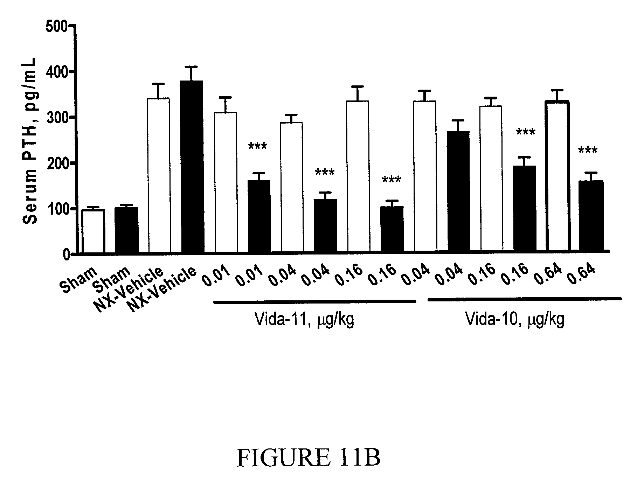

Figures 11A and 11B are graphs illustrating that one change at the C-2 position (a methylene group at C-2 for Vida-11 versus a cyclopropane group at C-2 for Vida-10) alters the effects of Vida-10 and Vida-11 on serum Ca (Fig. 11A) and PTH (Fig. 11B) in the 5/6 nephrectomized uremic rats. White bar: week 6 after surgery, before treatment. Black bar: week 8 after surgery, after drug treatment. **p<0.01, ***p<0.001 versus same group before treatment.

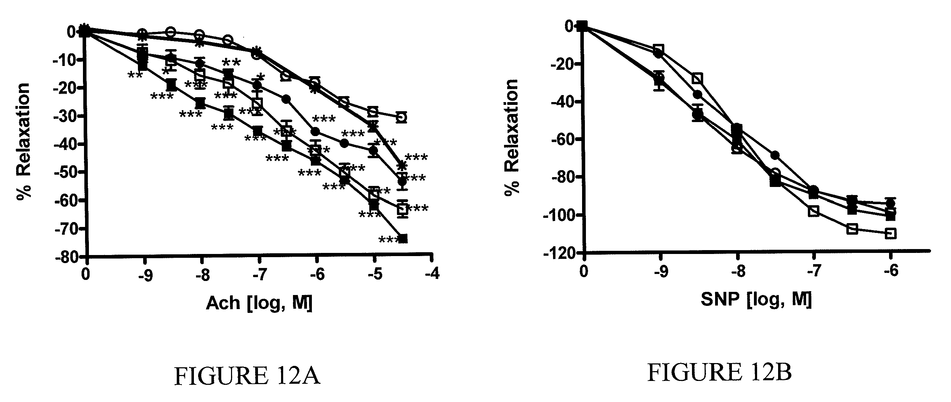

Figures 12A and 12B are graphs illustrating that Vida-5 improves endothelial-dependent acetylcholine (Ach)-induced aortic relaxation in a dose-dependent manner in the 5/6 nephrectomized uremic rats (Fig. 12A), but has no effect on endothelial-independent sodium nitroprusside (SNP)-induced relaxation (Fig. 12B). Sham: ■; 5/6NX-Vehicle: o; Vida-5 at 0.004 µg/kg: ◆; Vida-5 at 0.01 *g/kg: ●; Vida-5 at 0.16 µg/kg: □. *p<0.05, **p<0.01, ***p<0.001 vs. 5/6NX-Vehicle.

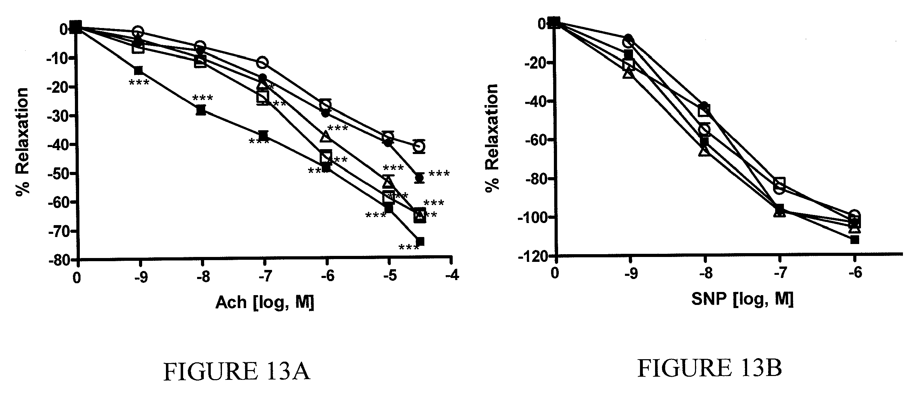

Figures 13A and 13B are graphs illustrating that Vida-11 improves endothelial-dependent acetylcholine (Ach)-induced aortic relaxation in a dose-dependent manner in the 5/6 nephrectomized uremic rats (Fig. 13A), and has no effect on sodium nitroprusside (SNP)-induced relaxation (Fig. 13B). Sham: ■; 5/6NX-Vehicle: o; Vida-11 at 0.01 µg/kg: ●; Vida-11 at 0.04 µg/kg: △; Vida-11 at 0.16 µg/kg: □. *p<0.05, **p<0.001 vs. 5/6NX-Vehicle.

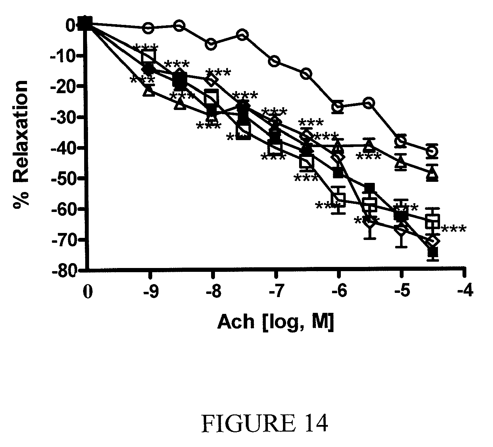

Figure 14 is a graph illustrating that Vida-10 improves endothelial function in the 5/6 nephrectomized uremic rats. Sham: ■; 5/6NX-Vehicle: o; Vida-10 at 0.04 [µg/kg: △; Vida-10 at 0.16 µg/kg: □; Vida-11 at 0.64 µg/kg: ◊. ***p<0.001 vs. 5/6NX-Vehicle at all Ach doses in the 3 treatment groups except the two points at 4.5 and 5 Ach [log, M] in the 0.04 µg/kg group

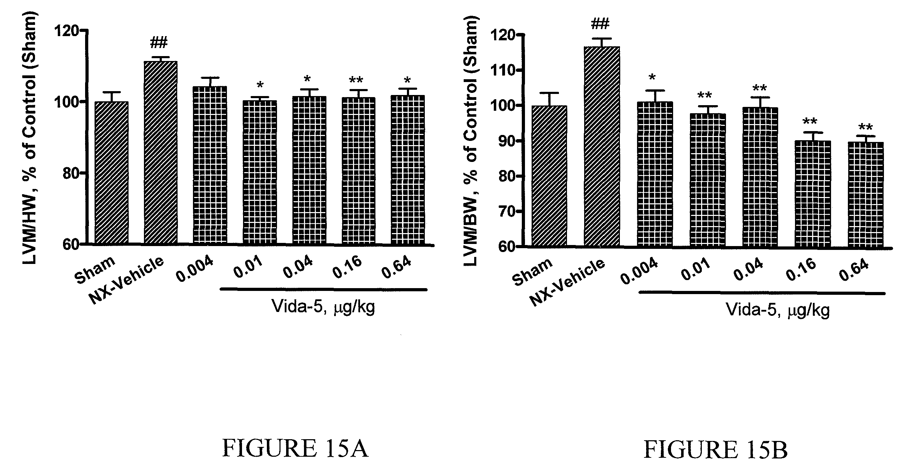

Figures 15A and 15B are graphs illustrating that Vida-5 significantly reduces the LVW/BW (Fig. 15A) and LVW/HW (Fig. 15B) ratios in a dose-dependent manner in the 5/6 nephrectomized uremic rats after 2 weeks of treatment. ^{##}p<0.01 vs. Sham; *p<0.05, **p<0.01 vs. 5/6NX-Vehicle. Striped bar: control; checkered bar: Vida-5.

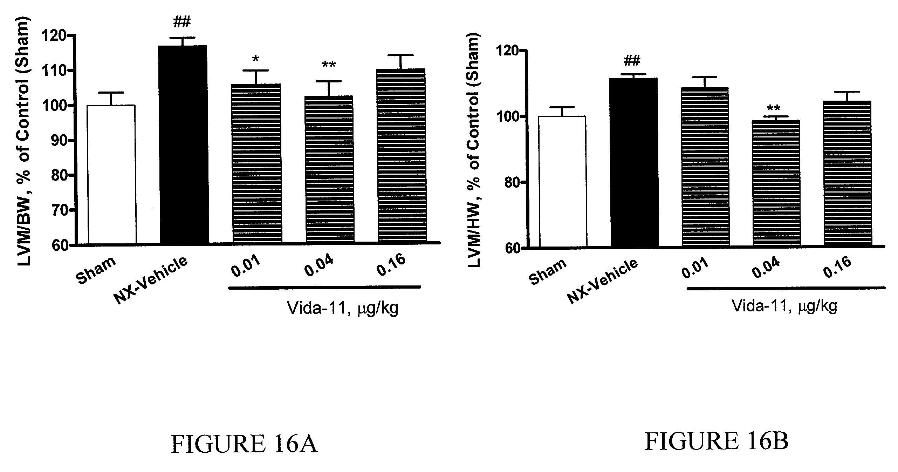

Figures 16A and 16B are graphs illustrating that the effects of Vida-11 on the LVW/BW (Fig. 16A) and LVW/HW (Fig. 16B) ratios in the 5/6 nephrectomized uremic rats after 2 weeks of treatment. ^{##}p<0.01 vs. Sham; *p<0.05, **p<0.01 vs. 5/6NX-Vehicle. White bar: sham; black bar: NX-vehicle; striped bar: Vida-11.

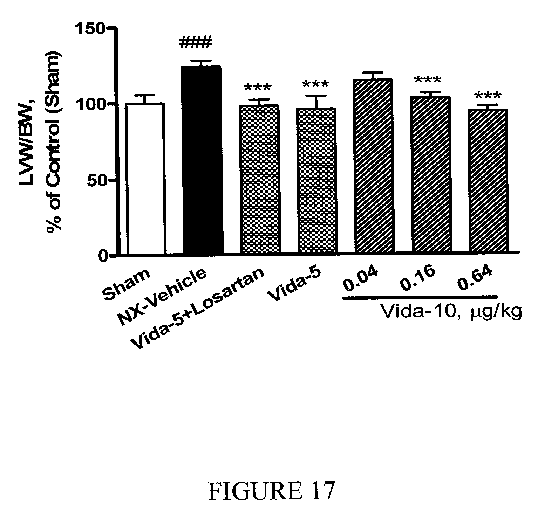

Figures 17 is a graph illustrating that the effects of Vida-5 at 0.04 µg/kg (3x/week, i.p. for 2 weeks) alone or in combination with losartan (0.025 mg/ml in drinking water for 2 weeks) and also Vida-10 at 0.04, 0.16 and 0.64 µg/kg on the LVW/BW in the 5/6 nephrectomized uremic rats. ^{###}p<0.001 vs. Sham; ***p<0.001 vs. Vehicle. White bar: sham; black bar: NX-vehicle; checkered bar: Vida-5 (either alone or with losartan); striped bar: Vida-10.

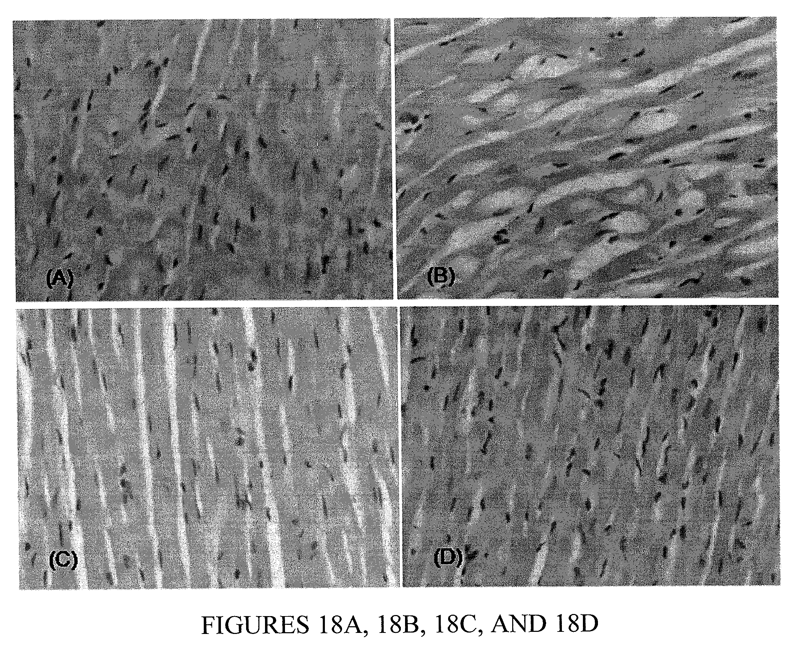

Figures 18A-D are pictures illustrating the cardiomyocytes morphology in the Sham rats (Fig. 18A), the 5/6 NX rats treated with vehicle (Fig. 18B) or with Vida-5 at 0.01 µg/kg (Fig. 18C) or with Vida-5 at 0.16 µg/kg, 3x/week, i.p. for 2 weeks (Fig. 18D).

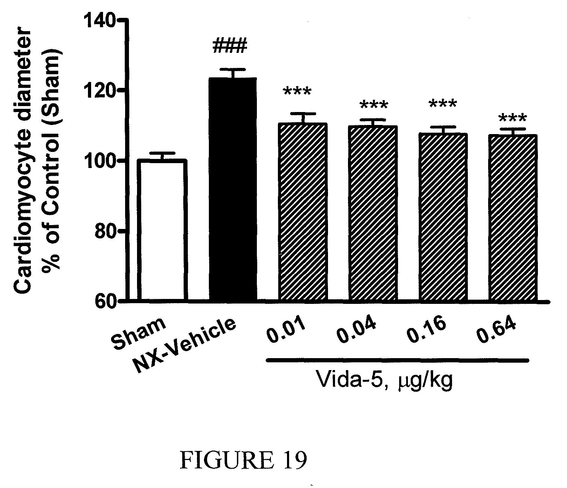

Figure 19 is a graph illustrating that Vida-5 at 0.01, 0.04, 0.16 and 0.64 µg/kg decreases cardiomyocytes diameter in the 5/6 nephrectomized uremic rats after 2 weeks of treatment. ^{###}p<0.001 vs. Sham; ***p<0.001 vs. Vehicle. White bar: sham; black bar: NX-vehicle; striped bar: Vida-5.

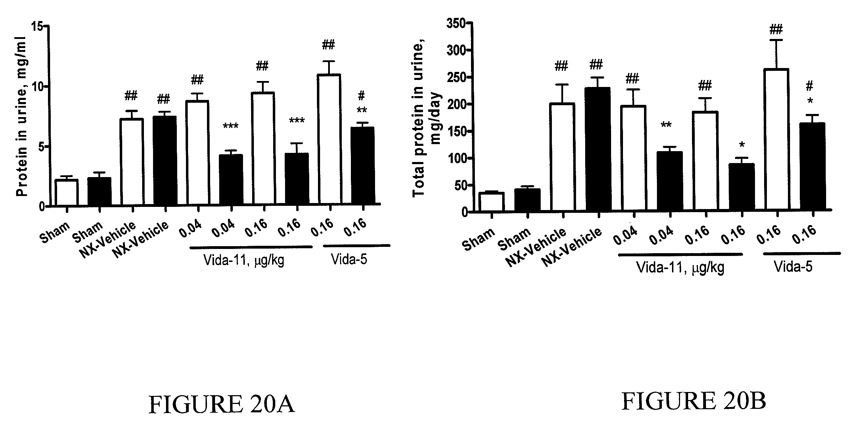

Figures 20A and 20B are graphs illustrating that the effects of Vida-11 at 0.04 and 0.16 µg/kg and Vida-5 at 0.16 µg/kg (3x/week, i.p. for 2 weeks) reduce proteinuria as measured by protein concentration (mg/ml, Fig. 20A) or total protein per day (Fig. 20B) in the 5/6 nephrectomized uremic rats. White bar: week 6 after surgery, before treatment. Black bar: week 8 after surgery, after drug treatment. ^{#}p<0.05, ^{##}p<0.01 vs. Sham-Week 6 (before treatment); *p<0.05, **p<0.01, ***p<0.001 vs. own group before treatment.

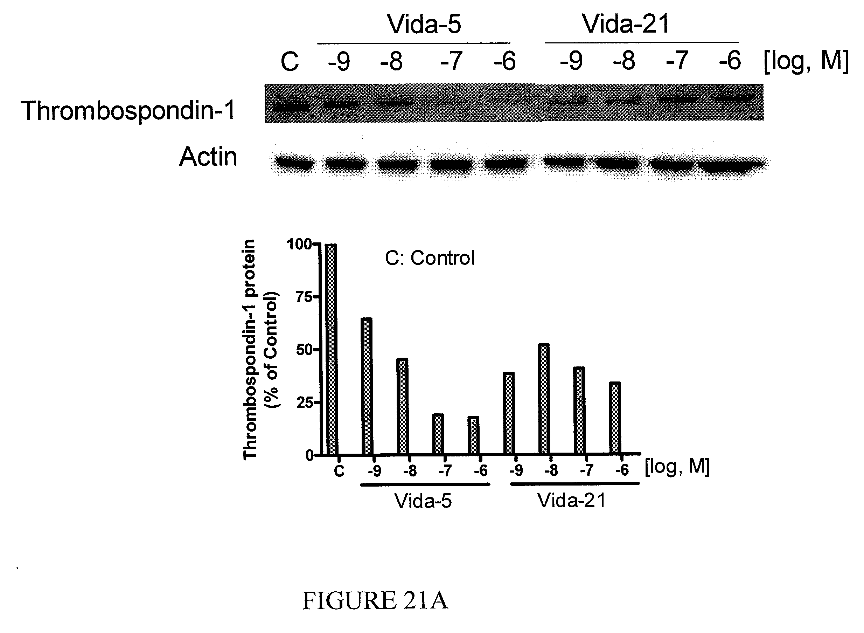

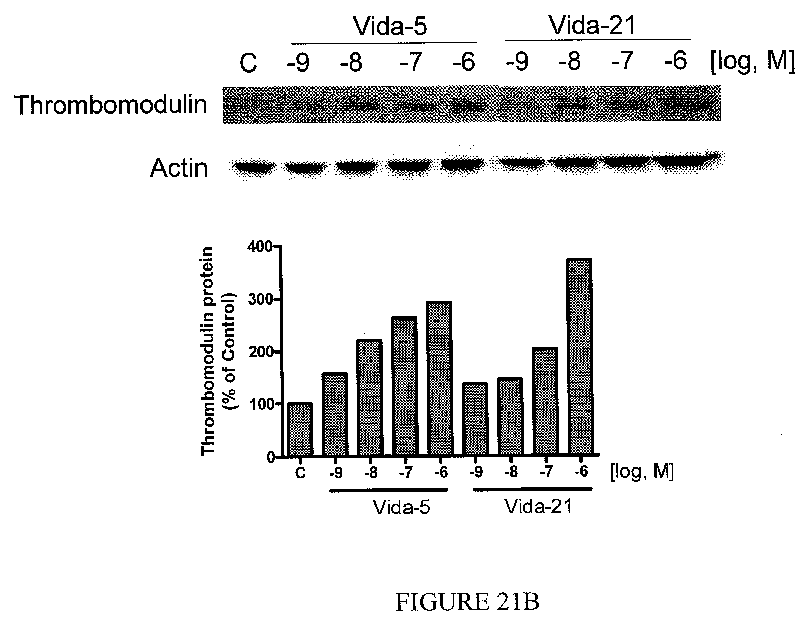

Figures 21A and 21B are graphs illustrating that the effects of Vida-5 and Vida-21 suppress the expression of thrombospondin-1 protein (Fig. 21A) but induce the expression of thrombomodulin protein (Fig. 21B) in primary culture of human coronary artery smooth muscle cells. C: Control (no drug).

### DETAILED DESCRIPTION OF THE INVENTION

In one aspect, the invention concerns a compound of the following formula: 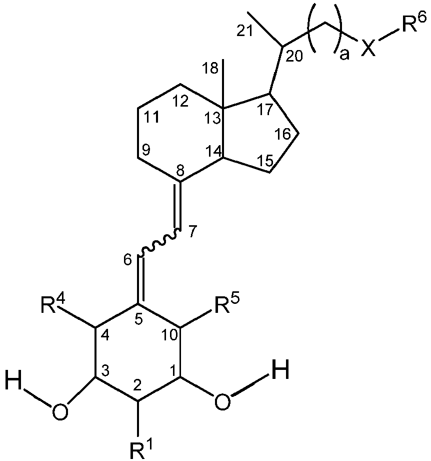 More specifically the invention provides a compound of the following Formula (I): 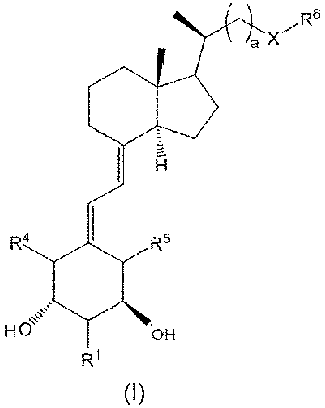 wherein
R¹ is =CH₂; or R¹ and the carbon to which it is bonded together form a cyclopropyl group; R⁴ and R⁵ are the same or different and each is selected from the group consisting of H, optionally substituted C₁₋₁₂ alkyl, hydroxyl, optionally substituted C₁₋₁₂ alkoxy, and halo; R⁶ is optionally substituted C₁₋₁₂ alkyl or optionally substituted aryl; X is oxygen or sulfur; and a is 0-5 (e.g., 0, 1, 2, 3, 4, or 5); when a is 0, R⁶ is optionally substituted C₁₋₁₂ alkyl; or a pharmaceutically acceptable salt thereof. The optional substituents for a C₁₋₁₂ alkyl, a C₁₋₁₂ alkoxy and an aryl group are shown below.

Compounds of Formula (I) can contain carbon-carbon double bonds and/or carbon-nitrogen double bonds in the Z or E configuration (e.g., between the 5 and 6 carbons and between the 7 and 8 carbons), in which the "Z" represents a compound having two higher priority substitutions on the same side of a carbon-carbon double bond or carbon-nitrogen double bond, whereas the term "*E*" represents a compound having two higher priority substitutions on the opposite side of a carbon-carbon double bond or carbon-nitrogen double bond. A compound or salt therof of Formula (I) can also be a mixture of "Z" and "*E*" isomers.

A compound or salt therof of Formula (I) can also exist as a tautomer or an equilibrium mixture thereof, wherein a proton of a compound shifts from one atom to another. Examples of tautomers include, but are not limited to, keto-enol, phenol-keto, oxime-nitroso, nitro-aci, and imine-enamine.

As used herein, unless otherwise specified, the term "alkyl" means a saturated straight chain or branched non-cyclic hydrocarbyl group having an indicated number of carbon atoms (e.g., C₁-C₁₂, C₁-C₈, C₁-C₄, etc.). An alkyl group can be unsubstituted or substituted. Examples of saturated straight chain unsubstituted alkyls include methyl, ethyl, *n*-propyl, *n*-butyl, *n*-pentyl, *n*-hexyl, *n*-heptyl, *n*-octyl, *n*-nonyl and *n*-decyl; while examples of saturated branched alkyls include isopropyl, *sec*-butyl, isobutyl, *tert*-butyl, isopentyl, 2-methylbutyl, 3-methylbutyl, 2-methylpentyl, 3-methylpentyl, 4-methylpentyl, 2-methylhexyl, 3-methylhexyl, 4-methylhexyl, 5-methylhexyl, 2,3-dimethylbutyl, 2,3-dimethylpentyl, 2,4-dimethylpentyl, 2,3-dimethylhexyl, 2,4-dimethylhexyl, 2,5-dimethylhexyl, 2,2-dimethylpentyl, 2,2-dimethylhexyl, 3,3-dimethylpentyl, 3,3-dimethylhexyl, 4,4-dimethylhexyl, 2-ethylpentyl, 3-ethylpentyl, 2-ethylhexyl, 3-ethylhexyl, 4-ethylhexyl, 2-methyl-2-ethylpentyl, 2-methyl-3-ethylpentyl, 2-methyl-4-ethylpentyl, 2-methyl-2-ethylhexyl, 2-methyl-3-ethylhexyl, 2-methyl-4-ethylhexyl, 2,2-diethylpentyl, 3,3-diethylhexyl, 2,2-diethylhexyl, 3,3-diethylhexyl and the like.

The term "cycloalkyl," as used herein, means a cyclic alkyl moiety containing from, for example, 3 to 7 carbon atoms (e.g., 3, 4, 5, 6, or 7 carbon atoms). Examples of such moieties include cyclopropyl, cyclobutyl, cyclopentyl, cyclohexyl, and cycloheptyl.

As used herein, unless otherwise specified, the term "alkoxy" means -O-(alkyl), wherein alkyl is defined above.

As used herein, unless otherwise specified, the term "halo" means fluoro, chloro, bromo, or iodo.

The term "aryl" refers to an unsubstituted or substituted aromatic carbocyclic moiety, as commonly understood in the art, and includes monocyclic and polycyclic aromatics which may be fused or unfused, such as, for example, phenyl, biphenyl, naphthyl, anthracenyl, pyrenyl, and the like. An aryl moiety generally contains from, for example, 6 to 30 carbon atoms, preferably from 6 to 18 carbon atoms, more preferably from 6 to 14 carbon atoms and most preferably from 6 to 10 carbon atoms. It is understood that the term aryl includes carbocyclic moieties that are planar and comprise 4n+2 π electrons, according to Hückel's Rule, wherein n = 1, 2, or 3. When substituted, the substituents can be at any available position (e.g., the 1-, 2-, 3-, 4-, 5-, or 6-position). If the rest of the molecule is at the 1-position, then the other substituents preferably are the 3-, 4-, and/or 5-positions.

As used herein, unless otherwise specified, the term "substituted alkyl" refers to an alkyl group substituted by one or more substituents (e.g., 1 to 5, 1 to 4, 1 to 3, 1 or 2) selected from the group consisting of alkenyl, alkynyl, cycloalkyl, aroyl, halo, haloalkyl (mono-, di-, or trihaloalkyl, e.g., trifluoromethyl), haloalkoxy (mono-, di- or trihaloalkoxy, e.g., trifluoromethoxy), hydroxy, alkoxy, alkoxycarbonyl, cycloalkyloxy, heterocyclooxy, oxo (=O), alkanoyl, aryl, arylalkyl, alkylaryl, heteroaryl, heteroarylalkyl, alkylheteroaryl, heterocyclyl, aryloxy, alkanoyloxy, amino, alkylamino (mono-, di-, and trialkylamino), arylamino, arylalkylamino, cycloalkylamino, heterocycloamino, alkanoylamino, aroylamino, aralkanoylamino, mercapto, alkylthio, arylthio, arylalkylthio, cycloalkylthio, heterocyclothio, alkylthiono, arylthiono, arylalkylthiono, alkylsulfonyl, arylsulfonyl, arylalkylsulfonyl, sulfamato (NH₂SO₂O-), alkylsulfamato, sulfonamido (-SO₂NH₂), alkyl sulfonamido, nitro, cyano, carboxy, carbamyl (NH₂CO-), and substituted carbamyl (alkyl-NH-CO, aryl-NH-CO, arylalkyl-NH-CO, or instances where there are two substituents on the nitrogen selected from alkyl or arylalkyl). In some embodiments, C₁₋₁₂ alkyl is optionally substituted with hydroxyl, and/or sulfamato (NH₂SO₂O-).

As used herein, unless otherwise specified, the term "substituted alkoxy" refers to an alkoxy group substituted by one or more substituents (e.g., 1 to 5, 1 to 4, 1 to 3, 1 or 2) selected from the group consisting of alkyl, alkenyl, alkynyl, cycloalkyl, aroyl, halo, haloalkyl (mono-, di-, or trihaloalkyl, e.g., trifluoromethyl), haloalkoxy (mono-, di- or trihaloalkoxy, e.g., trifluoromethoxy), hydroxy, alkoxycarbonyl, cycloalkyloxy, heterocyclooxy, oxo (=O), alkanoyl, aryl, arylalkyl, alkylaryl, heteroaryl, heteroarylalkyl, alkylheteroaryl, heterocyclyl, aryloxy, alkanoyloxy, amino, alkylamino (mono-, di-, and trialkylamino), arylamino, arylalkylamino, cycloalkylamino, heterocycloamino, alkanoylamino, aroylamino, aralkanoylamino, mercapto, alkylthio, arylthio, arylalkylthio, cycloalkylthio, heterocyclothio, alkylthiono, arylthiono, arylalkylthiono, alkylsulfonyl, arylsulfonyl, arylalkylsulfonyl, sulfamato (NH₂SO₂O-), alkylsulfamato, sulfonamido (-SO₂NH₂), alkyl sulfonamido, nitro, cyano, carboxy, carbamyl (NH₂CO-), and substituted carbamyl (alkyl-NH-CO, aryl-NH-CO, arylalkyl-NH-CO, or instances where there are two substituents on the nitrogen selected from alkyl or arylalkyl).

As used herein, unless otherwise specified, the term "substituted aryl" refers to an aryl group substituted by one or more substituents (e.g., 1 to 5, 1 to 4, 1 to 3, 1 or 2) selected from the group consisting of alkyl, alkenyl, alkynyl, cycloalkyl, aroyl, halo, haloalkyl (mono-, di-, or trihaloalkyl, e.g., trifluoromethyl), haloalkoxy (mono-, di- or trihaloalkoxy, e.g., trifluoromethoxy), hydroxy, alkoxy, alkoxycarbonyl, cycloalkyloxy, heterocyclooxy, oxo (=O), alkanoyl, arylalkyl, alkylaryl, heteroaryl, heteroarylalkyl, alkylheteroaryl, heterocyclyl, aryloxy, alkanoyloxy, amino, alkylamino (mono-, di-, and trialkylamino), arylamino, arylalkylamino, cycloalkylamino, heterocycloamino, alkanoylamino, aroylamino, aralkanoylamino, mercapto, alkylthio, arylthio, arylalkylthio, cycloalkylthio, heterocyclothio, alkylthiono, arylthiono, arylalkylthiono, alkylsulfonyl, arylsulfonyl, arylalkylsulfonyl, sulfamato (NH₂SO₂O-), alkylsulfamato, sulfonamido (-SO₂NH₂), alkyl sulfonamido, nitro, cyano, carboxy, carbamyl (NH₂CO-), and substituted carbamyl (alkyl-NH-CO, aryl-NH-CO, arylalkyl-NH-CO, or instances where there are two substituents on the nitrogen selected from alkyl or arylalkyl). In other embodiments, aryl is optionally substituted with alkyl and/or hydroxyl, as described herein.

Whenever a range of the number of atoms in a group or moiety is indicated (e.g., a C₁-C₁₂, C₁-C₈, C₁-C₄, or C₁-C₃ alkyl, haloalkyl, alkylamino, alkenyl, etc.), it is specifically contemplated that any sub-range or individual number of carbon atoms falling within the indicated range also can be used. Thus, for instance, the recitation of a range of 1-12 carbon atoms, 1-8 carbon atoms, 1-4 carbon atoms, 1-3 carbon atoms, or 2-8 carbon atoms as used with respect to any chemical group (e.g., alkyl, haloalkyl, alkylamino, alkenyl, etc.) referenced herein encompasses and specifically describes 1, 2, 3, 4, 5, 6, 7, 8, 9, 10, 11, or 12 carbon atoms, as appropriate, as well as any sub-range thereof (e.g., 1-2 carbon atoms, 1-3 carbon atoms, 1-4 carbon atoms, 1-5 carbon atoms, 1-6 carbon atoms, 1-8 carbon atoms, 1-10 carbon atoms, 1-12 carbon atoms, 2-3 carbon atoms, 2-4 carbon atoms, 2-5 carbon atoms, 2-8 carbon atoms, 2-12 carbon atoms, 3-4 carbon atoms, 3-5 carbon atoms, 3-8 carbon atoms, 3-10 carbon atoms, 3-12 carbon atoms, 4-5 carbon atoms, 4-6 carbon atoms, 4-8 carbon atoms, 4-12 carbon atoms, 5-6 carbon atoms, 5-8 carbon atoms, 5-12 carbon atoms, 6-8 carbon atoms, or 6-12 carbon atoms, etc., as appropriate).

Compounds of the invention can contain asymmetrically substituted carbon atoms in the R or S configuration, wherein the terms "*R*" and "*S*" are as defined in Pure Appl. Chem., 1976, 45: 11-30. Compounds with asymmetrically substituted carbon atoms with equal amounts of *R* and *S* configurations are racemic at those atoms. Atoms having excess of one configuration over the other are assigned the configuration in excess, preferably an excess of about 85-90%, more preferably an excess of about 95-99%, and still more preferably an excess greater than 99%. Accordingly, this invention is meant to embrace racemic mixtures and relatives and absolute diastereoisomers of the compounds thereof.

The invention encompasses all compounds described by Formula (I), and salts thereof, without limitation. However, for the purposes of further illustration, aspects and embodiments of the invention are discussed herein.

In an embodiment of the above, X is oxygen. In other embodiments, X is sulfur.

In an embodiment of the above, R¹ is =CH₂. In some instances of these embodiments (e.g., a is 0 and/or a is 1-5).

In other embodiments, R¹ and the carbon to which it is bonded together form cyclopropyl.

In any of the above embodiments, R⁴ and R³ are each H.

In an embodiment, a is 0 and R⁶ is a C₁₋₁₂ alkyl, or a substituted C₁₋₁₂ alkyl. Examples of suitable C₁₋₁₂ alkyl moieties for R⁶ include isopentyl, 2,3-dimethylbutyl, 3-ethylpentyl, and 4-ethylhexyl. Examples of suitable substituted C₁₋₁₂ alkyl include hydroxy C₁₋₁₂ alkyl moieties such as 3-hydroxy-3-methylbutyl, 2,3-dimethyl-3-hydroxybutyl, 3-ethyl-3-hydroxypentyl, and 4-ethyl-4-hydroxyhexyl.

In other embodiments, a is 1 to 5 (i.e., 1, 2, 3, 4, or 5) and R⁶ is aryl (e.g., phenyl), or substituted aryl. Preferably, R⁶ is an aryl substituted with a C₁₋₁₂ alkyl (e.g., methyl, ethyl, isopropyl) or a C₁₋₁₂ hydroxyalkyl (e.g., 2-hydroxypropan-2-yl). Preferably, a is 1.

The compound or phannaceutically acceptable salt of Formula (I) has the following structure

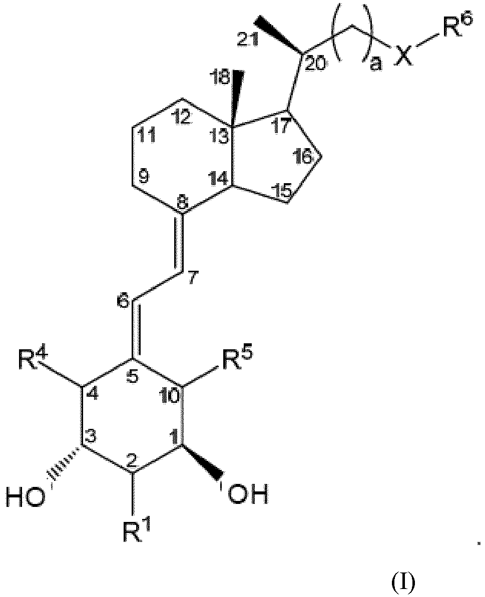

The compound or salt therof of Formula (I) has a structure selected from the group consisting of: 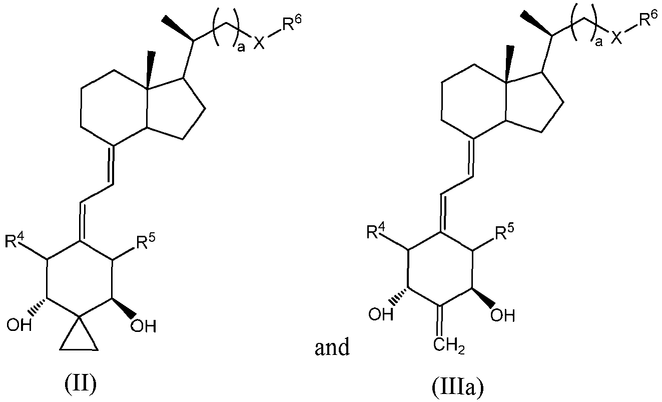 wherein for (II) and (IIIa), R⁴, R⁵, R⁶, X, and a are as defined above.

In a preferred embodiment, the compound or salt therof of Formula (I) is selected from the group consisting of: 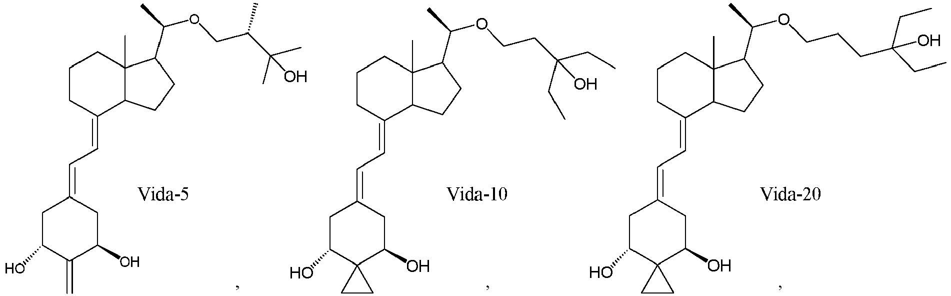 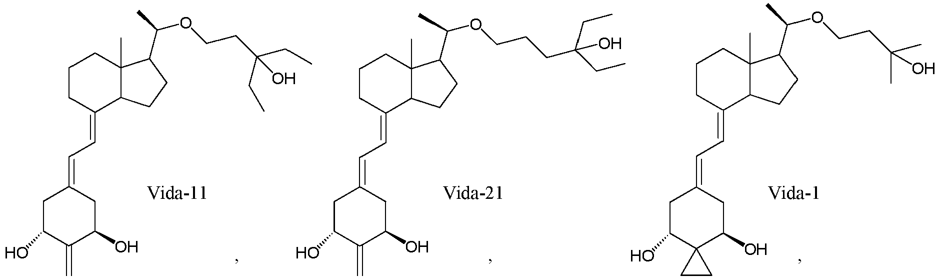 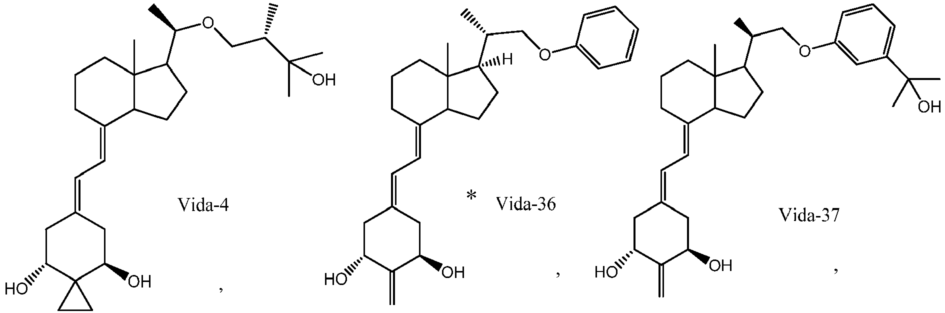 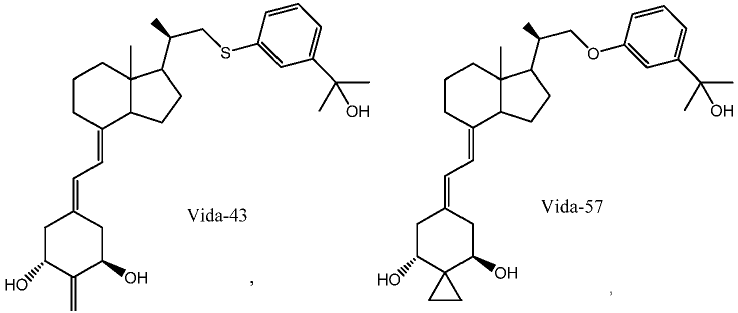 * Vida 36 is a Reference Example 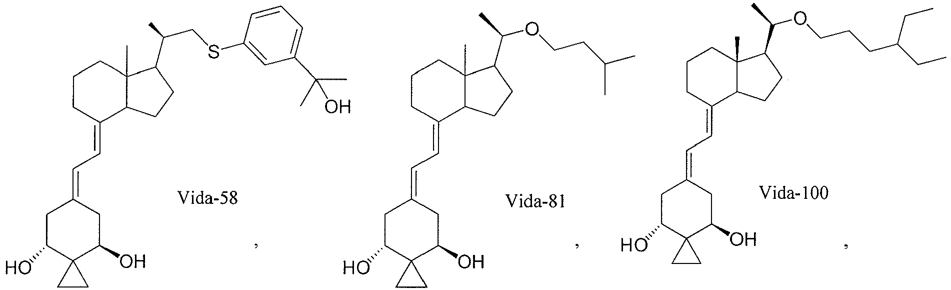 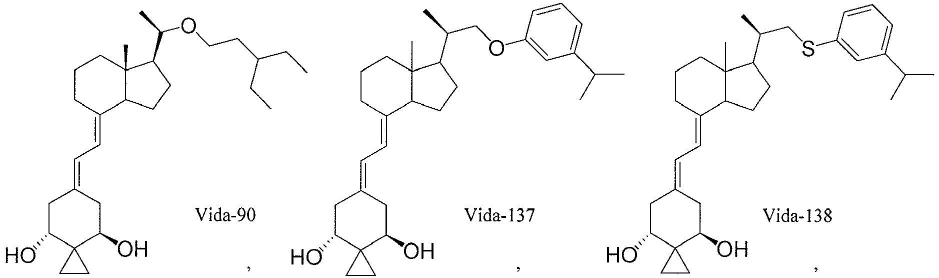 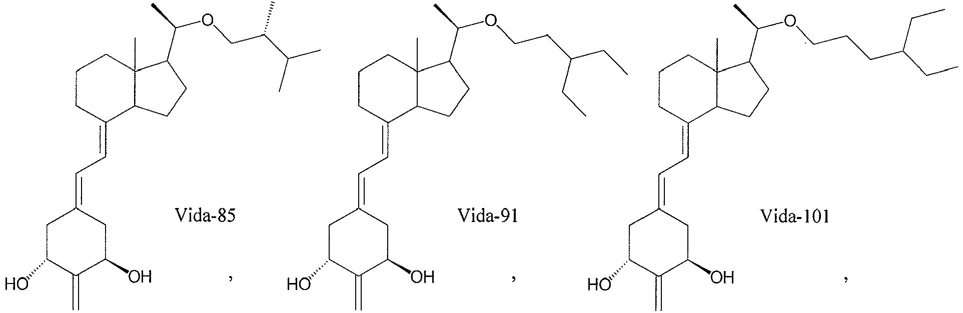 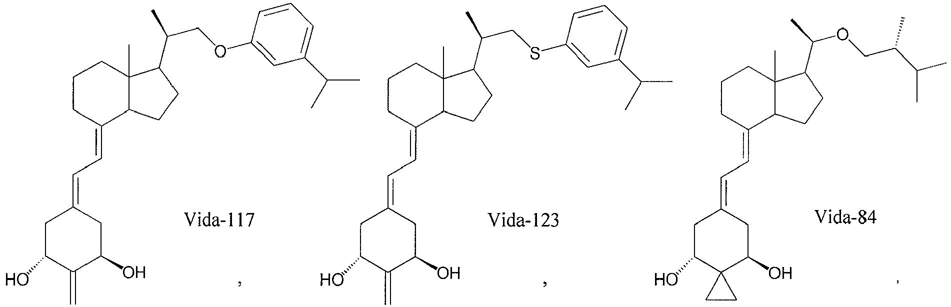 and pharmaceutically acceptable salts thereof.

Specific examples of compounds of Formula (I) include:
(*1R*,*3R*)-5-((*E*)-2-((*3aS*,*7αS*)-1-((*R*)-1-((*S*)-3-hydroxy-2,3-dimethylbutoxy)ethyl)-7α-methyldihydro-*1H*-inden-4(*2H*,*5H,6H,7H,7αH*)-ylidene)ethylidene)-2-methylenecyclohexane-1,3-diol (Vida-5);
(*4R, 8R*)*-*6*-*((*E*)*-*2*-*((*1S,7αS*)*-*1*-*((*R*)-1-(3-ethyl-3-hydroxypentyloxy)ethyl)-7α-methyldihydro-1H-inden-4(*2H,5H,6H*,*7H,*7*αH*)-ylidene)ethylidene)spiro[2.5]octane-4,8-diol (Vida-10);
(*4R, 8R*)*-6-*((*E*)*-2-*((*1S,7αS*)*-*1-((R)-1-(4-ethyl-4-hydroxyhexyloxy)ethyl)-7α-methyldihydro-*1H-*inden-4(*2H,5H,6H,7H,7αH*)-ylidene)ethylidene)spiro[2.5]octane-4,8-diol (Vida-20);
(*1R,3R*)-5-((*E*)-2-((*1S,3αS,7αS*)-1-((*R*)-1-(3-ethyl-3-hydroxypentyloxy)ethyl)-7α-methyldihydro*-1H*-inden-4(*2H,5H,6H,7H,7αH*)-ylidene)ethylidene)-2-methylenecyclohexane-1,3-diol (Vida-11);
(*1R,3R*)*-5-*((*E*)*-2-*((*1S,7αS*)-1-((*R*)-1-(4-ethyl-4-hydroxyhexyloxy)ethyl)-7α-methyldihydro-*1H-*inden-4(*2H,5H,6H,7H,7αH*)-ylidene)ethylidene)-2-methylenecyclohexane-1,3-diol (Vida-21);
(*4R,8R*)-6-((*E*)-2-((*1S,7αS*)-1-((*R*)-1-(3-hydroxy-3-methylbutoxy)ethyl)-7α-methyldihydro-1H-inden-4(*2H*,*5H*,*6H*,*7H,7αH*)-ylidene)ethylidene)spiro[2.5]octane-4,8-diol (Vida-1);
(*4R,8R*)-6-((*E*)-2-((*3αS,7αS*)-1-((*R)*-1-((*S*)-3-hydroxy-2,3-dimethylbutoxy)ethyl)-7α-methyldihydro-1H-inden-4(*2H,5H,6H,7H,7αH*)-ylidene)ethylidene)spiro[2.5]octane-4,8-diol (Vida-4);
(4*R*,8*R*)-6-((*E*)-2-(1-((*R*)-1-(3-(2-hydroxypropan-2-yl)phenoxy)propan-2-yl)-7α-methyldihydro-1H-inden-4(*2H,5H,6H*,*7H,7αH*)-ylidene)ethylidene)spiro[2.5]octane-4,8-diol (Vida-57);
(*4R, 8R*)-6-((*E*)-2-(1-((*R*)-1-(3-(2-hydroxypropan-2-yl)phenylthio)propan-2-yl)-7α-methyldihydro-1H-inden-4(*2H,5H,6H*,*7H,7αH*)-ylidene)ethylidene)spiro[2.5]octane-4,8-diol (Vida-58);
(1*R*,3*R*)-5-((*E*)-2-(1-((*R*)-1-(3-(2-hydroxypropan-2-yl)phenoxy)propan-2-yl)-7α-methyldihydro-1H-inden-4(*2H*,*5H,6H,7H,7αH*)-ylidene)ethylidene)-2-methylenecyclohexane-1,3-diol (Vida-37);
(*1R,3R*)-5-((*E*)-2-(1-((*R*)-1-(3-(2-hydroxypropan-2-yl)phenylthio)propan-2-yl)-7α-methyldihydro-1H-inden-4(*2H,5H,6H,7H,7αH*)-ylidene)ethylidene)-2-methylenecyclohexane-1,3-diol (Vida-43);
(*4R,8R*)-6-((*E*)-2-((*1S*,*7αS*)-1-((*R*)-1-(3-methylbutoxy)ethyl)-7α-methyldihydro-1H-inden-4(*2H,5H,6H,7H,7αH*)-ylidene)ethylidene)spiro[2.5]octane-4,8-diol (Vida-81);
(*4R,8R*)-6-((*E*)-2-((*3αS,7αS*)-1-((*R*)-1-((*S*)-2,3-dimethylbutoxy)ethyl)-7α-methyldihydro-1H-inden-4(*2H,5H,6H,7H,7αH*)-ylidene)ethylidene)spiro[2.5]octane-4,8-diol (Vida-84);
(*1R,3R*)-5-((*E*)-2-((*3αS*,7αS)-1-((*R*)-1-((*S*)-2,3-dimethylbutoxy)ethyl)-7α-methyldihydro-*1H-*inden-4(*2H,5H,6H,7H,7αH*)-ylidene)ethylidene)-2-methylenecyclohexane-1,3-diol (Vida-85);
(*4R,8R*)-6-((*E*)-2-((*1S,7αS*)-1-((*R*)-1-(3-ethyl-pentyloxy)ethyl)-7α-methyldihydro-1H-inden-4(*2H,5H,6H,7H,7αH*)-ylidene)ethylidene)spiro[2.5]octane-4,8-diol (Vida-90);
(*1R*,*3R*)-5-((*E*)-2-((*1S*,*3αS*,*7αS*)-1-((*R*)-1-(3-ethyl-pentyloxy)ethyl)-7α-methyldihydro-*1H-*inden-4(*2H,5H,6H,7H,7αH*)-ylidene)ethylidene)-2-methylenecyclohexane-1,3-diol (Vida-91);
(*4R*,*8R*)-6-((*E*)-2-((*1S*,*7αS*)-1-((R)-1-(4-ethyl-hexyloxy)ethyl)-7α-methyldihydro-*1H*-inden-4(*2H,5H,6H,7H,7αH*)-ylidene)ethylidene)spiro[2.5]octane-4,8-diol (Vida-100);
(*1R,3R*)-5-((*E*)-2-((*1S,7αS*)-1-((*R*)-1-(4-ethyl-hexyloxy)ethyl)-7α-methyldihydro-*1H*-inden-4(*2H,5H,6H,7H,7αH*)-ylidene)ethylidene)-2-methylenecyclohexane-1,3-diol (Vida-101);
(1*R*,3*R*)-5-((*E*)-2-(1-((*R*)-1-(3-isopropylphenoxy)propan-2-yl)-7α-methyldihydro-1H-inden-4(*2H,5H,6H,7H,7αH*)-ylidene)ethylidene)-2-methylenecyclohexane-1,3-diol (Vida-117);
(*1R*,*3R*)-5-((*E*)-2-(1-((*R*)-1-(3-isopropylphenylthio)propan-2-yl)-7α-methyldihydro-1H-inden-4(*2H*,*5H,6H,7H,7αH*)-ylidene)ethylidene)-2-methylenecyclohexane-1,3-diol (Vida-123)
(*4R,8R*)-6-((*E*)-2-(1-((*R*)-1-(3-isopropylphenoxy)propan-2-yl)-7α-methyldihydro-1H-inden-4(*2H,5H,6H,7H,7αH*)-ylidene)ethylidene)spiro[2.5]octane-4,8-diol (Vida-137); and
(*4R,8R*)-6-((*E*)-2-(1-((*R*)-1-(3-isopropylphenylthio)propan-2-yl)-7α-methyldihydro-1H-inden-4(*2H*,*5H*,*6H*,*7H,7αH*)-ylidene)ethylidene)spiro[2.5]octane-4,8-diol (Vida-138).

The pharmaceutically acceptable salts of any of the compounds of Formula (I) can be any phannaceutically acceptable salt, prepared in any manner. Typically, such salts can be prepared from a compound using relatively nontoxic acids or bases, depending on the particular starting "parent" compound. Base addition salts of parent compounds with relatively acidic functionalities can be prepared by contacting the free acid form of such compounds with a sufficient amount of the desired base, either neat or in a suitable inert solvent. Examples of phannaceutically acceptable base addition salts include sodium, potassium, calcium, ammonium, organic amino, magnesium salts, and the like. Acid addition salts of parent compounds having relatively basic functionalities can be obtained by contacting the free base form of such compounds with a sufficient amount of the desired acid, either neat or in a suitable inert solvent. Examples of phannaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, maleic, malonic, benzoic, succinic, suberic, fumaric, mandelic, phthalic, benzenesulfonic, *p*-tolylsulfonic, citric, tartaric, methanesulfonic, and the like. Also included are salts of amino acids such as arginate and the like, and salts of organic acids like glucuronic or galactunoric acids (see, for example, Berge et al., 1977, Journal of Pharmaceutical Science, 66: 1-19). In an embodiment, the anion can be any one of the 53 approved FDA anions. Compounds of the invention can contain both basic and acidic functionalities, which allow the compounds to be converted into either base or acid addition salts. The neutral fonns of the compounds can be regenerated by contacting the salt with a base or acid and isolating the parent compound in the conventional manner.

A compound or salt therof of Fonnula (I), including (II) and (IIIa), can be prepared by any of several techniques. For instance, the compounds can be prepared in accordance with Schemes 1-39 and the Examples set forth herein. It is meant to be understood that the orders of the steps in the syntheses provided can be varied, reagents, solvents, and reaction conditions can be substituted, and vulnerable moieties can be protected and deprotected, as necessary. Hydroxyl protecting groups include, but are not limited to, acetyl, allyl, allyoxycarbonyl, benzoyl, benzyl(phenylethylmethyl), benzylidene, benzyloxycarbonyl (Cbz), benzyl, *tert*-butyl, *tert-*butyldimethylsilyl, *tert*-butyldiphenylsilyl, 3,4-dimethoxybenzyl, 3,4-dimethoxybenzyloxycarbonyl, methoxyacetyl, 4-methoxybenzyloxycarbonyl, *para*-methoxybenzyl, methoxycarbonyl, methyl, *para-*toluenesulfonyl, 2,2,2-trichloroethoxycarbonyl, 2,2,2-trichloroethyl, triethylsilyl, tri-isopropylsilyl, trifluoroacetyl, 2-(trimethylsilyl)ethoxycarbonyl, trimethylsilylethyl, triphenylmethyl, 2-(triphenylphosphino)ethoxycarbonyl and the like.

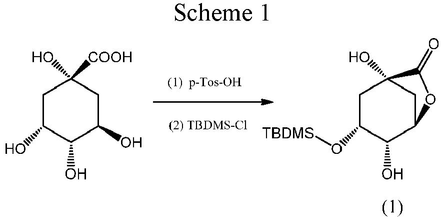

(-)-Quinic acid can be converted to compound 1 by reacting the former with p-toluene sulfonic acid monohydrate. Water is azeotropically removed (see, e.g., Elliot et al., 1981, J. Chem. Soc. Perkin trans 1, 1782-1789). The reaction typically is conducted in toluene at reflux. The obtained product can be protected by a suitable protecting group, such as *tert*-butyldimethylsilyl (TBDMS) chloride and base in *N,N*-dimethylformamide at ambient temperature. A suitable base is imidazole.

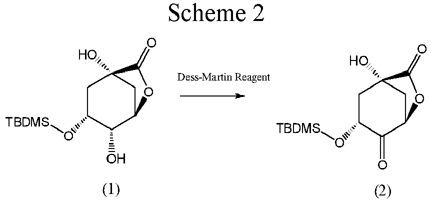

Compound 1 can be converted to compound 2 by reacting the former with an oxidizing agent (as described in Glebocka et al., 2006, J. Med. Chem., 49: 2909-2920). Oxidizing agents include the Dess-Martin reagent (periodinane) and the like. The reaction typically is conducted in methylene chloride at ambient temperature, preferably at a temperature of about 20-25 °C.

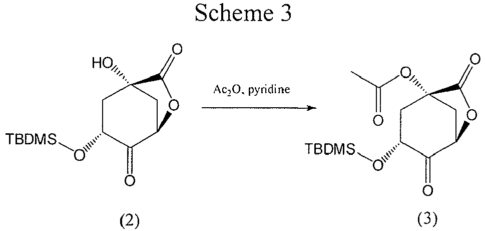

Compound 2 can be converted to compound 3 by reacting the former, acylating reagent, and base. Suitable acylating reagents include acetic anhydride, acetyl chloride, acetyl bromide, and acetyl iodide. Suitable bases include 4-(*N,N-*dimethyl)pyridine (DMAP), pyridine, and/or triethylamine. The reaction typically is conducted at ambient temperature, preferably at a temperature of about 20-25 °C.

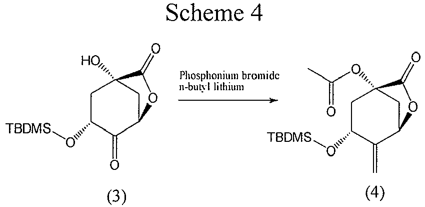

Compound 3 can be converted to compound 4 by reacting with a phosphonium bromide, e.g., methyl phosphonium bromide, and a base in tetrahydrofuran (THF). Suitable bases include *n*-butyl lithium and *sec*-butyl lithium. The reaction is typically conducted continuously in THF at about -70 °C for 20-30 min followed by -40 °C to about -50 °C for 2 h.

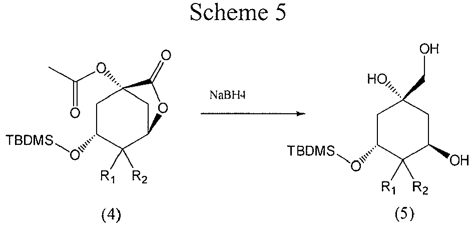

Compound 4 can be converted to compound 5 by reaction with a reducing agent in alcohol. Suitable reducing agents include sodium borohydride and sodium cyanoborohydride. Alcohols include methanol, ethanol, n-propyl alcohol, *sec*-propyl alcohol, and the like. The reaction is typically conducted continuously in ethanol at about 0 °C for 1 h, about 6 °C for 10 h, and at ambient temperature, e.g., about 20-25 °C, for 2 h.

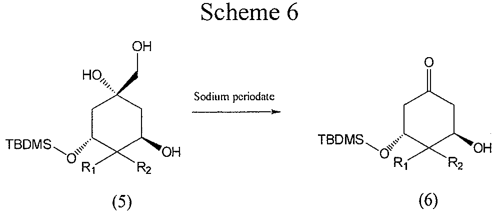

Compound 5 can be converted to compound 6 by reacting with sodium periodate in alcohol/water. Suitable alcohols include methanol, ethanol, *n*-propyl alcohol, and *sec-*propyl alcohol. The reaction is typically conducted continuously in methanol/water at about 0 °C for 1 h.

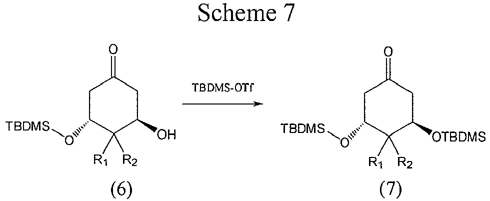

Compound 6 can be converted to compound 7 by reacting with *tert-*butyldimethylsilyl-*O*-triflate and a base in methylene chloride. Suitable bases include 2,6-lutidine and triethylamine. Silyl reagents include *tert*-butyldimethylsilyl-*O*-triflate , *tert-*butyldimethylsilyl chloride, and the like. The reaction is typically conducted in methylene chloride with *tert*-butyldimethylsilyl-*O*-triflate at about -50 °C for 30 min and at about -15 °C for 30 min.

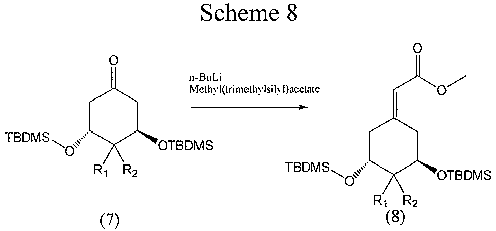

Compound 7 can be converted to compound 8 by reacting with methyl(trimethylsilyl)acetate, n-butyl lithium, and diisopropylamine in THF. The reaction typically is conducted in THF at -78 °C for 2 h.

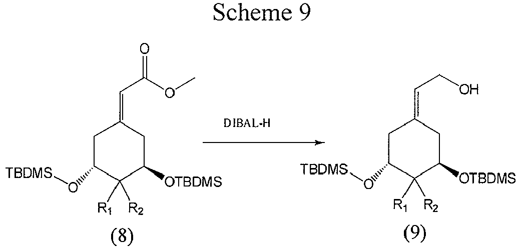

Compound 8 can be converted to compound 9 by reacting the former and diisobutylaluminum hydride (DIBAL-H) in a toluene/methylene chloride mixture. The reaction typically is conducted in toluene/methylene chloride at -78 °C for 1 h.

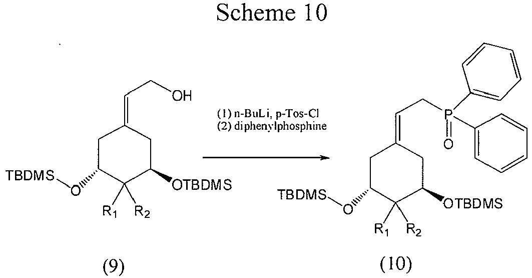

Compound 9 can be converted to compound 10 by reacting the former, *n*-butyl lithium in THF, and freshly recrystallized *p*-toluene sulfonyl chloride. The reaction typically is conducted at 0 °C for 5 min. The obtained *O*-tosylated compound can be reacted with pretreated diphenylphosphine by *n*-butyl lithium. The reaction typically is conducted at about 0 °C for 40 min continuously in THF.

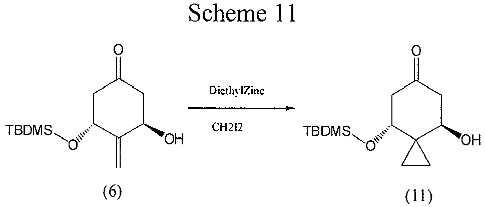

Compound 6 can be converted to compound 11 by reacting the former, diethyl zinc in toluene, followed by ethylene diiodide. The reaction typically is conducted at 0 °C for 3 h.

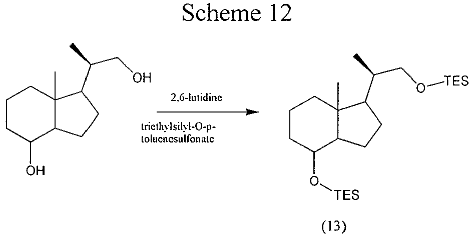

Compound 13 can be prepared by reacting the starting material with a silyl reagent, such as triethylsilyl-*O-p*-toluenesulfonate, triethylsilyl-O-triflate, triethylsilyl chloride and the like, in the presence of 2,6-lutidine in anhydrous THF. The reaction conditions are varied. The temperature is in a range of -78 °C to 0 °C depending on the reagent used. The reaction typically is conducted at -78 °C for 20 min to 1 h.

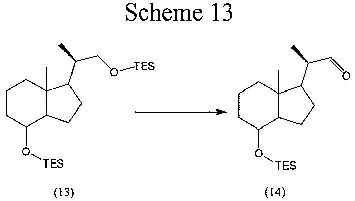

Compound 13 can be converted to compound 14 by reacting the former, oxalyl chloride, triethylamine in *N,N*-dimethyl sulfoxide (DMSO) in methylene chloride at lower temperature. The reaction typically is conducted at -60 °C for 1 h.

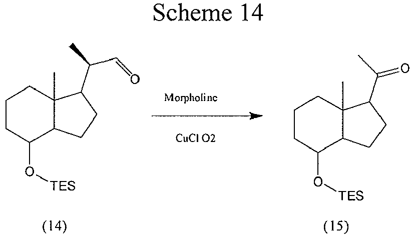

Compound 14 can be converted to compound 15 by reacting the former and morpholine in toluene under reflux conditions. The resulting water can be removed azeotropically. The resulting adduct can be reacted with cuprous chloride and oxygen in acetonitrile. Oxygen is bubbled through into the reaction mixture. The second reaction typically is conducted at a temperature of about 20-25 °C for 8 h.

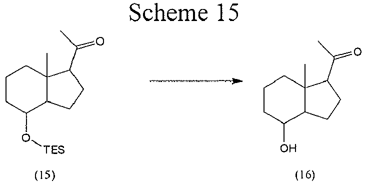

Compound 15 can be converted to compound 16 by reacting the former and a de-silylating agent, such as tetra(*n*-butyl)ammonium fluoride, hydrofluoric acid, and the like. The reaction typically is conducted at a temperature of about 20-25 °C for 5 h.

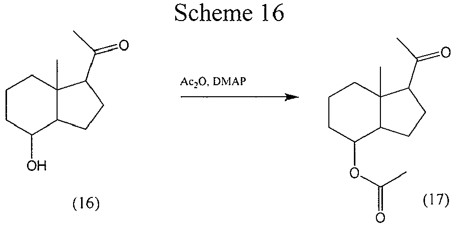

Compound 16 can be converted to compound 17 by reacting the former and an acylating reagent such as acetic anhydrous, acetyl chloride, acetyl bromide, acetyl iodide, and the like. The reaction typically is conducted at 0 °C for 2 h and at about 20-25 °C for 3 h in the presence of DMAP in methylene chloride and the like.

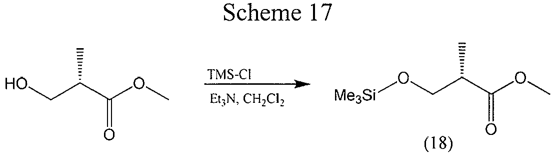

The compound ((*S*)-methyl-3-hydroxy-2-methylpropanoate) can be converted to compound 18 by reacting the former and trimethylsilyl chloride, trimethylsilyl bromide, or trimethylsilyl-O-triflate in the presence of triethylamine in methylene chloride. The reaction typically is conducted at a temperature of about 20-25 °C for 2 h.

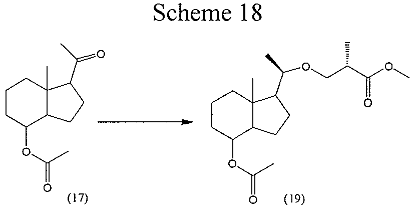

Compound 17 can be converted to compound 19 by reacting the former and compound 18 in methylene chloride in the presence of trimethylsilyl-*O*-triflate and triethylsilane. The reaction typically is conducted at -78 °C for 1 h and at about 20-25 °C for 4 h.

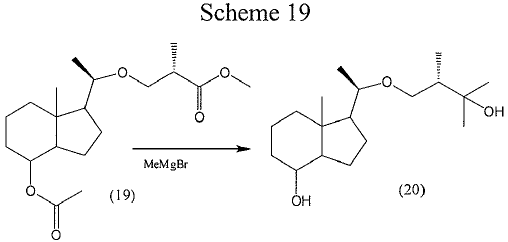

Compound 19 can be converted to compound 20 by reacting the former and methyl magnesium bromide in THF. The reaction typically is conducted at room temperature for overnight.

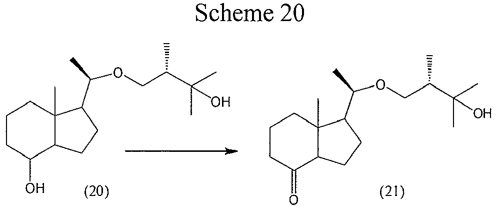

Compound 20 can be converted to compound 21 by reacting the former and an oxidizing reagent, such as the Dess-Martin reagent in methylene chloride. The reaction typically is conducted at about 20-25 °C for overnight.

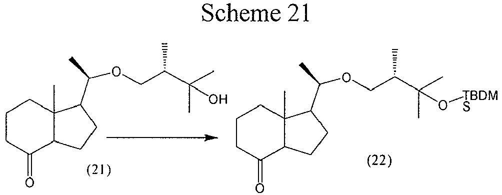

Compound 21 can be converted to compound 22 by reacting with *tert-*butyldimethylsilyl-*O*-triflate and a base in methylene chloride. Suitable bases include 2,6-lutidine and triethylamine. Silyl reagents include *tert*-butyldimethylsilyl-*O*-triflate, *tert-*butyldimethylsilyl chloride, and the like. The reaction is typically conducted in methylene chloride with *tert*-butyldimethylsilyl-*O*-triflate at about -50 °C for 30 min and at -15 °C for 30 min.

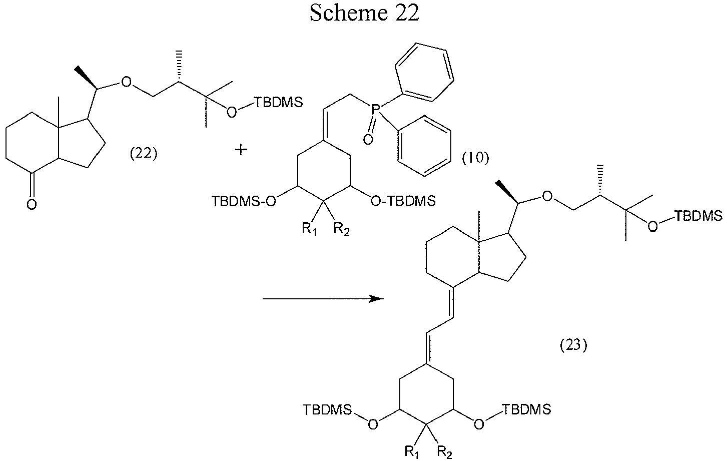

Compound 22 can be converted to compound 23 by reacting with compound 10. The reaction is conducted in anhydrous THF under an argon atmosphere. Initially, compound 10 is dissolved in THF and cooled to -78 °C. *n*-Butyl lithium is added to the reaction mixture and stirred. Compound 22 is then added, and stirred at -78 °C for 3 h and at about 6 °C for 16 h.

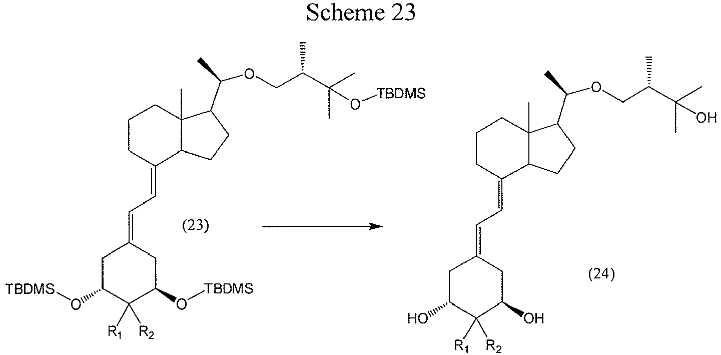

Compound 23 can be converted to compound 24 by reacting the former and a de-silylating reagent, such as tri(*n*-butyl)ammonium fluoride, hydrofluoric acid, and the like. The reaction typically is conducted at about 20-25 °C for 18 h in THF.

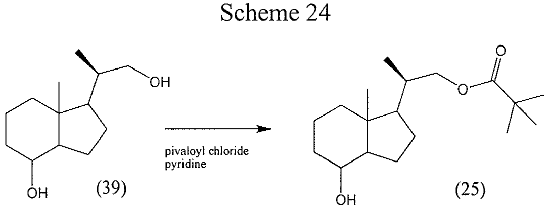

Compound 25 can be prepared by reacting compound 39 (see Scheme 38) and pivaloyl chloride in methylene chloride and pyridine mixture. The reaction typically is conducted at 0 °C for 4 h and at about 20-25 °C overnight.

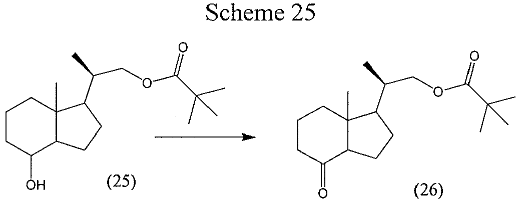

Compound 25 can be converted to compound 26 by reacting the former and an oxidizing agent, such as PDC in methylene chloride. The reaction typically is conducted at 0 °C for 8 h and at about 20-25 °C overnight.

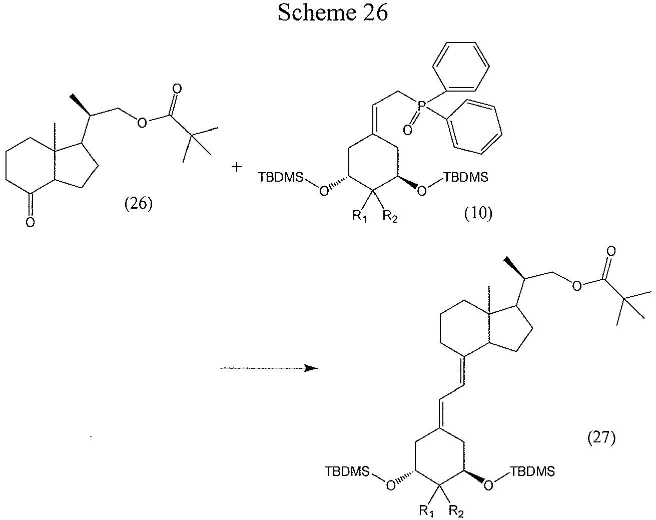

Compound 26 can be converted to compound 27 under the same conditions described in Scheme 22.

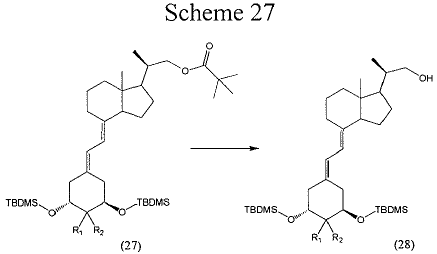

Compound 27 can be converted to compound 28 by reacting the former and lithium aluminum hydride in ether or THF. The reaction typically is conducted at -78 °C for 10 min and at 0 °C for 50 min.

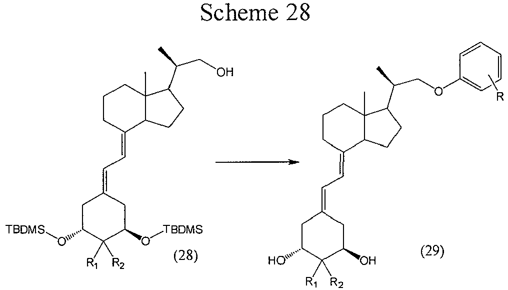

Compound 28 can be converted to compound 29 by reacting the former and substituted or unsubstituted phenol in toluene in the presence of triphenylphosphine and di-*tert*-butyl-azodicarboxylate in toluene. The reaction typically is conducted at about 85 °C for 4 h. The obtaining product can be then treated with the reagents described in Scheme 23.

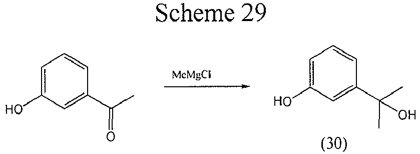

The compound 1-(3-hydroxyphenyl)ethanone can be converted to compound 30 by reacting the ketone and methyl magnesium bromide in anhydrous THF. The reaction typically is conducted at about 20-25 °C for 18 h and at reflux temperature for 2 h. The similar reaction is described in Scheme 19.

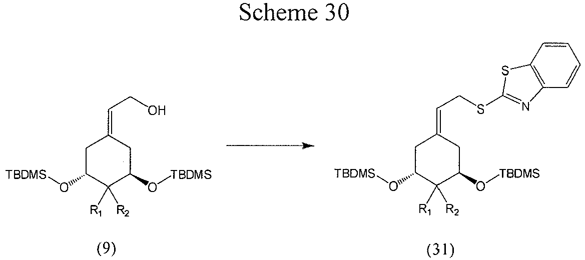

Compound 9 can be converted to compound 31 by reacting the former and benzo[d]thiazole-2-thiol in methylene chloride in the presence of triphenylphosphine and diisopropyl-azodicarboxylate. The reaction typically is conducted at 0 °C for 1 h. The reaction is similar to that described in Scheme 28.

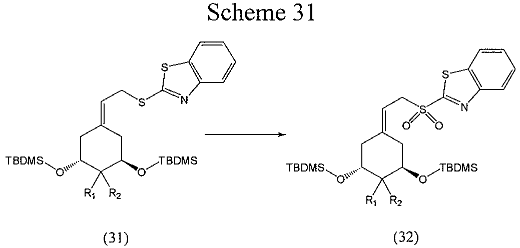

Compound 31 can be converted to compound 32 by reacting the former and an oxidizing agent, such as hydrogen peroxide or (NH₄)₆Mo₇.4H₂O, in ethyl alcohol. The reaction typically is conducted at 0 °C for 1 h and at about 20-25 °C for 3 h.

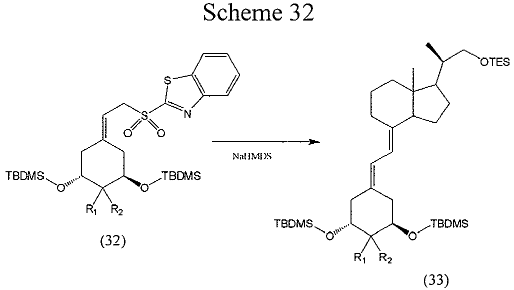

Compound 32 can be converted to compound 33 by reacting the former and compound 40 in the presence of NaHMDS in THF. The reaction typically is conducted at -78 °C for 2 h and at about 20-25 °C for 15 h.

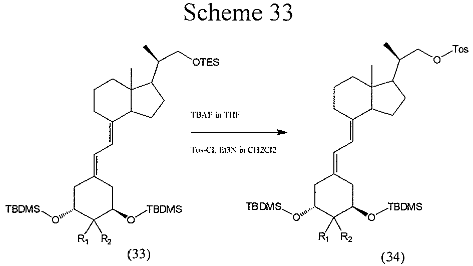

Compound 33 can be converted to compound 34 by reacting the former and a de-silylating agent such as TBAF (tetrabutylammonium fluoride) or hydrofluoric acid in THF, followed by the reaction ofp-toluene sulfonyl chloride in the presence of triethylamine and DMAP in methylene chloride. The initial reaction typically is conducted at about 20-25 °C for 3-4 h. And the second reaction typically is conducted at about 20-25 °C overnight.

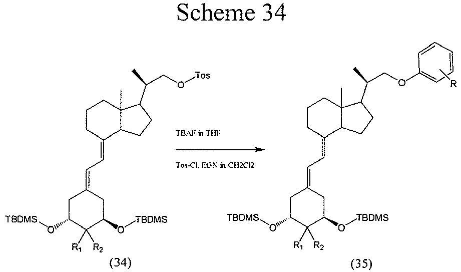

Compound 34 can be converted to compound 35 by reacting the former and substituted or unsubstituted phenol in the presence of sodium hydride in *N*,*N-*dimethylformamide (DMF). The reaction typically is conducted at about 20-25 °C overnight.

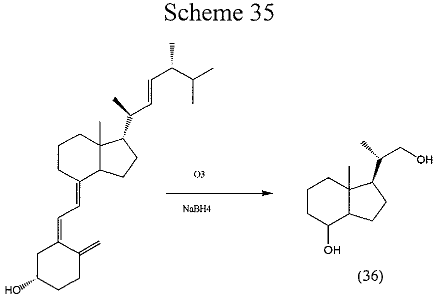

Ergocalciferol ((+)-vitamin D₂) can be converted to compound 36 by reacting the former and ozone in the presence of sodium bicarbonate in mixture of methylene chloride and methanol. The reaction typically is conducted at about 20-25 °C or 4-5 h while bubbling ozone into the reaction mixture. Sodium borohydride is then added to the mixture to generate alcohol. This reaction typically is conducted at about 20-25 °C until compete disappearance of the resulting aldehyde is observed.

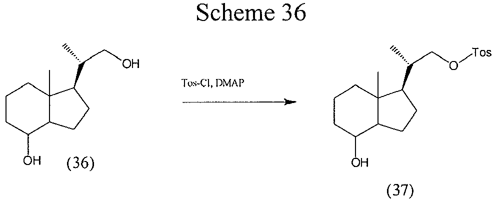

Compound 36 can be converted to compound 37 by reacting the former and *p-*toluenesulfonyl chloride in the presence of DMAP and 40% Et₃N solution in methylene chloride. The reaction typically is conducted at about 20-25 °C for 4-5 h.

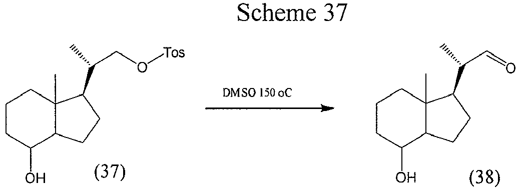

Compound 37 can be converted to compound 38 by reacting the former and DMSO in the presence of sodium bicarbonate. The reaction typically is conducted at about 150 °C for 30 min.

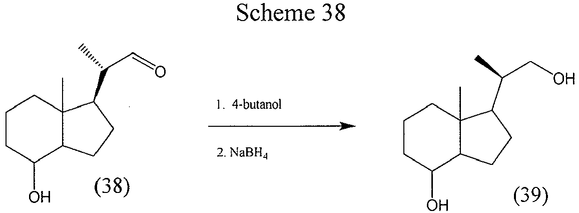

Compound 38 can be converted to compound 39 by reacting the former and tetra(*n*-butyl)ammonium hydroxide in methylene chloride. The reaction typically is conducted at about 20-25 °C for 16 h. The resulting product can be treated with sodium borohydride in methanol.

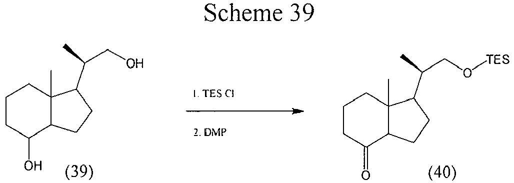

Compound 39 can be converted to compound 40 by reacting the former and triethylsilyl chloride in methylene chloride in the presence of 40% Et₃N solution and DMAP. The reaction typically is conducted at about 20-25 °C for 1 h. The resulting product can be treated with the Dess-Martin reagent in methylene chloride. The reaction typically conducted at about 20-25 °C for 2 h.

The invention also provides a pharmaceutical composition comprising (i) a compound or a pharmaceutically acceptable salt thereof of Formula (I) and (ii) a pharmaceutically acceptable carrier. The carrier can be any of those conventionally used and is limited only by physico-chemical considerations, such as solubility and lack of reactivity with the active compound(s), and by the route of administration. It will be appreciated by one of skill in the art that, in addition to the following described pharmaceutical composition, the compounds of the present inventive methods can be formulated as inclusion complexes, such as cyclodextrin inclusion complexes, or liposomes.

The pharmaceutically acceptable carriers described herein, for example, vehicles, adjuvants, excipients, and diluents, are well-known to those skilled in the art and are readily available to the public. It is preferred that the pharmaceutically acceptable carrier be one which is chemically inert to the active agent(s) and one which has no detrimental side effects or toxicity under the conditions of use.

The choice of carrier will be determined in part by the particular compound or salt thereof of the invention and other active agents or drugs used, as well as by the particular method used to administer the compound. Accordingly, there are a variety of suitable formulations of the pharmaceutical composition of the present inventive methods. The following formulations for oral, nasal, parenteral, subcutaneous, intrathecal, intravenous, intramuscular, interperitoneal, rectal, transdermal, sublingual, internasal, intranasal, ocular, and vaginal administration are exemplary and are in no way limiting. One skilled in the art will appreciate that these routes of administering the compound or salt thereof of the invention are known, and, although more than one route can be used to administer a particular compound, a particular route can provide a more immediate and more effective response than another route.

Formulations suitable for oral administration are preferred in accordance with the present invention and can consist of (a) liquid solutions, such as an effective amount of the compound or salt thereof dissolved in diluents, such as water, saline, or orange juice; (b) capsules, sachets, tablets, lozenges, and troches, each containing a predetermined amount of the active ingredient, as solids or granules; (c) powders; (d) suspensions in an appropriate liquid; and (e) suitable emulsions. Liquid formulations may include diluents, such as water and alcohols, for example, ethanol, benzyl alcohol, and the polyethylene alcohols, either with or without the addition of a pharmaceutically acceptable surfactant. Capsule forms can be of the ordinary hard- or soft-shelled gelatin type containing, for example, surfactants, lubricants, and inert fillers, such as lactose, sucrose, calcium phosphate, and corn starch. Tablet forms can include one or more of lactose, sucrose, mannitol, corn starch, potato starch, alginic acid, microcrystalline cellulose, acacia, gelatin, guar gum, colloidal silicon dioxide, croscarmellose sodium, talc, magnesium stearate, calcium stearate, zinc stearate, stearic acid, and other excipients, colorants, diluents, buffering agents, disintegrating agents, moistening agents, preservatives, flavoring agents, and pharmacologically compatible excipients. Lozenge forms can comprise the active ingredient in a flavor, usually sucrose and acacia or tragacanth, as well as pastilles comprising the active ingredient in an inert base, such as gelatin and glycerin, or sucrose and acacia, emulsions, gels, and the like containing, in addition to the active ingredient, such excipients as are known in the art.

The compounds or salts of the invention also can be made into an injectable formulation. The requirements for effective pharmaceutical carriers for injectable compositions are well-known to those of ordinary skill in the art (See, e.g., Pharmaceutics and Pharmacy Practice, J.B. Lippincott Company, Philadelphia, PA, Banker and Chalmers, eds., pages 238-250 (1982), and ASHP Handbook on Injectable Drugs, Toissel, 4th ed., pages 622-630 (1986)).

Topical formulations are well-known to those of skill in the art. Such formulations are particularly suitable in the context of the present invention for application to the skin.

The compounds or salts of the invention, alone or in combination with other suitable components, can be made into aerosol formulations to be administered via inhalation. These aerosol formulations can be placed into pressurized acceptable propellants, such as dichlorodifluoromethane, propane, nitrogen, and the like. They also may be formulated as pharmaceuticals for non-pressured preparations, such as in a nebulizer or an atomizer. Such spray formulations also may be used to spray mucosa.

Formulations suitable for parenteral administration include aqueous and non-aqueous, isotonic sterile injection solutions, which can contain anti-oxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. The compounds of the invention can be administered in a physiologically acceptable diluent in a pharmaceutical carrier, such as a sterile liquid or mixture of liquids, including water, saline, aqueous dextrose and related sugar solutions, an alcohol, such as ethanol, isopropanol, or hexadecyl alcohol, glycols, such as propylene glycol or polyethylene glycol, dimethylsulfoxide, glycerol ketals, such as 2,2-dimethyl-1,3-dioxolane-4-methanol, ethers, such as poly(ethyleneglycol) 400, an oil, a fatty acid, a fatty acid ester or glyceride, or an acetylated fatty acid glyceride with or without the addition of a pharmaceutically acceptable surfactant, such as a soap or a detergent, suspending agent, such as pectin, carbomers, methylcellulose, hydroxypropylmethylcellulose, or carboxymethylcellulose, or emulsifying agents and other pharmaceutical adjuvants.

Oils, which can be used in parenteral formulations include petroleum, animal, vegetable, or synthetic oils. Specific examples of oils include peanut, soybean, sesame, cottonseed, corn, olive, petrolatum, and mineral. Suitable fatty acids for use in parenteral formulations include oleic acid, stearic acid, and isostearic acid. Ethyl oleate and isopropyl myristate are examples of suitable fatty acid esters.

Suitable soaps for use in parenteral formulations include fatty alkali metal, ammonium, and triethanolamine salts. Suitable detergents include (a) cationic detergents such as, for example, dimethyl dialkyl ammonium halides, and alkyl pyridinium halides, (b) anionic detergents such as, for example, alkyl, aryl, and olefin sulfonates, alkyl, olefin, ether, and monoglyceride sulfates, and sulfosuccinates, (c) nonionic detergents such as, for example, fatty amine oxides, fatty acid alkanolamides, and polyoxyethylenepolypropylene copolymers, (d) amphoteric detergents such as, for example, alkyl-β-aminopropionates, and 2-alkyl-imidazoline quaternary ammonium salts, and (e) mixtures thereof.

The parenteral formulations can contain a certain amount of the active ingredient such as from about 0.001% to about 25% by weight. Preservatives and buffers can be used. In order to minimize or eliminate irritation at the site of injection, such compositions may contain one or more nonionic surfactants having a hydrophile-lipophile balance (HLB) of from about 12 to about 17. The quantity of surfactant in such formulations will typically range from about 5% to about 15% by weight. Suitable surfactants include polyethylene sorbitan fatty acid esters, such as sorbitan monooleate and the high molecular weight adducts of ethylene oxide with a hydrophobic base, formed by the condensation of propylene oxide with propylene glycol. The parenteral formulations can be presented in unit-dose or multidose sealed containers, such as ampoules and vials, and can be stored in a freeze-dried (lyophilized) condition requiring only the addition of the sterile liquid excipient, for example, water, for injections, immediately prior to use. Extemporaneous injection solutions and suspensions can be prepared from sterile powders, granules, and tablets of the kind previously described.

Additionally, the compounds of the invention, or compositions comprising such compounds, can be made into suppositories by mixing with a variety of bases, such as emulsifying bases or water-soluble bases. Formulations suitable for vaginal administration can be presented as pessaries, tampons, creams, gels, pastes, foams, or spray formulas containing, in addition to the active ingredient, such carriers as are known in the art to be appropriate.

The compounds of the invention described herein can be administered to a cell *in vitro.* As used herein, the term *"in vitro"* means that the cell is not in a living organism. The compounds of the invention also can be administered to a cell *in vivo.* As used herein, the term *"in vivo"* means that the cell is a part of a living organism or is the living organism. Furthermore, the compounds of the invention can be administered to a subject *in vivo* or *ex vivo.* The term "*ex vivo*" as used herein refers to the administration of a compound to a cell or a population of cells *in vitro,* followed by administration of the cell or population of cells to a subject.

The compounds of the invention can be administered to a cell, preferably to a cell of a subject. Subjects include, for example, bacteria, yeast, fungi, plants, and mammals. Preferably, the subject is a mammal. For purposes of the present invention, mammals include, but are not limited to, the order Rodentia, such as mice, and the order Logomorpha, such as rabbits. It is preferred that the mammals are from the order Carnivora, including Felines (cats) and Canines (dogs). It is more preferred that the mammals are from the order Artiodactyla, including Bovines (cows) and Swines (pigs) or of the order Perssodactyla, including Equines (horses). It is most preferred that the mammals are of the order Primates, Ceboids, or Simioids (monkeys) or of the order Anthropoids (humans and apes). An especially preferred mammal is the human. Furthermore, the subject can be the unborn offspring of any of the forgoing hosts, especially mammals (e.g., humans), in which case any screening of the subject or cells of the subject, or administration of compounds to the subject or cells of the subject, can be *performed in utero.*

The amount or dose of a compound of the invention should be sufficient to affect a therapeutic or prophylactic response in the subject over a reasonable time frame. The appropriate dose will depend upon the nature and severity of the disease or affliction to be treated or prevented, as well as by other factors. For instance, the dose also will be determined by the existence, nature and extent of any adverse side effects that might accompany the administration of a particular compound. Ultimately, the attending physician will decide the dosage of the compound of the present invention with which to treat each individual patient, taking into consideration a variety of factors, such as age, body weight, general health, diet, sex, compound to be administered, route of administration, and the severity of the condition being treated. A preferred dose of a compound or salt thereof of Formula (I) is the maximum that a patient can tolerate without incurring serious side effects. Typical doses might be, for example, about 0.01 µg/day to about 15,000 mg/day (e.g., 0.1 mg to 1 g daily, 5 mg to 500 mg daily) or 0.0001 µg/kg to about 200 mg /kg body weight per day.

Depending on the disease to be treated, a particularly advantageous daily dose of the compound or salt thereof of Formula (I) can be from 0.1 µg to 1000 µg per day per 75 kg patient or from 0.01 µg to 100 mg per day per 75 kg patient. For example, if the disease target is left ventricular hypertrophy, endothelial dysfunction, or proteinuria with minimal effects on serum Ca, a compound or salt therof of Formula (I) (e.g., Vida-5 or Vida-10) can be administered with the preferred dose range from 0.01 to 10,000 µg per day (e.g., 0.1 to 1000 µg per day) per 75 kg patient. Alternatively, if it is intended to raise serum Ca and at the same time treat left ventricular hypertrophy and proteinuria, a compound or salt therof of Formula (I) (e.g., Vida-11) can be administered with the preferred dose range from 0.01 to 10,000 µg per day (e.g., 0.1 to 1000 µg per day) per 75 kg patient.

The compounds can be used for any purpose including, without limitation, the treatment, prevention, or diagnosis of a disease or condition, the screening of compounds that can be used to treat, prevent, or diagnose a disease or condition, or the research of the underlying mechanisms or causes of a disease or condition, which research can be used, for example, in the development of methods to treat, prevent, or diagnose the disease or condition. Without wishing to be bound by any particular theory, it is believed that the compounds of the invention are particularly useful with respect to diseases and conditions involving the modulation of the vitamin D receptor.

Thus, one aspect of the invention provides a method of treating a disease which benefits from a modulation of the VDR comprising administering an effective amount of the compound or salt thereof of Formula (I) to a subject in need thereof. Suitable subjects are as previously described herein. The subject is desirably a mammal, especially a human. The foregoing method is most suitable for use in conjunction with a subject that is afflicted with a disease or at risk for developing a disease, such as a disease that benefits from the modulation of VDR (e.g., a disease associated with vitamin D deficiency and/or decreased VDR activation). Such diseases include, for example, a bone disorder, cardiovascular disease, a cardiovascular complication associated with renal disease, endothelial dysfunction, hyperparathyroidism, hypocalcemia, an immune disorder, left ventricular hypertrophy, a proliferative disease, proteinuria, renal disease, thrombosis, viral infection (e.g., influenza), bacterial infection (e.g., tuberculosis), musculoskeletal disorders, high blood pressure, hypertriglyceridemia, multiple sclerosis, myelodysplastic syndrome, proximal myopathy, premature aging, metabolic syndrome, insulin resistance, obesity, seasonal affective disorder, senile warts, and skin pigmentation disorders. More specifically, diseases include hypocalcemia, psoriasis, osteoporosis, primary hyperparathyroidism, secondary hyperparathyroidism, proteinuria, endothelial dysfunction, left ventricular hypertrophy, thrombosis, and cancer. Preferably, one or more symptoms of the disease is prevented, reduced, or eliminated subsequent to administration of at least one compound of the invention, thereby effectively treating or preventing the disease to at least some degree.

Hypocalcemia is a potentially life threatening biochemical abnormality that carries risks for serious errors in diagnosis and treatment. The most common cause of hypocalcemia in primary care is vitamin D deficiency (Cooper et al., 2008, British Medical Journal, 336, 1298-302). Hypocalcemia can be associated with chronic kidney disease (CKD), which is also linked to a vitamin D deficiency and reduced VDR activation due to reduced renal function. Compounds of Formula (I) (e.g., Vida-11) can increase VDR activation *in vivo* to treat hypocalcemia. In addition, if necessary, a compound or salt therof of Formula (I) can be co-administered with a second therapeutic agent, such as calcium gluconate or calcium chloride to treat hypocalcemia.

Compounds of Formula (I) can effectively inhibit abnormal skin cell proliferation, such as the skin cell proliferation associated with psoriasis. Topical administration for the treatment of psoriasis is preferred. Formulations for topical administration such as ointments, creams, gels, pastes, foams, and lotions are especially preferred. If needed, additional therapeutic agents can be administered with a compound or salt therof of Formula (I) to treat psoriasis, such as adalimumab, anthralin, desoximetasone, calcipotriol, a retinoid, methotrexate, and cyclosporine.

With osteoporosis, bone mineral density and/or bone volume is reduced. Vitamin D insufficiency is associated with bone loss. In some embodiments, a compound or salt therof of Formula (I) can be co-administered with an additional therapeutic agent, such as a bisphosphonate (e.g., sodium alendronate, risedronate, ibandronate), teriparatide, strontium renelate, or hormone replacement therapy (e.g., estrogen, testosterone) to treat osteoporosis.

Hyperparathyroidism is overactivity of the parathyroid glands resulting in excess production of PTH. The increased PTH production increases bone resorption and, in turn, increased bone loss. As a result, a common manifestation of hyperparathyroidism is a bone disorder (e.g. osteomalacia, osteoporosis). Primary hyperparathyroidism results from a hyperfunction of the parathyroid glands themselves and is often associated with a parathyroid tumor. Secondary hyperparathyroidism is the reaction of the parathyroid glands to a condition caused by something other than a parathyroid pathology (e.g., chronic renal failure).

Proteinuria means the presence of an excess of serum proteins in the urine, which is a marker for kidney disease. In an embodiment, the method includes administering a compound or salt therof of Formula (I) (e.g., Vida-4, Vida-5, or Vida-10) to treat proteinuria associated with CKD without raising serum calcium. A known side effect of certain vitamin D analogs, such as calcitriol, doxercalciferol, and paricalcitol, is hypercalcemia (an increase in serum calcium). Therefore, a compound or salt therof of Formula (I) (e.g., Vida-4, Vida-5, Vida-10) that can treat proteinuria without causing hypercalcemia are desirable.

Endothelial dysfunction is the loss of proper endothelial function, which is a major contributing factor to proteinuria and left ventricular hypertrophy. In an embodiment, the method includes improving endothelial function in CKD without raising serum calcium. Preferably, the compound or salt therof of Formula (I) is Vida-4, Vida-5, or Vida-10.

Left ventricular hypertrophy is the thickening of the myocardium of the left ventricle of the heart, which can be caused by abnormal hemodynamic factors such as high blood pressure and other risk factors, such as CKD, diabetes, endothelial dysfunction, oxidative stress, etc. Left ventricular hypertrophy is recognized as a marker for cardiovascular disease. In an embodiment, the method includes treating left ventricular hypertrophy associated with CKD without raising serum calcium. In such embodiment, the compound or salt therof of Formula (I) preferably is Vida-4, Vida-5, or Vida-10.

In an embodiment of the invention, the method includes treating hypocalcemia, proteinuria, endothelial dysfunction, and/or left ventricular hypertrophy with a compound of salt thereof of Formula (I), in which R⁴ and R⁵ are each hydrogen, X is oxygen, a is 0, and R⁶ is C₁₋₁₂ hydroxyalkyl. In addion for certain preferred embodiments of this method, R¹ is cyclopropyl or =CH₂.

In an embodiment of the invention, the method includes treating a viral infection (e.g., influenza), a bacterial infection (e.g., tuberculosis), or musculoskeletal disorder with a compound of salt thereof of Formula (I), as described herein.

Specific examples of cancers include cancer of the head and neck, eye, skin, mouth, throat, esophagus, chest, bone, lung, colon, sigmoid, rectum, stomach, prostate, breast, ovaries, kidney, liver, pancreas, brain, intestine, heart or adrenals. More particularly, cancers include solid tumor, sarcoma, carcinomas, fibrosarcoma, myxosarcoma, liposarcoma, chondrosarcoma, osteogenic sarcoma, chordoma, angiosarcoma, endotheliosarcoma, lymphangiosarcoma, lymphangioendothelio sarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, Kaposi's sarcoma, pinealoma, hemangioblastoma, acoustic neuroma, oligodendroglioma, menangioma, melanoma, neuroblastoma, retinoblastoma, a blood-born tumor, acute lymphoblastic leukemia, acute lymphoblastic B-cell leukemia, acute lymphoblastic T-cell leukemia, acute myeloblastic leukemia, acute promyelocytic leukemia, acute monoblastic leukemia, acute erythroleukemic leukemia, acute megakaryoblastic leukemia, acute myelomonocytic leukemia, acutenonlymphocyctic leukemia, acute undifferentiated leukemia, chronic myelocytic leukemia, chronic lymphocytic leukemia, hairy cell leukemia, or multiple myeloma. See, e.g., Harrison's Principles of Internal Medicine, Eugene Braunwald et al., eds., pp. 491-762 (15th ed. 2001).

In a preferred embodiment, the compound or salt therof of Formula (I) is the compound referred to herein as Vida-11, which is administered to a subject in need thereof to treat hypocalcemia and optionally reduce proteinuria associated with CKD, improve endothelial function associated with CKD, and/or treat left ventricular hypertrophy associated with CKD.

The following examples further illustrate the invention but, of course, should not be construed as in any way limiting its scope.

### EXAMPLE 1

This example describes the synthesis of (*1R,3R,4S,5R*)*-*3-(*tert-*butyldimethylsilyloxy)-1,4-dihydroxy-6-oxa-bicyclo[3.2.1]octan-7-one, which is an intermediate of a compound of Formula (I), in an embodiment of the invention.

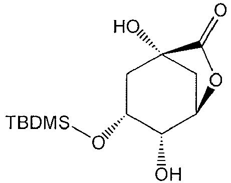

According to the method described in the literature (Elliot et al., 1981, J. Chem. Soc. Perkin Trans 1, 1782-1789), the above compound was prepared from (-)-quinic acid (9.6g, 0.05 mmol), *p*-toluene sulfonic acid monohydrate (500mg) (*p*-TosOH) in 150 mL of toluene. The solution was gently refluxed for 12 h, and the solvent was concentrated *in vacuo.* The obtained residue was dissolved in 75 mL of *N*,*N*'-dimethylformamide (DMF) and cooled in an ice bath. Imidazole (12.9g, 0.185mol), followed by *tert*-butyldimethylsilyl chloride (TBDMS-Cl) (8.68g, 0.057mol) were added. The mixture was stirred at 0 °C for 0.5 h, and at room temperature for 1 h. The mixture was poured into water, and the product was extracted by ethyl acetate (EtOAc). The organic layer was washed several times with water and dried over anhydrous magnesium sulfate (MgSO₄). After evaporated to dryness, the obtained product was recrystallized from petroleum ether/EtOAc to obtain 4.9 g of the title product. ¹H NMR (400 MHz, CDCl₃) δ 4.88 (1H, t, J = 5.4 Hz), 3.98 (1H, t, J = 4.4 Hz), 3.89 (1H, ddd, J = 11.7, 7.3, 4.4 Hz), 2.97 (1H, s), 2.85 (1H, s), 2.63 (1H, d, J = 11.7 Hz), 2.30 (1H, ddd, J = 11.2, 5.8, 2.4 Hz), 2.06-1.94 (2H, m), 0.90 (9H, s), 0.097 (6H, s).

### EXAMPLE 2

This example describes the synthesis of (*1R,3R,5R*)-3-(*tert-*butyldimethylsilyloxy)-1-hydroxy-6-oxa-bicyclo[3.2.1]octane-4,7-dione, which is an intermediate of a compound of Formula (I), in an embodiment of the invention.

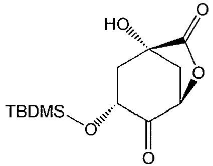

According to the method described in the literature (Glebocka et al., J. Med. Chem., 2006, 49, 2909-2920), the title compound was prepared as follows. The product of Example 1 (8.95g, 0.03mol) was added to a stirred suspension of Dess-Martin Reagent periodinane reagent (14.8g, 0.0345mol) in 225 mL of methylene chloride (CH₂CH₂). After stirring at room temperature for 18 h, it was poured into water. The product was extracted with EtOAc, and the EtOAc layer was washed several times with water, dried over MgSO₄, and then evaporated to dryness. The crystalline product (8.0g) was obtained in 93.2% yield. ¹H NMR (400 MHz, CDCl₃) δ 4.74 (1H, d, J = 6.3 Hz), 4.54 (1H, dd, J = 10.7, 9.3 Hz), 2.96 (1H, s), 2.86 (1H, ddd, J = 12.7, 6.8, 3.9 Hz), 2.5 (1H, ddd, J = 15.2, 6.4, 2.4 Hz), 2.42 (1H, d, J = 12.2 Hz), 2.16 (1H, dd, J = 12.2, 10.2 Hz), 0.90 (9H, s), 0.14 (3H, s), 0.044 (3H, s).

### EXAMPLE 3A

This example describes the synthesis of (1*R,*3*R,*5*R*)-3-(*tert-*butyldimethylsilyloxy)-4,7-dioxo-6-oxabicyclo[3.2.1]octan-1-yl acetate, which is an intermediate of a compound of Formula (I), in an embodiment of the invention.

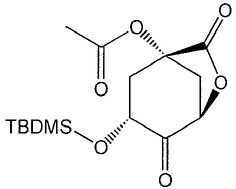

Acetic acid anhydride (24 mL) was added to a solution of Example 2 (7.0g, 24.5mmol) in 52 mL of anhydrous pyridine. After stirring at room temperature for 3 h, it was then poured into water, and the product was extracted with EtOAc. The EtOAc layer was washed with saturated sodium hydrogen carbonate (NaHCO₃), saturated cupric sulfate (CuSO₄), and water, then dried over MgSO₄. It was evaporated to dryness to yield an oily residue. The oily residue was purified by silica gel column chromatography, eluting with petroleum ether/EtOAc (4:1). Ultimately, 6.84g of the product was isolated as a crystalline form in 85.1% yield. ¹H NMR (400 MHz, CDCl₃) δ 4.81 (1H, d, J = 6.8 Hz), 4.58 (1H, dd, J = 10.3, 8.8 Hz), 3.56 (1H, ddd, J = 12.2, 6.8, 3.9 Hz), 2.66 (1H, ddd, J = 12.2, 8.8, 3.9 Hz), 2.32 (1H, d, J = 12.2 Hz), 2.29 (1H, dd, J = 12.2, 10.3 Hz), 2.17 (3H, s), 0.90 (9H, s), 0.14 (3H, s), 0.045 (3H,s).

### EXAMPLE 3B

This example describes the synthesis of (*1R,3R,5R*)-3-(*tert-*butyldimethylsilyloxy)-4-methylene-7-oxo-6-oxabicyclo[3.2.1]octan-1-yl acetate, which is an intermediate of a compound of Formula (I), in an embodiment of the invention.

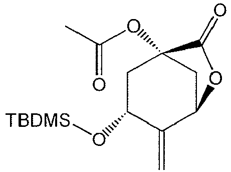

To a solution of methyl phosphonium bromide (3.93g, 0.011mol) in 168 mL of anhydrous tetrahydrofuran (THF) at 0°C, *n*-butyl lithium (2.5*M* in hexane, 4.4mL, 0.01mol) was dropwise added under nitrogen with stirring. The mixture was stirred at 20 °C for 20 min, and then cooled to -70 °C. A solution of the above compound (Example 3A, 2.80g, 8.54mmol) in 96mL of THF was added *via* syringe. The mixture was stirred at -40 °C to about -50 °C for 2 h. 1 %-Hydrochloric acid (22g), brine, 60mL of EtOAc, 40mL of benzene, 20mL of diethyl ether (Et₂O), 20mL of saturated NaHCO₃, and 20mL of water were added to the reaction mixture. The reaction mixture was vigorously stirred at room temperature for 18 h. The organic layer was separated, washed with brine, dried over MgSO₄, and evaporated to dryness. The compound was purified by silica gel column chromatography, eluting with petroleum ether/EtOAc (9:1) to yield the title compound (428mg) in 30.7% yield. ¹H NMR (400 MHz, CDCl₃) δ 5.32 (1H, s) 5.22 (1H, s), 5.20 (1H, s), 4.49 (1H, dd, J = 9.8, 7.8 Hz), 3.41 (1H, ddd, J = 10.7, 6.4, 3.0 Hz), 2.44 (1H, ddd, J = 11.7, 7.3, 2.9 Hz), 2.24-2.09 (2H, m), 2.20 (3H, s), 0.98 (9H, s), 0.15 (6H, s).

### EXAMPLE 4

This example describes the synthesis of (*1R,3R,5R*)-5-*(tert-*butyldimethylsilyloxy)-1-(hydroxymethyl)-4-methylenecyclohexane-1,3-diol, which is an intermediate of a compound of Formula (I), in an embodiment of the invention.

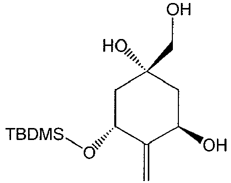

To a stirred solution of the product of Example 3B (1.24g, 3.43mmol) in 25mL of anhydrous ethyl alcohol (EtOH) at 0 °C was added sodium borohydride (1.30g, 34.3mmol), and the mixture was stirred at 0 °C for 1 h, at about 6 °C for 10 h and at room temperature for 2 h. After saturated ammonium chloride solution was added, the reaction mixture was poured into brine. The product was extracted several times with ether and CH₂Cl₂. Combined extracts were washed with brine, dried over MgSO₄, and evaporated to dryness. The compound was purified by silica gel column chromatography, eluting with hexane/EtOAc (2:8) to yield the title compound (0.5g) in 50.6% yield. ¹H NMR (400 MHz, CDCl₃) δ 5.14 (1H, t, J = 1.4 Hz), 4.98 (1H, s), 4.85 (1H, s), 4.80 (1H, s, br), 4.67 (1H, t, J = 3.2 Hz), 3.43 (1H, d, J = 11.0 Hz), 3.33 (1H, t, J = 7.3 Hz), 2.25 (2H, ddd, J = 11.9, 5.0, 2.3 Hz), 2.14 (1H, dt, J = 14.2, 2.8 Hz), 1.73 (1H, br), 1.54 (1H, dd, J = 14.2, 2.8 Hz), 0.89 (9H, s), 0.12 (3H, s), 0.087 (3H, s).

### EXAMPLE 5

This example describes the synthesis of (*3R*,*5R*)-3-(*tert*-butyldimethylsilyloxy)-5-hydroxy-4-methylenecyclohexanone, which is an intermediate of a compound of Formula (I), in an embodiment of the invention.

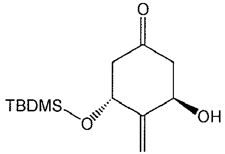

Sodium periodate-saturated water (13mL) of was added to a solution of the product in Example 4 (650mg, 2.26mmol) in 50mL of methanol at 0 °C. After stirring at 0 °C for 1 h, the reaction mixture was poured into brine, and the product was extracted with EtOAc and Et₂O. The combined organic layer was washed with brine, dried over MgSO₄, and evaporated to dryness. The residue was purified by silica gel column chromatography, eluting with hexane/EtOAc (7:3) to obtain the title compound (542mg) in 93.7% yield. ¹H NMR (400 MHz, CDCl₃) δ 5.24 (2H, d, J = 1.0 Hz), 4.77 (1H, dd, J = 6.4, 5.9 Hz), 4.71 (1H, dd, J = 6.4, 5.4 Hz), 2.77 (1H, ddd, J = 14.6, 4.9, 1.4 Hz), 2.67 (1H, ddd, J = 14.2, 4.4, 1.4 Hz) 2.51 (1H, ddd, J = 6.8, 2.9, 1.4 Hz), 2.47 (1H, ddd, J = 7.3, 3.4, 1.5 Hz) 0.87 (9H, s), 0.080 (3H, s), 0.060 (3H, s).

### EXAMPLE 6

This example describes the synthesis of ((3*R*,5*R*)-3,5-bis(*tert-*butyldimethylsilyloxy)-4-methylenecyclohexanone), which is an intermediate of a compound of Formula (I), in an embodiment of the invention.

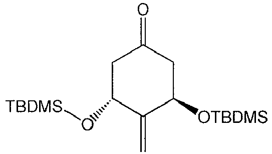

To a solution of Example 5 (178mg, 0.694mmol) in 5mL of CH₂Cl₂ at -50 °C was added 2,6-lutidine (190µL, 1.62mmol) and *tert*-butyldimethylsilyl-*O*-triflate (332 µL, 1.42mmol). It was stirred at -50 °C for 5 min, and at -15 °C for 30 min. Benzene and water were added to the reaction mixture, and the mixture was poured into water. The product was extracted with benzene, and the organic layer was washed with saturated CuSO₄, dried over MgSO₄, and then evaporated to dryness. Purification was carried out by using silica gel, eluting with hexane/EtOAc (95:5) to obtain 123mg of the title compound in 46.6% yield. ¹H NMR (400 MHz, CDCl₃) δ 5.16 (2H, s), 4.70-4.67 (2H, m), 2.64 (2H, ddd, J = 14.2, 4.4, 1.4Hz), 2.45 (2H, ddd, J = 14.2, 7.3, 2.0 Hz), 0.88 (18H, s), 0.068 (6H, s), 0.050 (6H, s).

### EXAMPLE 7

This example describes the synthesis of methyl-2-((3*R*,5*R*)-3,5-bis(*tert-*butyldimethylsilyloxy)-4-methylenecyclohexylidene)acetate), which is an intermediate of a compound of Formula (I), in an embodiment of the invention.

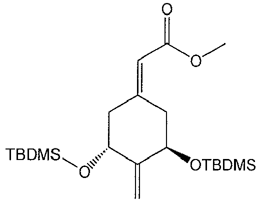

*n*-Butyl lithium (2.5*M* solution in hexane, 1.95mL, 4.88mmol) was added to a solution of diisopropylamine (0.72 mL, 5.10mmol) in 10mL of THF at -78 °C with stirring. Methyl(trimethylsilyl)acetate (0.85 mL, 5.18mmol) was then added. After stirred for 15 min, the product of Example 6 (218mg, 0.59mmol) in THF was added and the mixture was stirred at -78 °C for 2 h. The reaction was quenched by an addition of wet ether, and poured into brine. It was extracted with Et₂O and benzene. The combined organic layer was washed with brine, dried over MgSO₄, and concentrated *in vacuo.* Purification was carried out by using silica gel and eluting with hexane/EtOAc (50:1) to obtain 175mg of the title compound in 69.6% yield. ¹H NMR (400 MHz, CDCl₃) δ 5.74 (1H, s), 4.99 (2H, s), 4.48 (2H, dd, J = 10.3, 5.4 Hz), 3.68 (3 H, s), 3.06 (1H, dd, J = 13.7, 6.8 Hz), 2.98 (1H, dd, J = 13.2, 4.0 Hz), 2.47 (1H, dd, J = 12.7, 3.9 Hz), 2.25 (1H, dd, J = 12.7, 7.3 Hz), 0.88 (9H, s), 0.86 (9H, s), 0.072 (3H, s), 0.061 (3H, s), 0.035 (6H, s).

### EXAMPLE 8

This example describes the synthesis of (2-((3*R*,5*R*)-3,5-bis(*tert-*butyldimethylsilyloxy)-4-methylenecyclohexylidene)ethanol), which is an intermediate of a compound of Formula (I), in an embodiment of the invention.

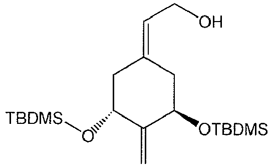

DIBAL-H (1.0*M* solution in toluene, 2.4mL, 2.4mmol) was slowly added to a solution of Example 7 (100mg, 0.234mmol) in 6mL of toluene/CH₂Cl₂ (2:1) at -78 °C under argon. After stirring at -78 °C for 1 h, potassium sodium tartrate (2M, 14mL), hydrochloric acid (2M, 14mL) and water (18mL) were added to the mixture. The reaction mixture was diluted with benzene and Et₂O. The organic layer was separated, washed with diluted NaHCO₃ and brine, dried over MgSO₄, and *concentrated in vacuo.* The crude material was purified by silica gel column chromatography, eluting with hexane/Et₂O (95:5) to obtain 50mg of the title compound in 85.9% yield. ¹H NMR (400 MHz, CDCl₃) δ 5.69 (1H, t, J = 7.4 Hz), 5.05 (1H, s), 4.95 (1H, s), 4.48 (1H, t, J = 4.4 Hz), 4.37 (1H, dd, J = 8.8, 4.9 Hz), 4.17 (1H, ddd, J = 11.7, 7.3, 3.4 Hz), 4.07-4.01 (1H, m), 2.51 (1H, dd, J = 13.2, 5.4 Hz), 2.45 (1H, dd, J = 12.2, 4.4 Hz), 2.22 (1H, dd, J = 14.2, 3.4 Hz), 2.12 (1H, dd, J = 12.2, 8.8 Hz), 0.90 (9H, s), 0.87 (9H, s), 0.073 (6H, s), 0.068 (6H, s).

### EXAMPLE 9

This example describes the synthesis of (1*R*,3*R*)-5-(2-(diphenylphosphoryl)ethylidene)-2-methylene-1,3 -(di-tert-butyldimethylsilyloxy)-cyclohexane, which is an intermediate of a compound of Formula (I), in an embodiment of the invention.

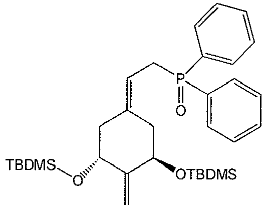

*n*-Buthyl lithium(*n*-BuLi, 2.5*M* in hexane, 50µL, 0.2mmol) was added to a solution of Example 8 (50mg, 0.2mmol) in 2mL of THF at 0 °C under argon. Freshly recrystallized *p*-toluene sulfonyl chloride (tos-Cl, 40.1mg, 0.21mmol) in 2mL of THF was added to the above solution. The mixture was stirred at 0 °C for 5 min. *n*-BuLi (2.5M solution in hexane, 160µL, 0.4mmol) was carefully added to a solution of diphenylphosphine (71 µL, 0.38mmol) in 5mL of THF under argon. The reddish solution was added to the above solution and stirred until the orange color remained. After stirring for an additional 40min at 0 °C, water (2.1mL) was added. Solvents were *concentrated in vacuo,* and the residue was dissolved in 3mL of CH₂Cl₂. Hydrogen peroxide (10% solution, 1.5mL) was added at 0 °C, and the mixture was stirred for 1 h. The separated organic layer was washed with cold aq. sodium sulfite and water. After evaporated to dryness, the crude material was purified by silica gel column chromatography, eluting with petroleum ether/EtOAc (85:15) to obtain 89mg of the title compound in 78.5% yield. ¹H NMR (400 MHz, CDCl₃) δ 7.76-7.68 (4H, m), 7.54-7.50 (2H,m), 7.48-7.44 (4H, m), 5.35 (1H, dd, J = 14.2, 6.8 Hz), 4.92 (2H, d, J = 12.7 Hz), 4.34 (2H, dd, J = 10.8, 4.9 Hz), 3.24-3.03 (2H, m), 2.33 (1H, dt, J = 12.7, 2.9 Hz), 2.08 (1H, ddd, J = 12.7, 7.8, 4.4 Hz), 2.02 (2H, d, J = 4.4Hz), 0.86 (18H, s), 0.016 (6H, s), 0.007 (3H, s), -0.004 (3H, s).

### EXAMPLE 10

This example describes the synthesis of ((*1R,3R*)-5-((*E*)-2-(1-(1-((*S*)-3-(*tert-*butyldimethylsilyloxy)-2,3-dimethylbutoxy)ethyl)-7a-methyldihydro-*1H*-inden-4(*2H,5H,6H,7H,7αH*)-ylidene)ethylidene)-2-methylenecyclohexane-1,3-diyl)bis(oxy)bis(tert-butyldimethylsilane), which is an intermediate of a compound of Formula (I), in an embodiment of the invention.

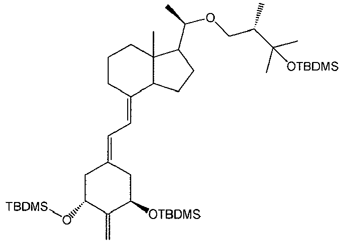

To a solution of Example 9 (20mg, 0.0343mmol) in THF at -78 °C was slowly added *tert*-BuLi (1.6M in pentane, 25 µL, 0.04mmol) under argon. The mixture was stirred at -78 °C for 20 min. The compound of Example 29 (16 mg, 0.04mmol) in 0.2mL of THF was slowly added. The mixture was stirred at -78 °C for 3 h and at about 6 °C for 16 h. EtOAc and water were added to the mixture. The organic layer was separated, washed with brine, dried over MgSO₄, and evaporated to dryness. The crude material was purified by preparative thin layer chromatography (prep-TLC) using petroleum ether/Et₂O (100:1) to obtain 10mg of the title compound in 37.6% yield. ¹H NMR (400 MHz, CDCl₃) δ 6.23 (1H, d, J = 11.2 Hz), 5.81 (1H, d, J = 11.2 Hz), 4.97 (1H, s), 4.92 (1H, s), 4.42 (2H, dd, J = 8.8, 3.9 Hz), 3.51 (1H, dd, J = 8.8, 3.0 Hz), 3.25-3.19 (2H, m), 2.83 (1H, d, J = 13.2 Hz), 2.51 (1H, dd, J = 13.2, 5.9 Hz), 2.46 (1H, dd, J = 12.7, 4.4 Hz), 2.33 (1H, dd, J = 12.7, 2.5 Hz), 2.22-2.13 (2H, m), 2.01 (1H, t, J = 9.8 Hz), 1.78-1.10 (10H, m), 1.20 (3H, s), 1.12 (3H, s), 1.06 (3H, d, J = 5.8 Hz), 0.95 (3H, d, J = 6.8 Hz), 0.90 (9H,s), 0.86 (9H, s), 0.85 (9H, s), 0.55 (3H, s), 0.080 (3H, s), 0.069 (6H, s), 0.065 (3H, s), 0.049 (3H, s), 0.025 (3H, s).

### EXAMPLE 11

This example describes the synthesis of (*1R,3R*)-5-((*E*)-2-((*3αS,7aS*)-1-((*R*)-1-((*S*)-3-hydroxy-2,3-dimethylbutoxy)ethyl)-7α-methyldihydro-*1H*-inden-4(*2H,5H,6H,7H,7αH*)-ylidene)ethylidene)-2-methylenecyclohexane-1,3-diol (Vida-5) in an embodiment of the invention.

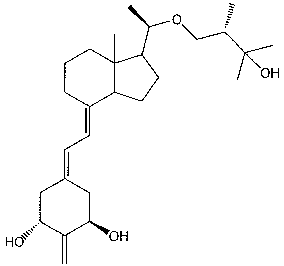

The product of Example 10 (20mg, 0.0258mmol) and tri(*n*-butyl)ammonium fluoride (518mg, 1.55mmol) were dissolved in 5mL of THF, and the mixture was stirred at room temperature for 18 h under nitrogen. The mixture was poured into brine, and the product was extracted with EtOAc. The organic layer was washed with brine, dried over MgSO4, and evaporated to dryness. The crude material was purified by preparative thin layer chromatography (prep-TLC) using Et₂O to obtain 10mg of the title compound in 59.7% yield. MS: m/z (%) 455 (19) [M + Na]⁺, 315 (34), 297 (100), 279 (49), 149 (56), 74 (91), 59 (43). ¹H NMR (400 MHz, CDCl₃) δ 6.36 (1H, d, J = 11.2 Hz), 5.86 (1H, d, J = 11.2 Hz), 5.10 (2H, d, J = 6.4 Hz), 4.52-4.42 (2H, m), 3.77 (1H, dd, J = 9.8, 4.4 Hz), 3.70 (1H, s, br), 3.32 (1H, dd, J = 9.3, 5.4 Hz), 3.27 (1H, dd, J = 9.8, 5.9 Hz), 2.84 (1H, dd, J = 13.7, 4.9 Hz), 2.81 (1H, dd, J = 10.8, 3.9 Hz), 2.57 (1H, dd, J = 13.2, 3.9 Hz), 2.35-2.27 (2H, m), 2.14 (1H, d, J = 12.2 Hz), 2.03 (1H, t, J = 8.3 Hz), 1.81-1.41 (10H, m), 1.24 (3H, s), 1.16 (3H, s), 1.13 (3H, d, J = 5.9 Hz), 1.00 (3H, d, J = 7.3 Hz), 0.57 (3H, s).

### EXAMPLE 12

This example describes the synthesis of (*4R*,*8R*)-4-(*tert*-butyldimethylsilyloxy)-8-hydroxyspiro[2.5]octan-6-one, which is an intermediate of a compound of Formula (I), in an embodiment of the invention.

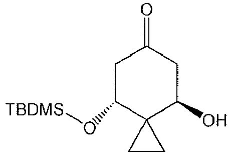

To a solution of Example 5 (888mg, 3.47mmol) in 10mL of toluene at -40 °C to -20°C under nitrogen was added diethyl zinc (1*M* solution in toluene, 8.33mL, 8.33mmol). After stirring for 10 min, CH₂I₂ (0.67mL, 8.33mmol) was added, and stirred at 0 °C for 3 h. The mixture was treated with saturated ammonium chloride, and the product was extracted with Et₂O. The ethereal solution was washed with saturated ammonium chloride, dried over MgSO₄, and the filtrate was evaporated to dryness. It was purified by silica gel column chromatography, eluting with petroleum ether/EtOAc (5:1) to obtain 870mg of the title compound in 92.8% yield. ¹H NMR (400 MHz, CDCl₃) δ 4.02 (1H, dd, J = 7.8, 4.4 Hz), 3.81 (1H, dd, J = 5.4, 4.4 Hz), 2.70-2.63 (2H, m), 2.45 (1H, ddd, J = 14.2, 5.9, 1.5 Hz), 2.40 (1H, ddd, J = 13.7, 7.8, 1.0 Hz), 0.89-0.78 (1H, m), 0.83 (9H, s), 0.69-0.64 (1H, m), 0.63-0.58 (1H, m), 0.49-0.44 (1H, m), 0.017 (6H, s).

### EXAMPLE 13

This example describes the synthesis of (*4R*,*8R*)-4,8-bis(*tert-*butyldimethylsilyloxy)spiro[2.5]octan-6-one, which is an intermediate of a compound of Formula (I), in an embodiment of the invention.

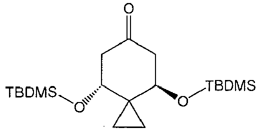

The title compound was prepared by the method described in Example 6 in 91.3% yield. This compound was used for the next reaction without further purification or characterization.

### EXAMPLE 14

This example describes the synthesis of methyl 2-((*4R,8R*)-4,8-bis(*tert-*butyldimethylsilyloxy)spiro[2.5]octan-6-ylidene)acetate, which is an intermediate of a compound of Formula (I), in an embodiment of the invention.

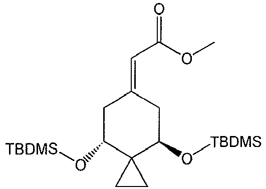

The title compound was prepared by the method described in Example 7 in 52.4% yield. ¹H NMR (400 MHz, CDCl₃) δ 5.71 (1H, s), 3.75 (1H, dd, J = 7.3, 3.7 Hz), 3.68 (3H, s), 3.62 (1H, t, J = 5.5 Hz), 2.99 (2H, d, J = 4.6 Hz), 2.44 (1H, dd, J = 13.8, 3.7, 0.9 Hz), 2.20 (1H, dd, J = 12.4, 7.3, 0.9 Hz), 0.85 (9H, s), 0.84 (9H, s), 0.58-0.50 (2H, m), 0.45-0.33 (2H, m), 0.044 (3H, s), 0.024 (3H, s), 0.009 (3H, s), 0.004 (3H, s).

### EXAMPLE 15A

This example describes the synthesis of 2-((*4R,8R*)-4,8-bis(*tert-*butyldimethylsilyloxy)spiro[2.5]octan-6-ylidene)ethanol, which is an intermediate of a compound of Formula (I), in an embodiment of the invention.

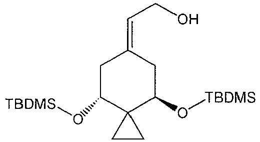

The title compound was prepared by the method described in Example 8 in 90.1 % yield. This compound was used for the next reaction without further purification or characterization.

### EXAMPLE 15B

This example describes the synthesis of (4R,8R)-6-(2-(diphenylphosphoryl)ethylidene)-4,8-(di-tert-butyldimethylsilyloxy)-spiro[2.5]octane, which is an intermediate of a compound of Formula (I), in an embodiment of the invention.

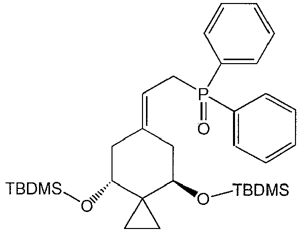

The title compound was prepared by the method described in Example 8 in 90.1 % yield. ¹H NMR (400 MHz, CDCl₃) δ 7.77-7.68 (4H, m), 7.55-7.50 (2H, m), 7.49-7.43 (4H, m), 5.29 (1H, dd, J = 14.6, 6.8 Hz), 3.57 (1H, dd, J = 6.9, 3.2 Hz), 3.52 (1H, dd, J = 6.9, 3.7Hz), 3.23-3.02 (2H, m), 2.30 (1H, dt, J = 12.8, 2.8 Hz), 2.10-2.01 (2H, m), 1.92 (1H, dd, J = 12.4, 6.4 Hz), 0.83 (9H, s), 0.81 (9H, s), 0.47-0.40 (2H, m), 0.36-0.25 (2H, m), -0.019 (3H, s), -0.032 (3H, s), -0.037 (3H, s), -0.041 (3H, s).

### EXAMPLE 16

This example describes the synthesis of ((4*R*,8*R*)-6-((*E*)-2-(1-(1-(3-ethyl-3-(triethylsilyloxy)pentyloxy)ethyl)-7α-methyldihydro-*1H*-inden-4(*2H,5H*,*6H*,*7H*,*7αH*)-ylidene)ethylidene)spiro[2.5]octane-4,8-diyl)bis(oxy)bis(*tert*-butyldimethylsilane) in an embodiment of the invention.

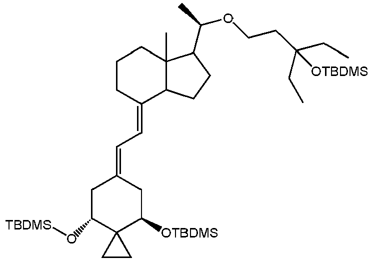

The title intermediate compound was prepared by the method described in Examples 9 and 10, by substituting the compound of Example 9 for Example 15B and the compound of Example 28 for Example 34. ¹H NMR (400 MHz, CDCl₃) δ 6.18 (1H, d, J = 10.8 Hz), 5.81 (1H, d, J = 11.2 Hz), 3.75 (1H, dd, J = 7.4, 3.0 Hz), 3.63 (1H, dd, J = 16.6, 8.3 Hz), 3.51-3.44 (1H, m), 3.31-3.22 (2H, m), 2.83 (1H, d, J= 12.7 Hz), 2.46-2.35 (3H, m), 2.17-2.11 (2H, m), 2.01 (2H, t, J = 9.3 Hz), 1.78-1.40 (8H, m), 1.36-1.29 (2H, m), 1.21 (4H, q, J = 7.3 Hz), 1.08 (3H, d, J = 5.4 Hz), 0.94 (9H, t, J = 7.8 Hz), 0.89-0.80 (6H, m), 0.847 (18H, s), 0.60-0.54 (1H, m), 0.57 (6H, q, J = 7.8 Hz), 0.56 (3H, s), 0.47-0.40 (2H, m), 0.28-0.24 (1H, m), 0.067 (6H, s), 0.042 (3H, s), 0.028 (3H, s).

### EXAMPLE 17

This example describes the synthesis of (4*R*,8*R*)-6-((*E*)-2-((1*S*,7*αS*)-1-((*R*)-1-(3-ethyl-3-hydroxypentyloxy)ethyl)-7α-methyldihydro-1H-inden-4(*2H,5H,6H,7H,7αH*)-ylidene)ethylidene)spiro[2.5]octane-4,8-diol (Vida-10) in an embodiment of the invention.

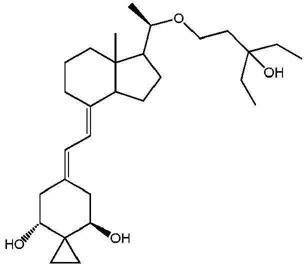

The title compound was prepared by the method described in Example 11. 96mg of Example 16 yielded 32mg of the title compound in 58.7% yield. MS: m/z (%) 483 (40) [M + Na]⁺, 301 (100), 293 (26), 267 (9), 122 (33). ¹H NMR (400 MHz, CDCl₃) δ 6.32 (1H, d, J = 11.0 Hz), 5.85 (1H, d, J = 11.0 Hz), 3.79 (1H, dd, J = 12.8, 9.2 Hz), 3.73 (1H, d, J = 4.2 Hz), 3.54 (1H, s), 3.46-3.37 (1H, m), 3.33 (1H, s), 3.28-3.22 (1H, m), 2.78 (2H, t, J = 15.3 Hz), 2.56 (1H, d, J = 12.8 Hz), 2.30-2.23 (2H, m), 2.12 (1H, d, J = 12.8 Hz), 2.01 (2H, t, J = 8.6 Hz), 1.83-1.37 (10H, m), 1.32-1.21 (6H, m), 1.12 (3H, d, J = 5.5 Hz), 0.85 (4H, q, J = 6.7 Hz), 0.71-0.65 (1H, m), 0.60-0.51 (2H, m), 0.55 (3H, s), 0.44-0.38 (1H, m).

### EXAMPLE 18A

This example describes the synthesis of 6-((1*R)*-1-((*E*)-4-(2-((*4R,8R*)-4,8-bis(*tert-*butyldimethylsilyloxy)spiro[2.5]octan-6-ylidene)ethylidene)-7a-methyloctahydro-1H-inden-1-yl)ethoxy)-3-ethylhexan-3-ol, which is an intermediate of a compound of Formula (I), in an embodiment of the invention.

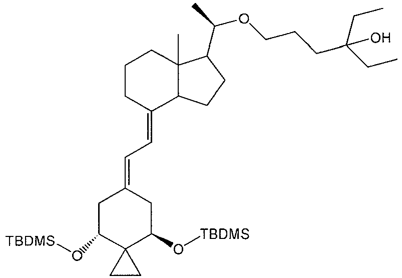

The title compound was prepared by the method described in Examples 9 and 10. ¹H NMR (400 MHz, CDCl₃) δ 6.18 (1H, d, J = 11.0 Hz), 5.81 (1H, d, J = 11.5 Hz), 3.75 (1H, dd, J = 7.76, 3.64 Hz), 3.59-3.52 (1H, m), 3.48 (1H, s, br), 3.31-3.21 (3H, m), 2.82 (1H, d, J = 12.8 Hz), 2.45-2.34 (3H, m), 2.16-2.12 (2H, m), 2.08-1.98 (2H, m), 1.75-1.23 (16H, m), 1.09 (3H, d, J = 5.5 Hz), 0.84 (18H, s), 0.84 (6H, t, J = 8.3 Hz), 0.58-0.51 (1H, m), 0.55 (3H, s), 0.45-0.40 (2H, m), 0.28-0.22 (1H, m), 0.04 (3H, s), 0.02 (3H, s), 0.00 (6H, s).

### EXAMPLE 18B

This example describes the synthesis of (*4R,8R*)-6-((*E*)-2-((*1S,7αS*)-1-((R)-1-(4-ethyl-4-hydroxyhexyloxy)ethyl)-7α-methyldihydro-*1H-*inden-4(*2H,5H,6H,7H,7αH*)-ylidene)ethylidene)spiro[2.5]octane-4,8-diol (Vida-20) in an embodiment of the invention.

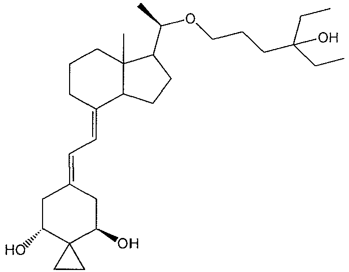

The title compound was prepared by the method described in Example 11, by substituting the compound of Example 9 for Example 15A and the compound of Example 28 for Example 37. MS: m/z (%) 497 (27) [M + Na]⁺, 421 (7), 311 (98), 293 (100), 267 (12), 122 (8). ¹H NMR (400 MHz, CDCl₃) δ 6.34 (1H, d, J = 11.4 Hz), 5.86 (1H, d, J = 11.4 Hz), 3.74 (1H, dd, J = 8.2, 4.1 Hz), 3.59-3.54 (2H, m), 3.31-3.19 (2H, m), 2.82 (1H, dd, J = 12.4, 4.1 Hz), 2.78 (1H, dd, J = 13.3, 3.7 Hz), 2.57 (1H, dd, J = 13.3, 3.2 Hz), 2.29 (1H, dd, J = 13.3, 2.8 Hz), 2.27 (1H, d, J = 13.3 Hz), 2.16 (1H, d, J = 12.4 Hz), 2.03 (1H, t, J = 9.6 Hz), 1.80-1.41 (17H, m), 1.33 (1H, td, J = 13.3, 4.1 Hz), 1.21-1.14 (1H, m), 1.09 (3H, d, J = 6.0 Hz), 0.55 (3H, t, J = 7.4 Hz), 0.85 (3H, t, J = 7.8 Hz), 0.71-0.66 (1H, m), 0.62-0.54 (2H, m), 0.56 (3H, s), 0.44-0.40 (1H, m).

### EXAMPLE 19

This example describes the synthesis of 1-(1-hydroxypropan-2-yl)-7α-methyloctahydro-*1H-*inden-4-ol, which is an intermediate of a compound of Formula (I), in an embodiment of the invention.

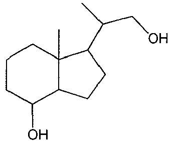

The title compound was prepared from Vitamin D2 according to the procedures described by Sardina et al. (1986, J. Org. Chem., 51(8): 1264-1269). A 2L three-necked dried flask equipped with a stir bar, gas inlet tube, and gas outlet tube was purged with argon. Vitamin D2 (20g, 0.05mol), 600 mL of CH₂Cl₂, 200mL of methanol, 300mg of NaHCO₃ were placed into the flask. The solution was cooled to -78 °C. Ozone was bubbled through the mixture for 2 days. Afterwards, the mixture was bubbled with argon until an ozone test showed negative. Sodium borohydride (10g, 0.275mol) was added, and the mixture was allowed to warm to room temperature overnight. 1*M* hydrochloric acid (200mL) was added to the mixture. CH₂Cl₂ was used to extract from the aqueous layer. The combined organic layer was washed with brine (100mL), dried over Na₂SO₄, and evaporated to dryness. The resulting syrup was purified by flash chromatography, eluting with petroleum ether and EtOAc (3:1) to obtain the title compound as a white solid (8.0g) in 75% yield. ¹H NMR (400 MHz, CDCl₃) δ 4.09 (1H, d, J = 2.4 Hz), 3.64 (1H, dd, J = 10.8, 3.4 Hz), 3.3 (1H, dd, J = 10.3, 6.4 Hz), 1.99 (1H, dt, J = 13.7, 3.0 Hz), 1.90-1.77 (3H, m), 1.64-1.40 (5H, m), 1.40-1.25 (3H, m), 1.22-1.14 (1H, m), 1.03 (3H, d, J = 6.4 Hz), 0.96 (3H, s).

### EXAMPLE 20

This example describes the synthesis of triethyl(7α-methyl-1-(1-(triethylsilyloxy)propan-2-yl)octahydro-*1H*-inden-4-yloxy)silane, which is an intermediate of a compound of Formula (I), in an embodiment of the invention.

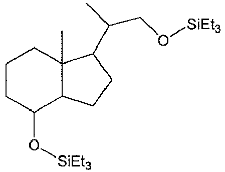

To a solution of Example 19 (6g, 0.028mol) in 200mL of THF at -78 °C was added 2,6-lutidine (15.14g, 0.14mol), followed by triethylsilyl-*O-p*-toluenesulfonate(16.4g, 0.062mol). After 20 min of stirring, 100mL of water was added to the mixture, and the reaction was warmed to room temperature. The compound was extracted with EtOAc (200mL x 2), and the organic layer was dried over Na₂SO₄, and evaporated to dryness. The residue was passed through a silica gel plug, eluting with petroleum ether to obtain the title compound (10g) as an oil in 80% yield. ¹H NMR (400 MHz, CDCl₃) δ 4.03 (1H, d, J = 2.4 Hz), 3.61 (1H, dd, J = 9.3, 3.4 Hz), 3.19 (1H, dd, J = 9.3, 8.3 Hz), 1.96 (1H, dt, J = 12.7, 2.5 Hz), 1.87-1.47 (4H, m), 1.43-1.00 (8H, m), 1.00-0.89 (24H, m), 0.61-0.49 (12H, m).

### EXAMPLE 21

This example describes the synthesis of 2-(7α-methyl-4-(triethylsilyloxy)octahydro-*1H-*inden-1-yl)propanal, which is an intermediate of a compound of Formula (I), in an embodiment of the invention.

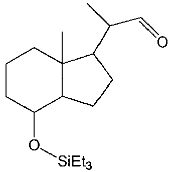

Oxalyl chloride (4.28g, 0.054mol) was added to a solution of dimethyl sulfoxide (DMSO, 5.26g, 0.27mol) in 10 mL of CH₂Cl₂ at -60 °C. After 2 min, pre-cooled Example 20 (11.9g, 0.027mol) was added *via* cannula, and the mixture was stirred at -60 °C for 1 h. Triethylamine (15mL) was added to the mixture, and the solution was allowed to warm to room temperature. Water was added, and the product was extracted with CH₂Cl₂ (3x50mL). The organic layer was dried over MgSO₄ and evaporated to dryness. The crude material was purified by flash column chromatography, eluting with petroleum ether/EtOAc (30:1) to obtain the title compound (7g) in 80% yield. ¹H NMR (400 MHz, CDCl₃) δ 9.56 (1H, d, J = 3.0 Hz), 4.06 (1H, d, J = 2.4 Hz), 2.40-2.31 (1H, m), 1.92-1.74 (3H, m), 1.73-1.50 (3H, m), 1.47-1.32 (4H, m), 1.30-1.14 (2H, m), 1.05 (3H, d, J = 6.9 Hz), 0.99-0.91 (12H, m), 0.56 (6H, q, J = 8.3 Hz).

### EXAMPLE 22

This example describes the synthesis of 1-(7α-methyl-4-(triethylsilyloxy)octahydro-*1H*-inden-1-yl)ethanone, which is an intermediate of a compound of Formula (I), in an embodiment of the invention.

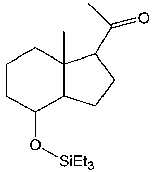

The product of Example 21 (6.6g, 0.02mol) and morpholine (5.3, 0.06mol) were heated in 200mL of toluene for overnight, while the resulting water was azeotropically removed. The mixture was concentrated *in vacuo,* and re-dissolved in 200mL of acetonitrile. After cuprous chloride (CuCl, 500mg) was added to the mixture, oxygen was bubbled through for 8 h. An insoluble material was removed by filtration, and the filtrate was evaporated to dryness. The crude was purified by flash column chromatography, eluting with petroleum ether/EtOAc (100:1) to obtain the title compound (2.5g) in 38.7% yield as an oil. ¹H NMR (400 MHz, CDCl₃) δ 4.07 (1H, d, J = 2.4 Hz), 2.47 (1H, t, J = 8.8 Hz), 2.25-2.14 (1H, m), 2.09 (3H, s), 2.01 (1H, dt, J = 11.7,3.0 Hz), 1.84 (1H, ddt, J = 26.9, 11.0,3.9 Hz), 1.75-1.67 (2H, m), 1.65-1.54 (1H, m), 1.50-1.35 (5H, m), 0.94 (9H, t, J = 7.8 Hz), 0.85 (3H, s), 0.55 (6H, q, J = 7.8 Hz).

### EXAMPLE 23

This example describes the synthesis of 1-(4-hydroxy-7a-methyloctahydro-*1H-*inden-1-yl)ethanone, which is an intermediate of a compound of Formula (I), in an embodiment of the invention.

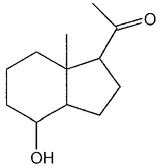

To a solution of the product of Example 22 (2.5g, 8.04mmol) in 200mL of THF was added tetra(*n*-butyl)ammonium fluoride (10.5g, 0.04mol). It was then stirred for 5 h, and the mixture was *concentrated in vacuo.* The crude product was purified by a silica gel plug, eluting with petroleum ether/EtOAc (4:1) to obtain the title compound in quantitative yield. ¹H NMR (400 MHz, CDCl₃ δ 4.13 (1H, d, J = 1.0 Hz), 2.50 (1H, t, J = 8.8 Hz), 2.28-2.18 (1H, m), 2.11 (3H, s), 2.08-2.03 (1H, m), 1.91-1.78 (2H, m), 1.74-1.41 (7H, m), 0.88 (3H, s).

### EXAMPLE 24

This example describes the synthesis of 1-acetyl-7a-methyloctahydro-*1H*-inden-4-yl acetate, which is an intermediate of a compound of Formula (I), in an embodiment of the invention.

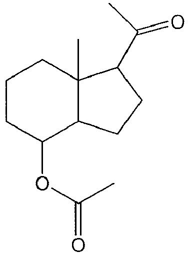

The product of Example 23 (1.3g, 6.6mmol) was dissolved in 40mL of CH₂Cl₂, and cooled to 0°C. *N,N-*dimethylaminopyridine (24.4mg, 0.2mmol), pyridine (1.15g, 14.5mmol), acetic anhydride (1.35g, 13.2mmol) were added, and stirred for 3 h while warming to room temperature. After the mixture was *concentrated in vacuo,* the crude product was purified by flash silica gel chromatography, eluting with petroleum ether/EtOAc (10:1) to obtain the title compound (1.4g) as an oil in 93.3 yield. ¹H NMR (400 MHz, CDCl₃) δ 5.18 (1H, d, J = 2.4 Hz), 2.50 (1H, t, J = 8.8 Hz), 2.22-2.14 (1H, m), 2.12 (3H, s), 2.09-2.05 (1H, m), 2.04 (3H, s), 1.89 (1H, dd, J = 14.2, 2.0 Hz), 1.80-1.42 (8H, m), 0.83 (3H, s).

### EXAMPLE 25

This example describes the synthesis of (*S*)-methyl 2-methyl-3-(trimethylsilyloxy)propanoate, which is an intermediate of a compound of Formula (I), in an embodiment of the invention.

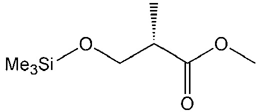

Methyl 3-hydroxy-(2S)-methyl-*n*-propanoate (Sigma-Aldrich Co., 2.1g, 17.8mmol) was dissolved in CH₂Cl₂. Et₃N (2.7g, 26.7mmol) and trimethylsilyl chloride (2.1 g, 19.6mmol) were added and stirred for 2 h. After cooling the reaction mixture to 0 °C, saturated NaHCO₃ and water were added to the mixture. The product was extracted with CH₂Cl₂, dried over Na₂SO₄, and evaporated to dryness. The crude product was purified by flash silica gel chromatography, eluting with hexane/EtOAc (20:1) to obtain the title compound (2.2g) as an oil in 65.1 yield. ¹H NMR (400 MHz, CDCl₃) δ 3.77 (1H, dd, J = 9.8, 6.8 Hz), 3.68 (3H, s), 3.60 (1H, dd, J = 9.8, 5.9 Hz), 2.69-2.61 (1H, m), 1.14 (3H, d, J = 7.3 Hz), 0.094 (9H, s).

### EXAMPLE 26

This example describes the synthesis of (*2S*)-methyl 3-(1-(4-acetoxy-7α-methyloctahydro-*1H*-inden-1-yl)ethoxy)-2-methylpropanoate, which is an intermediate of a compound of Formula (I), in an embodiment of the invention.

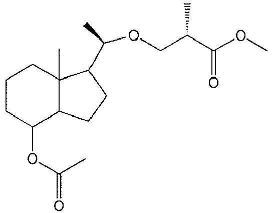

The products of Example 24 (1.4g, 5.88mmol) and Example 25 (1.45g, 7.65mmol) were dissolved in 40mL of CH₂Cl₂ cooled to -78 °C. Trimethylsilyl-O-triflate (1.3g, 5.88mmol) was added and stirred at -78 °C for 1 h. After triethylsilane (682mg, 5.88mmol) was added, the mixture was allowed to warm to room temperature and stirred for an additional 4 h. A saturated NaHCO₃ solution was carefully added and diluted with water. The product was extracted with CH₂Cl₂; the organic layer was dried over MgSO₄ and evaporated to dryness. The crude product was purified by flash silica gel chromatography, eluting with petroleum ether/EtOAc (10:1) to obtain the title compound (917mg) as an oil in 45.9% yield. ¹H NMR (400 MHz, CDCl₃) δ 5.15 (1H, s), 3.68 (3H, s), 3.56 (1H, dd, J = 8.8, 4.9 Hz), 3.40 (1H, t, J = 8.8 Hz), 3.32-3.25 (1H, m), 2.78-2.65 (1H, m), 2.04 (3H, s), 2.07-1.98 (1H, m), 1.85-1.81 (1H, m), 1.76-1.60 (3H, m), 1.52-1.17 (8H, m), 1.14 (3H, d, J = 6.8 Hz), 1.10 (1H, d, J = 2.9 Hz), 1.05 (3H, d, J = 5.8 Hz), 0.86 (3H, s).

### EXAMPLE 27

This example describes the synthesis of 1-(1-((*S*)-3-hydroxy-2,3-dimethylbutoxy)ethyl)-7α-methyloctahydro-*1H*-inden-4-ol, which is an intermediate of a compound of Formula (I), in an embodiment of the invention.

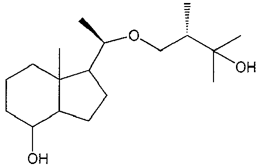

To a cooled solution of methylmagnesium bromide (19.3mL, 27mmol) in 20mL of THF was added the product of Example 26 (917mg, 2.7mmol) under nitrogen. After stirring overnight, the reaction mixture was quenched with aqueous ammonium chloride solution, followed by water. The product was extracted with EtOAc, and the organic layer was dried over MgSO₄ and evaporated to dryness to obtain the title compound (790mg) in 98.3% yield. ¹H NMR (400 MHz, CDCl₃) δ 4.09 (1H, d, J = 2.4 Hz), 3.77 (1H, dd, J = 9.8, 4.4 Hz), 3.73 (1H, s, br) 3.32 (1H, dd, J = 9.3, 5.8 Hz), 3.31-3.25 (1H, m), 2.13 (1H, d, J = 13.7 Hz), 1.81-1.77 (2H, m), 1.75-1.65 (2H, m), 1.56-1.30 (7H, m), 1.24 (3H, s), 1.20-1.18 (1H, m), 1.16 (3H, s), 1.11 (3H, d, J = 6.4 Hz), 1.01 (3H, d, J = 7.3 Hz), 0.96 (3H, s).

### EXAMPLE 28

This example describes the synthesis of 1-(1-((*S*)-3-hydroxy-2,3-dimethylbutoxy)ethyl)-7α-methylhexahydro-*1H*-inden-4(*2H*)-one, which is an intermediate of a compound of Formula (I), in an embodiment of the invention.

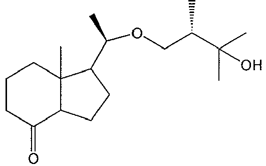

Dess-Martin reagent (1.57g, 3.7mmol) was added to a solution of the product of Example 27 (790mg, 2.65mmol) in 20mL of CH₂Cl₂. The mixture was stirred at room temperature for overnight. Saturated NaHCO₃ and Na₂S₂O₃ were added to the mixture, and the product was extracted with CH₂Cl₂. Organic layer was dried over Na₂SO₄, and *concentrated in vacuo.* The crude product was purified by flash silica gel chromatography, eluting with petroleum ether/EtOAc (40:1) to obtain the title compound (545mg) as an oil in 69.5% yield. ¹H NMR (400 MHz, CDCl₃) δ 3.80 (1H, dd, J = 9.8, 4.4 Hz), 3.51 (1H, s, br) 3.32 (1H, dd, J = 9.3, 5.4 Hz), 3.26 (1H, ddd, J = 15.2, 9.3, 5.9 Hz), 2.47 (1H, dd, J = 11.2, 7.3 Hz), 2.34-2.18 (3H, m), 2.07-1.99 (1H, m), 1.95-1.83 (1H, m), 1.81 1.68 (4H, m), 1.61-1.53 (2H, m), 1.25 (3H, s), 1.23-1.20 (1H, m), 1.17 (3H, s), 1.15 (3H, d, J = 5.8 Hz), 1.03 (3H, d, J = 7.3 Hz), 0.66 (3H, s).

### EXAMPLE 29

This example describes the synthesis of 1-(1-((*S*)-3-(tert-butyldimethylsilyloxy)-2,3-dimethylbutoxy)ethyl)-7α-methylhexahydro-*1H*-inden-4(*2H*)-one, which is an intermediate of a compound of Formula (I), in an embodiment of the invention.

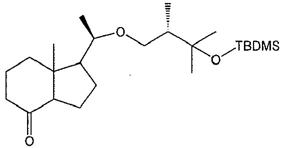

The title compound was prepared according to the method described in Example 6, except Example 27 was used instead of Example 5 as a starting material. A yield of 63.4% was observed. ¹H NMR (400 MHz, CDCl₃) δ 3.49 (1H, dd, J = 8.8, 3.0 Hz), 3.26 (1H, t, J = 8.8 Hz), 3.25-2.18(1H, m), 2.46 (1H, dd, J = 11.2, 7.3 Hz), 2.31-2.18 (3H, m), 1.93-1.82 (1H, m), 2.02-1.94 (1H, m), 1.80-1.69 (3H, m), 1.68-1.62 (1H, m), 1.57-1.52 (3H, m), 1.20 (3H, s),1.12 (3H, s), 1.07 (3H, d, J = 5.9 Hz), 0.95 (3H, d, J = 6.8 Hz), 0.85 (9H, s), 0.64 (3H, s), 0.069 (6H, s).

### EXAMPLE 30

This example describes the synthesis of methyl 3-hydroxypropanoate, which is an intermediate of a compound of Formula (I), in an embodiment of the invention.

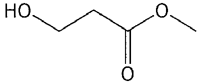

Sodium (115mg, 0.005mol) was carefully added to 50mL of methanol. 3-Hydroxy-*n*-propionic acid (Acros Co., 7.2g, 0.1mol) was added via syringe to the above solution. The reaction mixture was stirred at 50 °C overnight. After evaporating the mixture to dryness, the crude product was purified by flash silica gel chromatography, eluting with petroleum ether/EtOAc (0:1) to obtain the title compound (7.5g) as an oil in 72.1% yield. This compound was used for the next reaction without further purification or characterization.

### EXAMPLE 31

This example describes the synthesis of methyl 3-(trimethylsilyloxy)propanoate, which is an intermediate of a compound of Formula (I), in an embodiment of the invention.

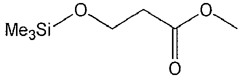

The title compound was prepared using the procedure described in Example 25. Example 30 was used instead of Example 24 as a starting material. 64.6% of yield was observed. ¹H NMR (400 MHz, CDCl₃) δ 3.87 (2H, t, J = 6.8 Hz), 3.69 (3H, s), 2.55 (2H, t, J = 6.4 Hz), 0.115 (9H, s).

### EXAMPLE 32

This example describes the synthesis of methyl 3-(1-(4-acetoxy-7α-methyloctahydro-*1H*-inden-1-yl)ethoxy)propanoate, which is an intermediate of a compound of Formula (I), in an embodiment of the invention.

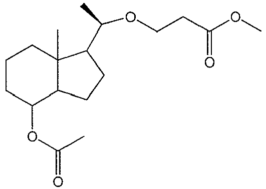

The title compound was prepared using the procedure described in Example 26, by substituting the products of Example 25 for Example 31 to yield 80.3% of the title product. ¹H NMR (400 MHz, CDCl₃ δ 5.15 (1H, s), 3.84-3.78 (1H, m), 3.69 (3H, s), 3.56-3.49 (1H, m), 3.35-3.29 (1H, m), 2.63-2.50 (2H, m), 2.04 (3H, s), 1.83 (1H, d, J = 12.2 Hz), 1.70-1.62 (2H, m), 1.50-1.24 (7H, m), 1.16-1.10 (2H, m), 1.07 (3H, d, J = 5.5 Hz), 0.88 (3H, s).

### EXAMPLE 33A

This example describes the synthesis of 1-(1-(3-ethyl-3-hydroxypentyloxy)ethyl)-7α-methyloctahydro-*1H*-inden-4-ol, which is an intermediate of a compound of Formula (I), in an embodiment of the invention.

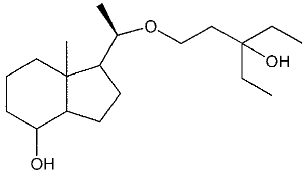

The title compound was prepared using the procedure described in Example 28, by substituting the product of Example 27 for Example 32. ¹H NMR (400 MHz, CDCl₃) δ 4.09 (1H, d, J = 2.9 Hz), 3.80 (1H, td, J = 9.2, 4.4 Hz), 3.43 (1H, dt, J = 8.8, 5.4 Hz), 3.36 (1H, s, br), 3.28 (1H, ddd, J = 12.2, 10.3, 5.9 Hz), 2.10 (1H, dt, J = 12.2, 1.5 Hz), 1.85-1.75 (2H, m), 1.75-1.29 (13H, m), 1.22-1.13 (2H, m), 1.11 (3H, d, J = 5.8 Hz), 0.95 (3H, s), 0.87 (3H, t, J = 7.4 Hz), 0.85 (3H, t, J = 7.3 Hz).

### EXAMPLE 33B

This example describes the synthesis of 1-(1-(3-ethyl-3-hydroxypentyloxy)ethyl)-7α-methylhexahydro-*1H*-inden-4(*2H*)-one, which is an intermediate of a compound of Formula (I), in an embodiment of the invention.

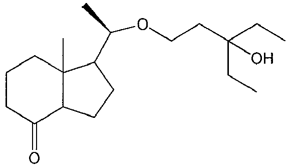

The title compound was prepared using the procedure described in Example 28, by substituting the products of Example 26 for Example 33A to yield 71.3% of the title product. ¹H NMR (400 MHz, CDCl₃) δ 3.82 (1H, td, J = 9.3, 4.4 Hz), 3.42 (1H, dt, J = 9.3, 4.9 Hz), 3.25 (1H, ddd, J = 11.8, 9.3, 5.9 Hz), 3.16 (1H, s), 2.46 (1H, dd, J = 11.2, 7.4 Hz), 2.30-2.17 (3H, m), 2.06-1.99 (1H, m), 1.95-1.42 (11 H, m), 1.28-1.16 (2H, m), 1.14 (3H, d, J = 5.9 Hz), 0.87 (3H, t, J = 7.3 Hz), 0.86 (3H, t, J = 7.4 Hz), 0.65 (3H, s).

### EXAMPLE 34

This example describes the synthesis of 1-(1-(3-ethyl-3-(triethylsilyloxy)pentyloxy)ethyl)-7α-methylhexahydro-*1H*-inden-4(*2H*) -one, which is an intermediate of a compound of Formula (I), in an embodiment of the invention.

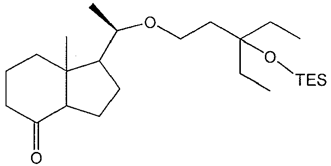

The title compound was prepared using the procedure described in Example 6, by substituting the products of Example 5 for Example 33B, and *tert*-butyldimethylsilyl chloride (TBDMS chloride) to triethylsilyl chloride to yield 71.3% of the title product. ¹H NMR (400 MHz, CDCl₃) δ 3.69-3.63 (1H, m), 3.30-3.23 (2H, m), 2.47 (1H, dd, J = 10.8, 6.8 Hz), 2.30-2.18 (3H, m), 2.04-1.95 (1H, m), 1.95-1.83 (1H, m), 1.79-1.66 (5H, m), 1.53-1.41 (4H, m), 1.41-1.13 (3H, m), 1.10 (3H, d, J = 5.8 Hz), 0.94 (9H, t, J = 7.8 Hz), 0.84 (6H, t, J = 7.3 Hz), 0.65 (3H, s), 0.58 (6H, q, J = 7.8 Hz).

### EXAMPLE 35A

This example describes the synthesis of methyl 4-hydroxybutanoate, which is an intermediate of a compound of Formula (I), in an embodiment of the invention.

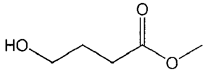

The title compound was prepared using the procedure described in Example 30, by substituting 3-hydroxy-*n*-propioninc acid for 4-hydroxy-*n*-butyric acid (Sinopharm Chemical Reagent Co., Ltd.) to yield 87.6% of the title compound. ¹H NMR (400 MHz, CDCl₃ δ 3.69 (2H, t, J = 6.1 Hz), 3.69 (3H, s), 2.45 (2H, t, J = 6.8 Hz), 1.93-1.86 (2H, m).

### EXAMPLE 35B

This example describes the synthesis of methyl 4-(trimethylsilyloxy)butanoate, which is an intermediate of a compound of Formula (I), in an embodiment of the invention.

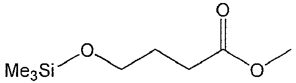

The title compound was prepared using the procedure described in Example 25, by substituting the starting material of Example 25 for the product of Example 35 A, to yield 71.3% of the title product. ¹H NMR (400 MHz, CDCl₃) δ 3.67 (3H, s), 3.61 (2H, t, J = 6.4 Hz), 2.39 (2H, t, J = 7.8 Hz), 1.88-1.81 (2H, m), 0.113 (9H, s).

### EXAMPLE 36

This example describes the synthesis of methyl 4-(1-(4-acetoxy-7α-methyloctahydro-*1H*-inden-1-yl)ethoxy)butanoate, which is an intermediate of a compound of Formula (I), in an embodiment of the invention.

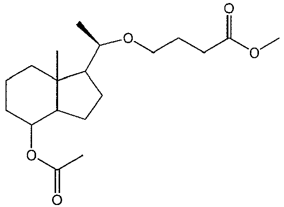

The title compound was prepared using the procedure described in Example 26, substituting the product of Example 25 for Example 35A to yield 71.8% of the title product. ¹H NMR (400 MHz, CDCl₃) δ 5.15 (1H, d, J = 2.4 Hz), 3.68 (3H, s), 3.59-3.53 (1H, m), 3.30-3.21 (2H, m), 2.41 (2H, t, J = 7.3 Hz), 2.04 (3H, s), 1.91-1.81 (2H, m), 1.74-1.61 (3H, m), 1.54-1.29 (7H, m), 1.21-1.08 (2H, m), 1.05 (3H, d, J = 6.4 Hz), 0.89 (3H, s).

### EXAMPLE 37

This example describes the synthesis of 1-(1-(4-ethyl-4-hydroxyhexyloxy)ethyl)-7α-methylhexahydro-*1H*-inden-4(*2H*)-one, which is an intermediate of a compound of Formula (I), in an embodiment of the invention.

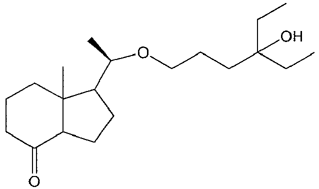

The title compound was prepared using the procedure described in Examples 27 and 28, substituting the product of Example 26 for Example 36, to yield 69.9% of the title product. ¹H NMR (400 MHz, CDCl₃) δ 3.58 (1H, dd, J = 14.7, 5.9 Hz), 3.30-3.20 (2H, m), 2.47 (1H, dd, J = 10.7, 7.3 Hz), 2.31 2.18 (3H, m), 2.03-1.95 (1H, m), 1.94-1.84 (1H, m), 1.81-1.69 (3H, m), 1.63-1.52 (3H, m), 1.51-1.44 (5H, m), 1.31-1.14 (3H, m), 1.10 (3H, d, J = 5.9 Hz), 0.86 (6H, t, J = 7.4 Hz), 0.65 (3H, s).

### EXAMPLE 38

This example describes the synthesis of ((*1R,3R*)-5-((*E*)-2-(1-(1-(3-ethyl-3-(triethylsilyloxy)pentyloxy)ethyl)-7α-methyldihydro-*1H*-inden-4(*2H,5H,6H, 7H,7αH*)-ylidene)ethylidene)-2-methylenecyclohexane-1,3-diyl)bis(oxy)bis(*tert*-butyldimethylsilane), which is an intermediate of a compound of Formula (I), in an embodiment of the invention.

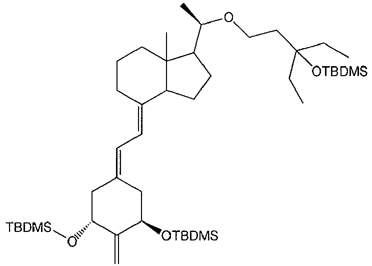

The title compound was prepared by the method described in Examples 9 and 10 in 27.3% yield. ¹H NMR (400 MHz, CDCl₃) δ 6.23 (1H, d, J = 11.2 Hz), 5.82 (1H, d, J = 11.2 Hz), 4.97 (1H, s), 4.92 (1H, s), 4.45-4.43 (2H, m), 3.67-3.59 (1H, m), 3.31-3.23 (2H, m), 2.83 (1H, d, J = 11.7 Hz), 2.51 (1H, dd, J = 13.7, 6.3 Hz), 2.46 (1H, dd, J = 13.7, 5.4 Hz), 2.33 (1H, dd, J = 13.7, 3.4 Hz), 2.21-2.13 (2H, m), 2.02 (1H, t, J = 9.3 Hz), 1.80-1.51 (11H, m), 1.47 (4, q, J=7.3 Hz), 1.09 (3H, d, J = 5.8Hz), 0.94 (9H, t, J = 7.8 Hz), 0.90 (9H, s), 0.87 (9H, s), 0.84 (6H, t, J = 7.3 Hz), 0.58 (6H, q, J = 7.8 Hz), 0.56 (3H, s), 0.081 (3H, s), 0.067 (3H, s), 0.050 (3H, s), 0.026 (3H, s).

### EXAMPLE 39

This example describes the synthesis of (*1R,3R*)-5-((*E*)-2-((*1S,3αS,7αS*)-1-((*R*)-1-(3-ethyl-3-hydroxypentyloxy)ethyl)-7α-methyldihydro-*1H*-inden-4(*2H,5H,6H,7H,7αH*)-ylidene)ethylidene)-2-methylenecyclohexane-1,3-diol (Vida-11) in an embodiment of the invention.

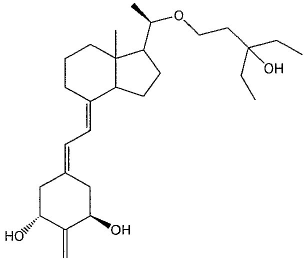

The title compound was prepared by the method described in Example 11 in 58.7% yield. MS: m/z (%) 469 (27) [M + Na]⁺, 315 (40), 297 (100), 279 (54), 149 (8). ¹H NMR (500 MHz, CDCl₃) δ 6.36 (1H, d, J = 11.2 Hz), 5.86 (1H, d, J = 11.2 Hz), 5.10 (2H, d, J = 7.8 Hz), 4.50-4.44 (2H, m), 3.80 (1H, td, J = 9.2, 4.5 Hz), 3.44-3.39 (1H, m), 3.30 (1H, s, br), 3.29-3.23 (1H, m), 2.82 (2H, td, J = 13.2, 4.5 Hz), 2.57 (1H, dd, J = 13.4, 3.9 Hz), 2.35-2.27 (2H, m), 2.12 (1H, d, J = 12.7 Hz), 2.02 (1H, t, J = 9.5 Hz), 1.90-1.41 (13H, m), 1.39-1.10 (2H, m), 1.13 (3H, d, J = 6.0 Hz), 0.86 (6H, t, J = 7.4 Hz), 0.56 (3H, s).

### EXAMPLE 40

This example describes the synthesis of 6-(1-((*E*)-4-(2-((*3R,5R*)-3,5-bis(tert-butyldimethylsilyloxy)-4-methylenecyclohexylidene)ethylidene)-7α-methyloctahydro-*1H-*inden-1-yl)ethoxy)-3-ethylhexan-3-ol, which is an intermediate of a compound of Formula (I), in an embodiment of the invention.

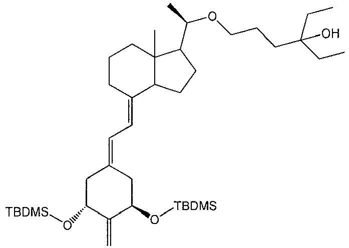

The title compound was prepared by the method described in Examples 9 and 10 in 15.8% yield. ¹H NMR (400 MHz, CDCl₃) δ 6.22 (1H, d, J = 11.0 Hz), 5.82 (1H, d, J = 11.0 Hz), 4.97 (1H, s), 4.92 (1H, s), 4.44-4.3 6 (2H, m), 3.56 (1H, dd, J = 14.6, 6.1 Hz), 3.28 (1H, dd, J = 9.8, 5.5 Hz), 3.25-3.18 (1H, m), 2.83 (1H, d, J = 11.6 Hz), 2.53-2.41 (2H, m), 2.37-2.30 (1H, m), 2.24-2.11 (2H, m), 2.02 (1H, t, J = 9.8 Hz), 1.79-1.10 (13H, m), 1.47 (4H, q, J = 4.3 Hz), 1.09 (3H, d, J = 5.5 Hz), 0.892 (9H, s), 0.86 (9H, s), 0.85 (6H, t, J = 7.3 Hz), 0.55 (3H, s), 0.076 (3H, s), 0.060 (3H, s), 0.046 (3H, s), 0.021 (3H, s).

### EXAMPLE 41

This example describes the synthesis of (*1R,3R*)-5-((*E*)-2-((*1S,7αS*)-1-((*R*)-1-(4-ethyl-4-hydroxyhexyloxy)ethyl)-7α-methyldihydro-*1H-*inden-4(*2H*,*5H,6H,7H,7αH*)-ylidene)ethylidene)-2-methylenecyclohexane-1,3-diol (Vida-21) in an embodiment of the invention.

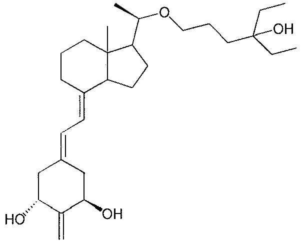

The title compound was prepared by the methods described in Examples 9-11 in 40.9% yield. MS: m/z (%) 483 (55) [M + Na]⁺, 315 (34), 297 (100), 279 (45), 149 (13), 122 (37). ¹H NMR (400 MHz, CDCl₃) δ 6.37 (1H, d, J = 11.0 Hz), 5.87 (1H, d, J = 11.0 Hz), 5.10 (2H, d, J = 6.7 Hz), 4.5 7-4.42 (2H, m), 3.57 (1H, dd, J = 8.6, 6.1 Hz), 3.33∼3.17 (2H, m), 2.89∼2.77 (2H, m), 2.58 (1H, dd, J = 13.4, 3.7 Hz), 2.36∼2.27 (2H, m), 2.24∼2.14 (1H, m), 2.06∼2.01 (2H, m), 1.86∼1.10 (12H, m), 1.49 (4H, q, J = 7.3 Hz), 1.09 (3H, d, J = 6.1 Hz), 0.85 (6H, t, J = 7.4 Hz), 0.56 (3H, s).

### EXAMPLE 42

This example describes the synthesis of 1-((R)-1-(3-hydroxy-3-methylbutoxy)ethyl)-7α-methyloctahydro-1H-inden-4-ol, which is an intermediate of a compound of Formula (I), in an embodiment of the invention.

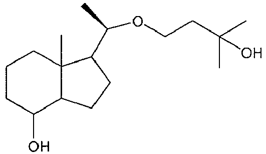

To a cooled (0 °C) solution of methyl magnesium bromide (19.7mL, 28mmol, 5 equiv. mol) in 20 mL of THF was added Example 32 (1.8g, 5.5mmol) under nitrogen. The mixture was stirred for 3 h. The reaction was quenched by additions of aqueous ammonium chloride solution and water. The product was extracted with EtOAc, the organic layer was dried over MgSO₄, and evaporated to dryness to obtain the title compound (1.1g) as a clear oil in 70.1 % yield. This compound was used in the next reaction without further purification or characterization.

### EXAMPLE 43

This example describes the synthesis of 1-((*R*)-1-(3-hydroxy-3-methylbutoxy)ethyl)-7α-methylhexahydro-1H-inden-4(2H)-one, which is an intermediate of a compound of Formula (I), in an embodiment of the invention.

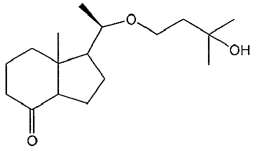

The title compound was prepared by the method described in the preparation of Example 28, by substituting the product of Example 27 for Example 42 in 89.1 % yield. This compound was used in the next reaction without further purification or characterization.

### EXAMPLE 44

This example describes the synthesis of 7α-methyl-1-(1-(3-methyl-3-(triethylsilyloxy)butoxy)ethyl)hexahydro-*1H*-inden-4(*2H*)-one, which is an intermediate of a compound of Formula (I), in an embodiment of the invention.

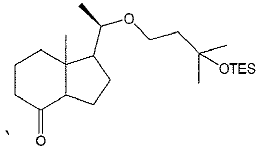

To a cooled (-78 °C) solution of Example 43 (980mg, 3.45mmol) in THF (20mL) was added 2,6-lutidine (1.85g, 17.3mmol) and triethylsilyl-*O*-triflate (TESOTf,1.64g, 6.21mmol). The resulting solution was stirred at -78 °C for 5 min, and then warmed to 0 °C for 30 min. It was thenextracted with CH₂Cl₂, dried over MgSO₄, and evaporated to dryness. The residue was purified by flash silica gel chromatography, eluting with petroleum ether/EtOAc (20:1) to obtain the title compound as a clear oil (900mg, 65.7%). ¹H NMR (400 MHz, CDCl₃) δ 3.72 (1H, dd, J = 16.5, 8.2 Hz), 3.35-3.23 (2H, m), 2.47 (1H, t, J = 8.2 Hz), 2.29-2.18 (3H, m), 2.03-1.95 (1H, m), 1.94-1.88 (1H, m), 1.74-1.70 (5H, m), 1.63-1.53 (3H, m), 1.24 (3H, s), 1.22 (3H, s), 1.10 (3H, d, J = 5.5 Hz), 0.94 (9H, t, J = 7.8 Hz), 0.65 (3H, s), 0.56 (6H, q, J = 7.8 Hz).

### EXAMPLE 45

This example describes the synthesis of ((*4R,8R*)-6-((*E*)-2-(7α-methyl-1-(1-(3-methyl-3-(triethylsilyloxy)butoxy)ethyl)dihydro-*1H*-inden-4(*2H,5H,6H,7H,7αH*)-ylidene)ethylidene)spiro[2.5]octane-4,8-diyl)bis(oxy)bis(*tert*-butyldimethylsilane), which is an intermediate of a compound of Formula (I), in an embodiment of the invention.

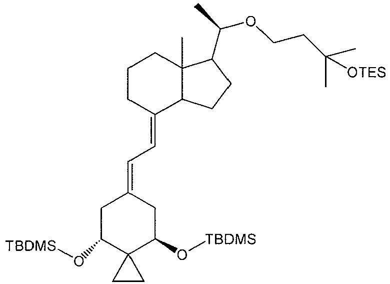

The compound of Example 15A (240mg, 0.4mmol) was dissolved in 10mL of THF and cooled to -78 °C under argon. *tert*-Butyl lithium (1.6M in pentane, 0.33mL, 0.52mmol) was slowly added to the stirring mixture. The reaction mixture was stirred at -78 °C for 20 min. The product of Example 44 (160mg, 0.4mmol)) in 2mL of THF was then added to the mixture. After stirring at -78 °C for 3 h and at 6°C for 16 h, EtOAc and water were added. The organic layer was separated, washed with brine, dried over MgSO₄, and was concentrated *in vacuo.* The crude material was purified by preparative thin layer chromatography, eluting with petroleum ether/Et₂O (50:1) to obtain 92 mg of the title compound in 29.7% yield. ¹H NMR (400 MHz, CDCl₃) δ 6.19 (1H, d, J = 11.0 Hz), 5.82 (1H, d, J = 11.4 Hz), 3.77-3.73 (1H, m), 3.71-3.60 (1H, m), 3.48 (1H, t, J = 5.5 Hz), 3.35-3.23 (2H, m), 2.83 (1H, d, J = 12.4 Hz), 2.46-2.35 (3H, m), 2.17-2.12 (2H, m), 2.01 (1H, t, J = 9.6 Hz), 1.78-1.69 (3H, m), 1.66-1.52 (6H, m), 1.48-1.30 (2H, m), 1.23 (3H, s), 1.21 (3H, s), 1.09 (3H, d, J = 6.0 Hz), 0.94 (9H, t, J = 7.8 Hz), 0.85 (18H, s), 0.59-0.53 (1H, m), 0.56 (6H, q, J = 7.8 Hz), 0.56 (3H, s), 0.47-0.39 (2H, m), 0.18-0.24 (1H, m), 0.069 (3H, s), 0.045 (3H, s), 0.029 (3H, s), 0.009 (3H, s).

### EXAMPLE 46

This example describes the synthesis of (*4R,8R*)-6-((*E*)-2-((1*S,*7*αS*)-1-((*R*)-1-(3-hydroxy-3-methylbutoxy)ethyl)-7α-methyldihydro-1H-inden-4(*2H,5H,6H,7H,7αH*)-ylidene)ethylidene)spiro[2.5]octane-4,8-diol (Vida-1) in an embodiment of the invention.

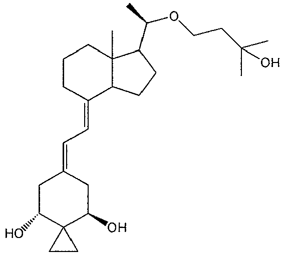

The title compound was prepared by the method described in Example 11. 92mg of Example 45 as the starting material yielded 23mg of the title compound in 44% yield. MS: m/z (%) 455 (36) [M + Na]⁺, 397 (10), 311 (67), 293 (100), 267 (26), 122 (45). ¹H NMR (400 MHz, CDCl₃) δ 6.33 (1H, d, J = 11.0 Hz), 5.86 (1H, d, J = 11.0 Hz), 3.88-3.81 (1H, m), 3.74 (1H, dd, J = 8.2, 4.1 Hz), 3.61 (1H, s, br), 3.55 (1H, dd, J = 5.5, 3.7 Hz), 3.48-3.43 (1H, m), 3.31-3.24 (1H, m), 2.93 (1H, t, J = 7.8 Hz), 2.83-2.74 (2H, m), 2.57 (1H, dd, J = 13.3, 2.8 Hz), 2.33-2.24 (2H, m), 2.12 (1H, d, J = 12.8 Hz), 2.02 (1H, t, J = 9.6 Hz), 1.89 (1H, ddd, J = 14.2, 9.6, 5.0 Hz), 1.80-1.48 (8H, m), 1.45-1.29 (1H, m), 1.24 (3H, s), 1.23 (3H, s), 1.14 (3H, d, J = 5.5 Hz), 0.97 (1H, t, J = 7.3 Hz), 0.73-0.66 (1H, m), 0.62-0.54 (2H, m), 0.56 (3H, s), 0.44-0.40 (1H, m).

### EXAMPLE 47

This example describes the synthesis of 1-((*R*)-1-((*S*)-2,3-dimethyl-3-(triethylsilyloxy)butoxy)ethyl)-7α-methylhexahydro-1H-inden-4(2H)-one, which is an intermediate of a compound of Formula (I), in an embodiment of the invention.

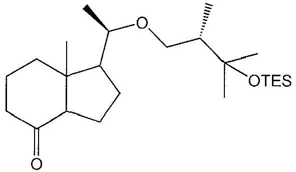

The title compound was prepared from the method described in the preparation of Example 44, by substituting the product of Example 42 for Example 28 in 62.3% yield. ¹H NMR (400 MHz, CDCl₃) δ 3.50 (1H, dd, J = 8.3, 2.3 Hz), 3.28-3.17 (2H, m), 2.46 (1H, dd, J = 11.0, 8.3 Hz), 2.34-2.17 (3H, m), 2.04-1.83 (2H, m), 1.83-1.64 (4H, m), 1.63-1.51 (3H, m), 1.20 (3H, s), 1.11 (3H, s), 1.07 (3H, d, J = 6.0 Hz), 0.95 (3H, d, J = 7.8 Hz), 0.94 (9H, t, J = 7.8 Hz), 0.64 (3H, s), 0.57 (6H, q, J = 7.8 Hz).

### EXAMPLE 48

This example describes the synthesis of (4*R*,8*R*)-6-((*E*)-2-(1-((*R*)-1-((*S*)-3-tert-butyldimethylhydroxy-2,3-dimethylbutoxy)ethyl)-4,8-(di-tert-butyldimethylsilyloxy)-7α-methyldihydro-1H-inden-4(*2H*,*5H,6H,7H,7αH*)-ylidene)ethylidene)spiro[2.5]octane, which is an intermediate of a compound of Formula (I), in an embodiment of the invention.

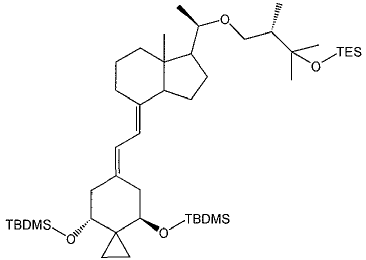

The title compound was prepared by the method described in Example 45, substituting the product of Example 15A by Example 47 in 40% yield. ¹H NMR (400 MHz, CDCl₃) δ 6.19 (1H, d, J = 11.5 Hz), 5.82 (1H, d, J = 11.4 Hz), 3.76 (1H, dd, J = 7.8, 3.7 Hz), 3.52 (1H, dd, J = 8.7, 3.2 Hz), 3.49 (1H, t, J = 5.0 Hz), 3.26-3.21 (2H, m), 2.83 (1H, d, J = 11.9 Hz), 2.44-2.36 (3H, m), 2.19-2.13 (2H, m), 2.01 (1H, t, J = 9.6 Hz), 1.78-1.48 (8H, m), 1.33-1.24 (2H, m), 1.20 (3H, s), 1.12 (3H, s), 1.06 (3H, d, J = 6.0 Hz), 0.96 (3H, d, J = 6.0 Hz), 0.95 (9H, t, J = 7.8 Hz), 0.86 (18 H, s), 0.60-0.54 (1H, m), 0.57 (6H, q, J = 7.8 Hz), 0.56 (3H, s), 0.47-0.41 (2H, m), 0.29-0.24 (1H, m), 0.076 (3H, s), 0.051 (3H, s), 0.036 (3H, s), 0.014 (3H, s).

### EXAMPLE 49

This example describes the synthesis of (4*R*,8*R*)-6-((*E*)-2-((*3αS,7αS*)-1-((*R*)-1-((*S*)-3-hydroxy-2,3-dimethylbutoxy)ethyl)-7α-methyldihydro-1H-inden-4(*2H,5H,6H,7H, 7αH*)-ylidene)ethylidene)spiro[2.5]octane-4,8-diol (Vida-4) in an embodiment of the invention.

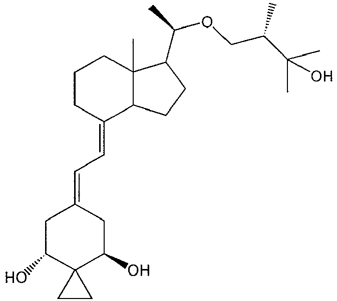

The title compound was prepared by the method described in Example 11. 63mg of Example 48 as the starting material yielded 25mg of the title compound in 70.1 % yield. MS: m/z (%) 469 (18) [M + Na]⁺, 429 (8), 411 (13), 311 (86), 293 (100), 267 (26). ¹H NMR (400 MHz, CDCl₃) δ 6.29 (1H, d, J = 11.0 Hz), 5.84 (1H, d, J = 11.4 Hz), 3.74 (1H, dd, J = 9.2, 4.1 Hz), 3.71 (1H, dd, J = 8.3, 4.1 Hz), 3.51 (1H, dd, J = 5.9, 3.6 Hz), 3.30 (1H, dd, J = 9.2, 5.5 Hz), 3.25 (1H, dd, J = 9.6, 5.9 Hz), 2.80-2.70 (2H, m), 2.54 (1H, dd, J = 13.3, 2.8 Hz), 2.22-2.27 (2H, m), 2.11 (1H, d, J = 12.8 Hz), 1.99 (1H, t, J = 9.2 Hz), 1.79-1.41 (9H, m), 1.35-1.24 (1H, m), 1.22 (3H, s), 1.13 (3H, s), 1.11 (3H, d, J = 6.0 Hz), 0.98 (3H, d, J = 6.9 Hz), 0.70-0.62 (1H, m), 0.587-0.511 (2H, m), 0.55 (3H, s), 0.41-0.36 (1H,).

### EXAMPLE 50

This example describes the synthesis of (4*R*,8*R*)-6-((*E*)-2-((*1S,7αS*)-1-((*R*)-1-(4-ethyl-4-hydroxyhexyloxy)ethyl)-7α-methyldihydro-1H-inden-4(*2H,5H,6H, 7H, 7αH*)-ylidene)ethylidene)spiro[2.5]octane-4,8-dily)bis(oxy) bis(*tert*-butyldimethylsilane), which is an intermediate of a compound of Formula (I), in an embodiment of the invention.

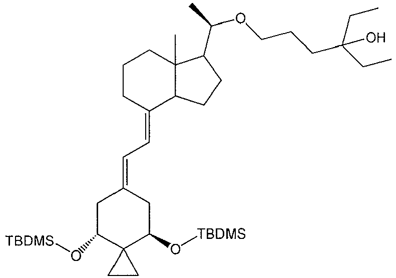

The title compound was prepared by the method described in Example 45, by substituting of the product of Example 44 for Example 37. Example 37 (65mg, 0.2mmol) and Example 15B (120mg, 0.2mmol) were used to obtain 16mg of the title compound in 11.4% yield. ¹H NMR (400 MHz, CDCl₃) δ 6.18 (1H, d, J = 11.0 Hz), 5.81 (1H, d, J = 11.5 Hz), 3.75 (1H, dd, J = 7.76, 3.64 Hz), 3.59-3.52 (1H, m), 3.48 (1H, s, br), 3.31-3.21 (3H, m), 2.82 (1H, d, J = 12.8 Hz), 2.45-2.34 (3H, m), 2.16-2.12 (2H, m), 2.08-1.98 (2H, m), 1.75-1.23 (16H, m), 1.09 (3H, d, J = 5.5 Hz), 0.84 (18H, s), 0.84 (6H, t, J = 8.3 Hz), 0.58-0.51 (1H, m), 0.55 (3H, s), 0.45-0.40 (2H, m), 0.28-0.22 (1H, m), 0.04 (3H, s), 0.02 (3H, s), 0.00 (6H, s).

### EXAMPLE 51

This example describes the synthesis of (*4R,8R*)-6-((*E*)-2-((*1S,7αS*)-1-((R)-1-(4-ethyl-4-hydroxyhexyloxy)ethyl)-7α-methyldihydro-*1H-*inden-4(*2H,5H*,*6H,7H,7αH*)-ylidene)ethylidene)spiro[2.5]octane-4,8-diol (Vida-20) in an embodiment of the invention.

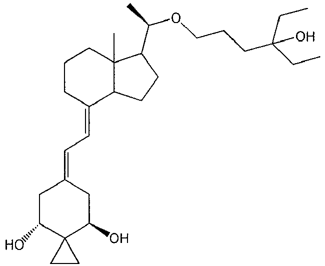

The title compound was prepared from the product of Example 50 (64mg, 0.091mmol) and tetra(*n*-butyl)ammonium fluoride (10 mL), according to the procedure described in Example 11. Accordingly, 20mg of the title compound was obtained in 46.3% yield. MS: m/z (%) 497 (27) [M + Na]⁺, 421 (7), 311 (98), 293 (100), 267 (12), 122 (8). ¹H NMR (400 MHz, CDCl₃) δ 6.34 (1H, d, J = 11.4 Hz), 5.86 (1H, d, J = 11.4 Hz), 3.74 (1H, dd, J = 8.2, 4.1 Hz), 3.59-3.54 (2H, m), 3.31-3.19 (2H, m), 2.82 (1H, dd, J = 12.4,4.1 Hz), 2.78 (1H, dd, J = 13.3, 3.7 Hz), 2.57 (1H, dd, J = 13.3, 3.2 Hz), 2.29 (1H, dd, J = 13.3, 2.8 Hz), 2.27 (1H, d, J = 13.3 Hz), 2.16 (1H, d, J = 12.4 Hz), 2.03 (1H, t, J = 9.6 Hz), 1.80-1.41 (17H, m), 1.33 (1H, td, J = 13.3, 4.1 Hz), 1.21-1.14 (1H, m), 1.09 (3H, d, J = 6.0 Hz), 0.55 (3H, t, J = 7.4 Hz), 0.85 (3H, t, J = 7.8 Hz), 0.71-0.66 (1H, m), 0.62-0.54 (2H, m), 0.56 (3H, s), 0.44-0.40 (1H, m).

### EXAMPLE 52

This example describes the synthesis of (*S*)-2-((1*R*,3*αR*,4*S*,7*αR*)-4-hydroxy-7α-methyloctahydro-1H-inden-1-yl)propyl pivalate, which is an intermediate of a compound of Formula (I), in an embodiment of the invention.

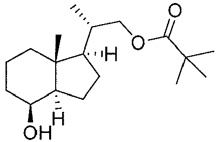

The compound of Example 19 (1.57 g, 6.7 mmol) was dissolved in 10 mL of CH₂Cl₂ and 5 mL of pyridine; the solution was cooled to 0 °C, and 0.94 mL of pivaloyl chloride was added dropwise over 5 min. The resultant mixture was stirred at 0 °C for 4 h and then warmed to ambient temperature overnight. The reaction was quenched with water, and the mixture was concentrated *in vacuo* with the bath maintained at below ambient temperature. The crude material was partitioned between ether and 0.5*N* aqueous HCl; the organic phase was washed with 0.5*N* aqueous HCl, then brine, and dried over Na₂SO₄. The solvents were removed *in vacuo;* the residue was purified by chromatography on an Analogix IntelliFlash 280, eluting with a gradient of 10% to 20% EtOAc in hexanes. The product was isolated as a white solid (1.25 g = 63% yield).

### EXAMPLE 53

This example describes the synthesis of (*S*)-2-((1*R*,3*αR*,7*αR*)-7α-methyl-4-oxooctahydro-1H-inden-1-yl)propyl pivalate, which is an intermediate of a compound of Formula (I), in an embodiment of the invention. 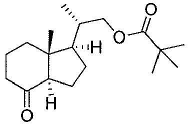 The compound of Example 52 (1.1 g, 3.7 mmol) was dissolved in 5 mL of CH₂Cl₂ and cooled to 0 °C; 4.1 g of pyridinium dichromate (PDC) was added, followed by 30 mg of pyridinium *p*-toluenesulfonate (PPTS). The resultant mixture was stirred for 8 h at 0 °C; an additional 1.8 g of PDC and 20 mg of PPTS was added, and the mixture was allowed to warm to ambient temperature overnight. The mixture was diluted with ether and filtered through a pad of Celite, followed by an ether wash. The filtrate was washed with 1*N* aqueous HCl, then filtered through a plug of silica gel. The crude product was purified by chromatography on an Analogix IntelliFlash 280, eluting with a gradient of 10% to 15% EtOAc in hexanes. The title compound was isolated as a colorless oil. Yield 1.0 g, 94% yield.

### EXAMPLE 54

This example describes the synthesis of intermediate compound 19-nor-1α,3-bis(*tert-*butyldimethylsilyloxy)-2-methylene-23-oxa-24-oxo-25-methyl Vitamin D3 in an embodiment of the invention.

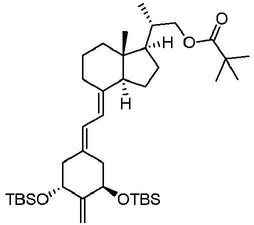

The following synthesis was performed in a darkened hood. The compound of Example 9 (0.75 g, 1.3 mmol) was combined with the compound of Example 53 (0.66 g, 2.2 mmol), and the resultant mixture was dried by azeotroping twice with 5 mL of toluene. THF (10 mL) was added, and the solution was cooled to -78 °C. A solution of LiHMDS (1*M* in THF; 2.0 mL) was added dropwise, producing a yellow-orange color that faded over 20 min. The solution was stirred at this temperature for 2.2 h. The reaction was quenched by the addition of 10 ml of 1*N* aqueous NF₄Cl, and the mixture was extracted with EtOAc. The organic extract was washed with brine and dried over Na₂SO₄. The solvents were removed *in vacuo,* and the residue was purified by chromatography on an Analogix IntelliFlash 280, eluting with a gradient from 0% to 20% EtOAc in hexanes. The title compound (0.53 g, 63% yield) was isolated as a colorless oil, along with 0.34 g of unreacted ketone.

### EXAMPLE 55

This example describes the synthesis of (*S*)*-*2*-*((*1R*,*3αS*,*7αR,E*)-4-(2-((3*R,*5*R*)-3,5-bis(tert-butyldimethylsilyloxy)-4-methylenecyclohexylidene)ethylidene)-7α-methyloctahydro-1H-inden-1-yl)propan-1-ol, which is an intermediate of a compound of Formula (I), in an embodiment of the invention.

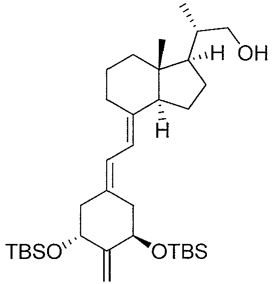

The following synthesis was performed in a darkened hood. The compound of Component E (0.53 g, 0.80 mmol) was dissolved in 8 mL of ether and cooled to -78 °C. A solution of lithium aluminium hydride (LAH) in THF (1.0 *M,* 4 mL, 5 eq) was added; the mixture was stirred for 10 min, and then warmed to 0 °C for 50 min. The reaction was quenched by cautious addition of EtOAc, followed by Rochelle's salt. After stirring for 2 h, the mixture was extracted with EtOAc; the organic extract was washed with brine and dried over Na₂SO₄. The solvents were removed *in vacuo,* leaving a white solid, 0.46 g = 100% yield.

### EXAMPLE 56

This example describes the synthesis of (1*R*,3*R*)-5-((*E*)-2-((1*R*,3*αS*,7*αR*)-7α-methyl-1-((*S*)-1-phenoxypropan-2-yl)dihydro-1H-inden-4(*2H*,*5H, 6H, 7H, 7αH*)-ylidene)ethylidene)-2-methylenecyclohexane-1,3-diol (Vida-36) in an embodiment of the invention.

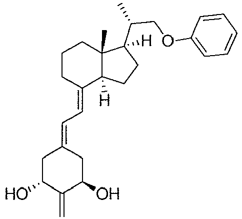

The following synthesis was performed in a darkened hood. The compound of Example 55 (16 mg, 0.028 mmol) was combined with 8 mg of phenol and 20 mg of triphenyl phosphine (TPP) in 2 mL of toluene, followed by 18 mg of di(*tert-*butyl)benzylazodicarboxylate (DBAD). The mixture was purged with argon and heated at 85 °C for 4 h. After solvent removal *in vacuo,* the residue was purified by chromatography on an Analogix IntelliFlash 280, eluting with a gradient from 0% to 5% EtOAc in hexanes. The product (6 mg) was dissolved in 1.5 mL of 1*N* TBAF in THF. After stirring for 3 h at ambient temperature, the reaction mixture was partitioned between EtOAc and water. The organic phase was washed with brine and dried over Na₂SO₄. The solvents were removed *in vacuo,* and the residue was purified by chromatography on an Analogix IntelliFlash 280, eluting with a gradient from 25% to 45% EtOAc in hexanes. The product was isolated as a colorless oil, 4 mg.

### EXAMPLE 57

This example describes the synthesis of (4R*,*8*R*)-6-((*E*)-2-(1-((*R*)-1-(3-(2-hydroxypropan-2-yl)phenoxy)propan-2-yl)-7a-methyldihydro-1H-inden-*4*(*2H,5H,6H,7H,7αH*)*-*ylidene)ethylidene)spiro[2.5]octane-4,8-diol (Vida-57) in an embodiment of the invention. Examples 57A to 57I concern intermediate steps.

### EXAMPLE 57-A

This example describes the synthesis of (1R,3αR,7αR)-7a-methyl-1-((R)-1-(triethylsilyloxy)propan-2-yl)octahydro-1H-inden-4-ol in an embodiment of the invention.

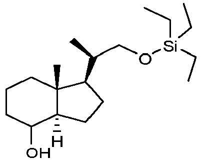

To a solution of (1R,3αR,7αR)-1-((R)-1-hydroxypropan-2-yl)-7α-methyloctahydro-1H-inden-4-ol (0.2 g, 0.94 mmol) in 10 mL of anhydrous CH₂Cl₂ is added a 40% NEt₃ solution (0.26 mL), DMAP (18 mg, 0.15 mmol) followed by TESCl (0.22 g, 1.42 mmol) at room temperature. The solution is stirred at ambient temperature for 1 h and then quenched with saturated NH₄Cl solution (15 mL). The mixture is extracted with CH₂Cl₂, and the combined organic phase is washed with saturated NH4Cl solution, dried over MgSO₄, filtered, and concentrated to yield a colorless oil (0.37 g). The crude product is used in the next step without further purification.

### EXAMPLE 57-B

This example describes the synthesis of (1R,3αR,7αR)-7α-methyl-1-((R)-1-(triethylsilyloxy)propan-2-yl)hexahydro-1H-inden-4(2H)-one in an embodiment of the invention.

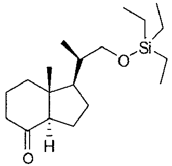

(1R,3αR,7αR)-7α-methyl-1-((R)-1-(triethylsilyloxy)propan-2-yl)octahydro-1H-inden-4-ol (Example 57-A) is dissolved in CH₂Cl₂ (10 mL), then PCC (1.41 mmol) is added. After stirring for 2h, the reaction mixture is quenched with saturated NH₄Cl solution, and the mixture is extracted with EtOAc. The combined organic phase is washed with saturated NaCl solution, dried over MgSO₄, filtered, and concentrated. The resulting residue is chromatographed on a silica gel column to give ketone (268 mg, 87%). ¹H-NMR(400 MHz, CDCl₃): δ 0.60 (q, 6H, J = 4.4 Hz), 0.66 (s, 3H), 0.93(d, 3H, J = 2.4 Hz), 0.98(t, 9H, J = 2.8 Hz), 1.25-1.70(m, 5H), 1.80-2.10(m, 6H), 2.20-2.30(m, 2H), 2.40-2.50(m, 1H), 3.35-3.45(m, 1H), 3.60-3.70(m, 1H).

### EXAMPLE 57-C

This example describes the synthesis of methyl 2-((4R,8R)-4,8-bis(*tert-*butyldimethylsilyloxy)spiro[2.5]octan-6-ylidene)acetate in an embodiment of the invention.

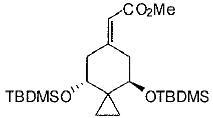

To a stirred solution of DIPEA (72ml, 0.7mmol) in anhydrous THF (1mL) is added n-BuLi (0.29 ml, 2.5M in hexane) at -78 °C. The mixture is stirred at -78 °C for 10 min and Me₃SiCH₂CO₂Et (115 mg, 0.7 mmol) in anhydrous THF (1.5 ml) is added. The mixture is stirred at -78 °C for 30 min. (4R,8R)-4,8-bis(*tert-*butyldimethylsilyloxy)spiro[2.5]octan-6-one (Example 57-B, 100 mg, 0.27 mmol) is added dropwise at -78 °C. The reaction mixture is stirred at -78 °C for 40 min, poured into saturated NH₄Cl solution (10ml) and brine (50ml), extracted with ethyl acetate,washed with brine, dried (Na₂SO₄), and evaporated to get an oil (92 mg). ¹H-NMR(400 MHz, CDCl₃): δ0.05 (s, 12 H), 0.37-0.60 (m, 4 H), 0.88 (s, 18 H), 1.29 (t, 3 H, J = 6 Hz), 2.23-2.43 (m, 2 H), 2.97-3.07 (m, 2 H), 3.62-3.63 (d, 1 H, J = 3.2 Hz), 3.80-3.81 (d, 1 H, J = 4 Hz), 5.74 (s, 1 H).

### EXAMPLE 57-D

This example describes the synthesis of 2-((4R,8R)-4,8-bis(*tert-*butyldimethylsilyloxy)spiro[2.5]octan-6-ylidene)ethanol in an embodiment of the invention.

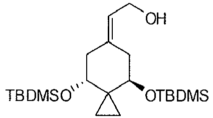

To a stirred solution of methyl 2-((4R,8R)-4,8-bis(*tert-*butyldimethylsilyloxy)spiro[2.5]octan-6-ylidene)acetate (Example 57-C, 89 mg, 0.22mmol) in toluene (1 mL) and DCM (1 ml) is added DIBALH (2.1ml, 1.0M in toluene). The reaction mixture is stirred for 45 min, poured into saturated NH₄Cl solution (10ml), 0.1 N HCl solution and brine, extracted with ethyl acetate, washed with brine, dried (Na₂SO₄), and evaporated to get oil (500 mg). The oil is purified by a chromatogram gel column to give 46 mg of solid (yield 77%).

### EXAMPLE 57-E

This example describes the synthesis of 2-(2-((4R,8R)-4,8-bis(*tert-*butyldimethylsilyloxy)spiro[2.5]octan-6-ylidene)ethylsulfonyl)benzo[d]thiazole in an embodiment of the invention.

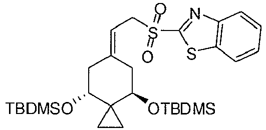

To a stirred solution of benzo[d]thiazole-2-thiol (37 mg, 0.22mmol) and PPh₃ (146 mg, 0.55 mmol) in DCM is added 2-((4R,8R)-4,8-bis(*tert-*butyldimethylsilyloxy)spiro[2.5]octan-6-ylidene)ethanol (Example 57-D, 46 mg, 0.11mmol) in DCM, then DIAD (115 mg, 0.55 mmol) is added dropwise. The reaction mixture is stirred at 0 °C for 1h and then concentrated to give a yellow oil. This oil is dissolved into ethanol and 30% H₂O₂ and (NH₄)₆Mo₇O₂₄·4H₂O (20mg) was added at room temperature. The reaction mixture Is stirred at room temperature for 3h. It is then quenched with a saturated NaHCO₃ solution and extracted with ethyl acetate. The ethyl acetate layer is washed with brine, dried over Na₂SO₄, and evaporated to give 25 mg of white solid. ¹H-NMR(400 MHz, CDCl₃): δ0.05 (s, 12 H), 0.30-0.49 (m, 4 H), 0.85 (s, 18 H), 2.07-2.11 (m, 2 H), 2.21-2.40 (m, 2 H), 3.56 (d, 1 H, J = 3.6 Hz), 3.63 (d, 1 H, J = 3.6 Hz), 4.24-4.33 (m, 2 H), 5.36 (s, 1 H), 7.62-7.66 (m, 2 H), 8.03 (d, 1 H, J = 7.6 Hz), 8.24 (d, 1 H, J = 8 Hz).

### EXAMPLE-57-F

This example describes the synthesis of ((4R,8R)-6-((E)-2-((lR,3aS,7aR)-7a-methyl-1-((R)-1-(triethylsilyloxy)propan-2-yl)dihydro-1H-inden-4(2H,5H,6H,7H,7αH)-ylidene)ethylidene)spiro[2.5]octane-4,8-diyl)bis(oxy)bis(tert-butyldimethylsilane) in an embodiment of the invention.

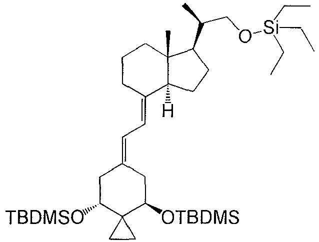

To a 50 mL two-necked flask is added 2-(2-((4R,8R)-4,8-bis(*tert-*butyldimethylsilyloxy)-spiro[2.5]octan-6-ylidene)ethylthio)benzo[d]thiazole (Example 57-E, 0.6 g, 1.01 mmol) and dried THF in a nitrogen atmosphere. The solution is cooled to -78 °C. NaHMDS (1.0 M, 1 mmol) in THF is added dropwise to the solution. Then (1R,3aR,7aR)-7a-methyl-1-((R)-1-(triethylsilyloxy)propan-2-yl)hexahydro-1H-inden-4(2H)-one (360 mg, 1.1 mmol) is added. After one hour stirring at -78 °C, the reaction is warmed to room temperature and stirred for 1 h. The mixture is quenched with a saturated NH₄Cl solution and extracted with EtOAc. The organic phases are combined and washed with brine, dried with NaSO₄, and concentrated *in vacuo.* Purification on silica gel column gave 150 mg of the title product (80% purity) as a colorless oil. ¹H NMR(400 MHz, CDCl₃):δ0.08 (m, 21 H), 0.60 (m, 6 H), 0.63 (s, 3 H), 0.90 (s, 18 H), 0.88-2.51 (m, 20 H), 3.33 (t, 1 H, J = 7.8 Hz), 3.73 (dd, 1 H, J = 10.0, 4.4 Hz), 4.42 (m, 2 H), 4.96 (d, 2 H, J = 4.4 Hz), 6.12 (d, 1 H, J = 10.8 Hz), 6.31 (d, 1 H, J = 11.2 Hz).

### EXAMPLE 57-G

This example describes the synthesis of (2R)-2-((1R,3αS,7αR,E)-4-(2-((4R,8R)-4,8-bis(*tert*-butyldimethylsilyloxy)spiro[2.5]octan-6-ylidene)ethylidene)-7α-methyloctahydro-1H-inden-1-yl)propan-1-ol in an embodiment of the invention.

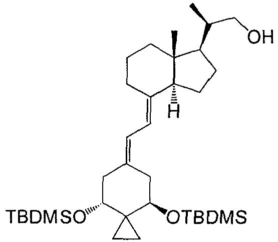

To a solution of ((4R,8R)-6-((E)-2-((1R,3αS,7αR)-7α-methyl-1-((R)-1-(triethylsilyloxy)propan-2-yl)dihydro-1H-inden-4(2H,5H,6H,7H,7aH)-ylidene)ethylidene)spiro[2.5]octane-4,8-diyl)bis(oxy)bis(tert-butyldimethylsilane) (Example 57-G, 200 mg, 0.45 mmol) in THF is added TBAF (0.45mmol) in a nitrogen atmosphere, and the mixture is stirred at room temperature for 3.5 hours. The mixture is concentrated and the crude product is purified on a short gel column to give 90 mg of the title product as a colorless oil. The crude product is used for next step without further purification.

### EXAMPLE 57-H

This example describes the synthesis of (2R)-2-((1R,3αS,7αR,E)-4-(2-((4R,8R)-4,8-bis(*tert*-butyldimethylsilyloxy)spiro[2.5]octan-6-ylidene)ethylidene)-7α-methyloctahydro-1H-inden-1-yl)propyl 4-methylbenzenesulfonate in an embodiment of the invention.

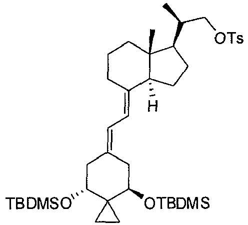

To a solution of (2R)-2-((1R,αS,7αR,E)-4-(2-((4R,8R)-4,8-bis(*tert-*butyldimethylsilyloxy)spiro-[2.5] octan-6-ylidene)ethylidene)-7α-methyloctahydro-1H-inden-1-yl)propan-1-ol (Example 57-G, 195 mg, 0.33 mmol) in DCM is added NEt₃ (166 mg, 1.65 mmol), DMAP (100 mg, 0.097mol), and then TsCl (191 mg, 1 mmol) at 10 °C. The mixture is stirred at 25 °C overnight. The reaction solution is then diluted with DCM and quenched by adding saturated aqueous NaHCO₃ The mixture is stirred at room temperature for 20 minutes and then separated. The organic phase is washed with brine, dried with Na₂SO₄, and concentrated *in vacuo.* The crude product is purified to afford the title compound (192 mg) as a colorless oil.

### EXAMPLE 57-I

This example describes the synthesis of 2-(3-((2R)-2-((1R,3αS,7αR,E)-4-(2-((4R,8R)-4,8-bis(tert-butyldimethylsilyloxy)spiro[2.5]octan-6-ylidene)ethylidene)-7α-methyloctahydro-1H-inden-1-yl)propoxy)phenyl)propan-2-ol in an embodiment of the invention.

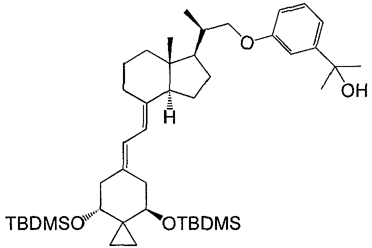

To a suspension of NaH (60%, 23 mg, 0.58 mmol) in DMF (2 mL) is added 3-(2-hydroxypropan-yl)-phenol (100 mg, 0.672 mmol) in a nitrogen atmosphere. The mixture is stirred at room temperature for one hour. To the solution is added (2R)-2-((1R,3αS,7αR,E)-4-(2-((4R,8R)-4,8-bis(tert-butyldimethylsilyloxy)spiro[2.5]octan-6-ylidene)ethylidene)-7α-methyloctahydro-1H-inden-1-yl)propyl 4-methylbenzenesulfonate (Example 57-H, 57 mg, 0.07 mmol). The mixture is stirred at room temperature overnight and then quenched with water and extracted with DCM. The organic phases are combined and washed with brine, dried with Na₂SO₄, and *concentrated in vacuo.* Purification on a short column gives the title compound (43 mg) as a colorless oil.

### EXAMPLE 57-J

This example describes the synthesis of (4*R*,8*R*)-6-((*E*)-2-(1-((*R*)-1-(3-(2-hydroxypropan-2-yl)phenoxy)propan-2-yl)-7a-methyldihydro-1 H-inden-4(2*H,5H,6H,7H,7αH*)-ylidene)ethylidene)spiro[2.5]octane-4,8-diol (Vida-57) in an embodiment of the invention.

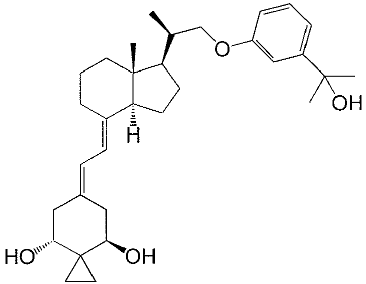

To a solution of 2-(3-((2R)-2-((1R,3αS,7aR,E)-4-(2-((4R,8R)-4,8-bis(*tert-*butyldimethylsilyloxy)spiro[2.5]octan-6-ylidene)ethylidene)-7a-methyloctahydro-1H-inden-1-yl)propoxy)phenyl)propan-2-ol (Example 57-I, 43 mg, 0.06 mmol) in MeOH is added CSA (800 mg), and the mixture is stirred at room temperature for 8 hours. The mixture is then quenched with water, extracted with DCM, and concentrated *in vacuo.* Purification gives the title compound (23 mg) as a colorless oil. ¹H NMR(400 MHz, CDCl₃): δδ0.73 (s, 3 H), 1.09 (d, 3 H, J = 6.8Hz), 1.58 (s, 6 H), 1.29-2.62 (m, 20 H), 4.01(d, 1 H, J = 3.6 Hz), 4.50 (m, 2 H), 5.12 (d, 2 H, J = 3.2 Hz), 6.19 (d, 1 H, J = 11.6 Hz), 6.53 (d, 1 H, J = 11.6 Hz), 6.78 (d, 1 H, J = 8.0 Hz), 7.06 (m, 2 H), 7.26 (m, 2 H). ¹³C-NMR (400 MHz, CDCl₃): δ13.31 (C18), 17.23 (C21), 24.02 (C15), 26.26 (C16), 27.28 (C9), 31.74 (C26,27), 35.68 (C20), 36.88 (C10), 39.17 (C12), 40.02 (C4), 46.45-46.55 (C13), 51.72 (C17), 55.87 (C14), 71.21 (C3), 71.56 (C1), 71.99 (C25), 72.58 (C22), 107.78 (C28), 111.26 (C31), 112.13(C19), 116.59 (C7), 119.72 (C29), 125.23 (C6), 129.19 (C30), 131.18 (C8), 142.00 (C5), 150.87 (C24), 152.01 (C2), 159.24 (C23).

### EXAMPLE 58

This example describes the synthesis of (1*R*,3*R*)-5-((*E*)-2-(1-((*R*)-1-(3-(2-hydroxypropan-2-yl)phenoxy)propan-2-yl)-7a-methyldihydro-1H-inden-4(*2H,5H,6H,7H,7αH*)-ylidene)ethylidene)-2-methylenecyclohexane-1,3-diol (Vida-37) in an embodiment of the invention.

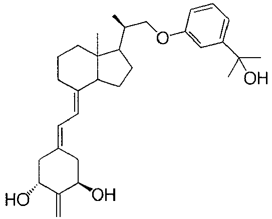

To a solution containing the compound of Example 63 (34 mg, 0.048 mmol) and MeOH(2 mL) was added Dowex 50WX4 resin (200 mg). The mixture was stirred at room temperature for 4 h. The mixture was filtered and the filtrate cake was washed with MeOH. The organic phases were combined and concentrated. The residue was purified by preparative TLC (PE/EA = 2/1) to give title product (12 mg, 67%).1H-NMR(400 MHz, CDCl3): δ0.73 (s, 3 H), 1.09 (d, 3 H, J = 6.8Hz), 1.58 (s, 6 H), 1.29-2.62 (m, 20 H), 4.01(d, 1 H, J = 3.6 Hz), 4.50 (m, 2 H), 5.12 (d, 2 H, J = 3.2 Hz), 6.19 (d, 1 H, J = 11.6 Hz), 6.53 (d, 1 H, J = 11.6 Hz), 6.78 (d, 1 H, J = 8.0 Hz), 7.06 (m, 2 H), 7.26 (m, 2 H). 13C-NMR (400 MHz, CDCl3): δ13.31 (C18), 17.23 (C21), 24.02 (C15), 26.26 (C16), 27.28 (C9), 31.74 (C26,27), 35.68 (C20), 36.88 (C10), 39.17 (C12), 40.02 (C4), 46.45-46.55 (C13), 51.72 (C17), 55.87 (C14), 71.21 (C3), 71.56 (C1), 71.99 (C25), 72.58 (C22), 107.78 (C28), 111.26 (C31), 112.13(C19), 116.59 (C7), 119.72 (C29), 125.23 (C6), 129.19 (C30), 131.18 (C8), 142.00 (C5), 150.87 (C24), 152.01 (C2), 159.24 (C23).

### EXAMPLE 59A

This example describes the synthesis of 2-(3-((2*R*)-2-((1*R*,7*αR*,*E*)-4-(2-((3*R*,5*R*)-3,5-bis(*tert*-butyldimethylsilyloxy)-4-methylenecyclohexylidene)ethylidene)-7α-methyloctahydro-1H-inden-1-yl)propylthio)phenyl)propan-2-ol, which is an intermediate of a compound of Formula (I), in an embodiment of the invention.

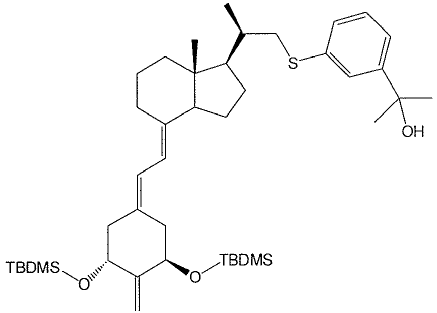

To a suspension ofNaH (60%, 15.6 mg, 0.39 mmol) in DMF (1 mL) was added 2-(3-mercaptophenyl)propan-2-ol (70 mg, 0.42 mmol) at N₂ atmosphere. The mixture was stirred at room temperature for 1 h. To the solution was added compound of Example 62 (90 mg, 0.12 mmol). The mixture was stirred at room temperature overnight and then quenched with water (20 mL) and extracted with AcOEt (20 mL x 3). The organic phases were combined, washed with brine (20 mL), dried with Na₂SO₄, and *concentrated in vacuo.* Purification on silica gel column (petroleum ether/AcOEt = 20/1) gave the title compound (77mg, 86%) as a colorless oil. ¹H-NMR(400 MHz, CDCl₃): δ 0.02-0.10 (m, 12 H) 0.63 (s, 3 H), 0.85 -0.95 (m, 18 H), 1.08 (d, 3 H, *J=* 6.4 Hz), 1.25-2.70 (m, 24 H), 2.72-2.82 (m, 1 H), 3.24-3.31 (m, 1 H), 4.05 (d, 1 H, *J* = 2 Hz), 4.40 (d, 2 H, *J =* 4.8 Hz), 4.96 (d, 2 H, *J* = 3.2 Hz), 6.12 (d, 1 H, *J* = 11.2 Hz), 6.29 (d, 1 H, *J=* 11.2 Hz), 7.22-7.31 (m, 3 H), 7.50 (d, 1 H, *J* = 1.6 Hz).

### EXAMPLE 59B

This example describes the synthesis of (1*R*,3*R*)-5-((*E*)-2-(1-((*R*)-1-(3-(2-hydroxypropan-2-yl)phenylthio)propan-2-yl)-7α-methyldihydro-1H-inden-4(2*H,5H,6H,7H,7αH*)-ylidene)ethylidene)-2-methylenecyclohexane-1,3-diol (Vida-43) in an embodiment of the invention.

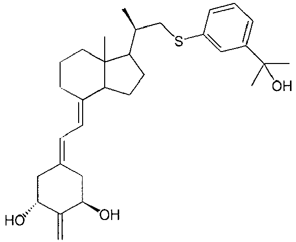

To a solution of the compound from Example 59A (70mg, 0.09mmol) in MeOH(2 mL) was added +(-)camphor sulfonic acid (CSA) (72mg, 0.155mmol), and the mixture was stirred at room temperature for 4 h, quenched with saturated NaHCO₃ aqueous (20 mL), and extracted with AcOEt (20 mL x 3). The organic phases were combined, and washed with brine (20 mL), dried with Na₂SO₄, and concentrated *in vacuo.* The residue was purified by preparative TLC (petroleum ether/AcOEt = 2/1) to give title product (29 mg, 60%). ¹H-NMR(400 MHz, CDCl₃) δ0.63 (s, 3 H), 1.08 (d, 3 H, *J* = 6.4Hz), 1.25-1.26 (m, 4 H), 1.58 (s, 6 H), 1.92-2.72 (m, 16 H), 2.78 (d, 1 H, *J* = 4 Hz), 3.24 (d, 1 H, *J* = 8 Hz), 4.47 (d, 2 H, *J* = 3.6 Hz), 5.09 (d, 1H, *J* = 2.8 Hz), 6.17 (d, 1 H, *J* = 7.2 Hz), 6.50 (d, 1 H, *J* = 11.2 Hz), 7.22-7.19 (m, 3 H), 7.50 (s, 1 H). ¹³C-NMR (400 MHz, CDCl₃): δ13.29 (C18), 18.87 (C21), 24.02 (C15,11), 26.17(C16), 27.11 (C9), 31.71 (C26,27), 35.15 (C20), 36.84 (C10), 39.13 (C12), 40.49 (C22), 41.15(C4),46.48-46.53 (C13), 54.15 (C14), 55.78 (C17), 71.16 (C3), 71.48 (C1), 72.51 (C25), 107.81 (C19), 119.82 (C7), 121.96(C28), 125.07 (C29), 125.30(C31),127.29 (C6), 128.69 (C30), 131.37 (C8), 137.46(C23),141.70 (C5), 149.82 (C24), 151.96 (C2).

### EXAMPLE 60

This example describes the synthesis of ((1*R,*3*R*)-5-((*E*)*-2*-((*1R,3αS,7ααR*)-7α-methyl-1-((*R*)-1-(triethylsilyloxy)propan-2-yl)dihydro-1H-inden-4(*2H,5H,6H,7H,7αH*)-ylidene)ethylidene)-2-methylenecyclohexane-1,3-diyl)bis(oxy)bis(tert-butyldimethylsilane), which is an intermediate of a compound of Formula (I), in an embodiment of the invention.

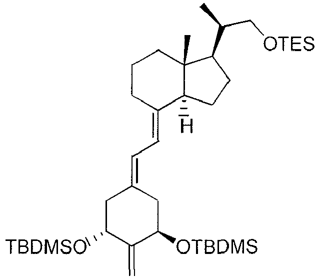

To a 50 mL two-necked flask was added 2-(2-((3*R,*5*R*)-3,5-bis(*tert-*butyldimethylsilyloxy)-4-methylenecyclohexylidene)ethylsulfonyl)benzo[d]thiazole (Example 64, 0.434 g, 0.75 mmol), (*1R,3αR,7αR*)-7α-methyl-1-((*R*)-1-(triethylsilyloxy)propan-2-yl)hexahydro-1H-inden-4(2H)-one (Example 69, 268 mg, 0.825 mmol), and dried THF(5 mL) under a nitrogen atmosphere. The solution was cooled to -78 °C. Next, NaHMDS (1.0 M, 0.75 mL, 0.75 mmol) in THF was added dropwise to the solution. After 1 h, the addition was complete, and the solution was stirred at -78 °C for 2 h, then warmed to room temperature and stirred for 15 h. The mixture was quenched with saturated NH₄Cl solution (20 mL) and extracted with EtOAc (20 mL x2). The organic phases were combined and washed with brine, dried with Na₂SO₄ and *concentrated in vacuo.* Purification on silica gel column (petroleum ether/EtOAc = 100/1) gave 91 mg of the title product (17%, 80% purity) as a colorless oil. ¹H NMR(400 MHz, CDCl₃): δ0.08 (m, 21 H), 0.60 (m, 6 H), 0.63 (s, 3 H), 0.90 (s, 18 H), 0.88-2.51 (m, 20 H), 3.33 (t, 1 H, J = 7.8 Hz), 3.73 (dd, 1 H, J = 10.0, 4.4 Hz), 4.42 (m, 2 H), 4.96 (d, 2 H, J = 4.4 Hz), 6.12 (d, 1 H, J = 10.8 Hz), 6.31 (d, 1 H, J = 11.2 Hz).

### EXAMPLE 61

This example describes the synthesis of (*R*)-2-((*1R,3αS*,*7αR,E*)-4-(2-((3*R*,5*R*)-3,5-bis(*tert*-butyldimethylsilyloxy)-4-methylenecyclohexylidene)ethylidene)-7α-methyloctahydro-1H-inden-1-yl)propan-1-ol, which is an intermediate of a compound of Formula (I), in an embodiment of the invention.

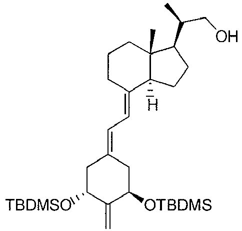

To a solution of compound of Example 60 (91 mg, 0.14 mmol) in THF (4 mL) was added *tetra*-butyl ammonium fluoride (0.28mmol) at nitrogen atmosphere, and the mixture was stirred at room temperature for 3.5 h until the compound of Example 60 was consumed. The reaction mixture was concentrated, and the crude product was purified on preparative TLC (PE/AcOEt = 10/1) to give 51 mg of title product as a colorless oil. ¹H NMR (400 MHz, CDCl₃):δ0.24-0.96 (m, 12 H), 0.70 (s, 3 H), 0.91 (s, 18 H), 1.01 (d, 3 H, J = 6.8 Hz), 1.27-2.52 (m, 18 H), 3.52 (m, 1 H), 3.75 (m, 1 H), 4.41 (m, 2 H), 4.96 (d, 2 H, J = 5.6 Hz), 6.13 (d, 1 H, J = 10.2 Hz), 6.31 (d, 1 H, J = 10.2 Hz).

### EXAMPLE 62

This example describes the synthesis of (*R*)-2-((1*R,*3*αS,*7α*R,E*)-4-(2-((3*R,5R*)-3,5-bis(*tert*-butyldimethylsilyloxy)-4-methylenecyclohexylidene)ethylidene)-7α-methyloctahydro-1H-inden-1-yl)propyl 4-methylbenzenesulfonate, which is an intermediate of a compound of Formula (I), in an embodiment of the invention.

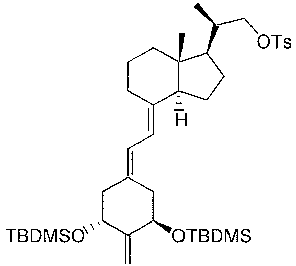

To a solution of compound of Example 61 (56 mg, 0.097 mmol) in CH₂Cl₂ (2 mL) was added Et₃N (870 mg, 2.82 mmol), *N,N*-dimethylaminopyridine (10 mg, 0.097mol), and then *p*-toluene sulfonyl chloride (360 mg, 1.96 mmol) at 10 °C., and the mixture was stirred at 25 °C overnight. The reaction solution was diluted with CH₂Cl₂ (20 mL) and quenched by adding saturated aqueous NaHCO₃ (20 mL). The mixture was stirred at room temperature for 20 min, and then separated. The organic phase was washed with brine (20 mL), dried with Na₂SO₄ and *concentrated in vacuo.* The crude material was purified on Preparative TLC(petroleum ether/EtOAc = 40/1) to afford the title compound (49 mg, 70%) as a colorless oil. ¹H NMR(400 MHz, CDCl₃): δ 0.21-0.96 (m, 12 H), 0.53 (s, 3 H), 0.94 (s, 18 H), 0.87-2.51 (m, 20 H), 2.49 (s, 3 H), 3.86 (t, 1H, J = 9.6 Hz), 4.15 (d, 1 H, J = 9.6 Hz), 4.40 (m, 2 H), 4.96 (d, 2 H, J = 4.4 Hz), 6.11 (d, 1H, J = 10.8 Hz), 6.27 (d, 1 H, J = 11.2 Hz), 7.35 (d, 2 H, J = 8.4 Hz), 7.80 (d, 2 H, J = 8.0 Hz).

### EXAMPLE 63

This example describes the synthesis of 2-(3-((*R*)-2-((*1R,3αS,7αR,E*)-4-(2-((3*R*,5*R*)-3,5-bis(*tert-*butyldimethylsilyloxy)-4-methylenecyclohexylidene)ethylidene)-7α-methyloctahydro-1H-inden-1-yl)propoxy)phenyl)propan-2-ol, which is an intermediate of a compound of Formula (I), in an embodiment of the invention.

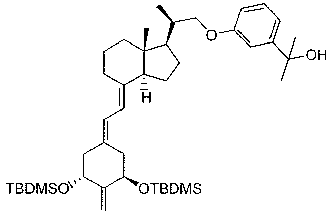

To a suspension of NaH (60%, 25.5 mg, 0.638 mmol) in DMF (1 mL) was added 3-(2-hydroxypropan-yl)-phenol (Example 65, 100 mg, 0.672 mmol) under a nitrogen atmosphere. The mixture was stirred at room temperature for 1 h. To the solution was added the compound of Example 62 (49 mg, 0.067 mmol). The mixture was stirred at room temperature overnight and then quenched with water (20 mL) and extracted with AcOEt (20 mL x3). The organic phases were combined and washed with brine (20 mL), dried with Na₂SO₄ and *concentrated in vacuo.* Purification on silica gel column (petroleum ether/AcOEt = 20/1) gave the title compound (35mg, 67%) as a colorless oil, and recovered 13 mg of Example 62. ¹H-NMR: δ 0.24-0.97 (m, 12 H), 0.71 (s, 3 H), 0.92 (s, 18 H), 1.10 (d, 3 H, *J* = 6.8 Hz), 1.60 (s, 3 H), 1.20-2.21 (m, 18 H), 3.80 (m, 1H), 4.03 (m, 1H), 4.43 (m, 2 H), 4.97 (d, 2 H, *J* = 4.8 Hz), 6.15 (d, 1H, *J* = 10.2 Hz), 6.31 (d, 1H, *J =* 10.2 Hz), 6.79 (d, 1 H, *J* = 7.8 Hz), 7.08 (m, 1 H), 7.29 (m, 1 H).

### EXAMPLE 64

This example describes the synthesis of (2-(2-((3*R,*5*R*)-3,5-bis(*tert-*butyldimethylsilyloxy)-4-methylenecyclohexylidene)ethylsulfonyl)benzo[*d*]thiazole), which is an intermediate of a compound of Formula (I), in an embodiment of the invention.

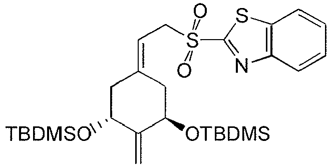

To a stirred solution of benzo[d]thiazole-2-thiol (187 mg, 1.12mmol)) and triphenyl phosphine (294 mg, 1.12mmol) in CH₂Cl₂ (5ml) was added the compound of Example 8 (280 mg, 0.70mmol) in CH₂Cl₂ (2ml) at 0 °C, then diisopropyl azodicarboxylate (DIAD, 0.28 ml, 0.98 mmol) was added dropwise at 0 °C. The reaction mixture was stirred at 0 °C for 1 h and then concentrated to get crude material (1.2 g) as a yellow oily residue.

The oily residue was dissolved in ethanol (10ml) and 30% H₂O₂ (1ml) and (NH₄)₆Mo₇O₂₄·4H₂O (200mg) was added at 0 °C. The reaction mixture was stirred at room temperature for 3 h. The reaction was quenched with a saturated NaHCO₃ solution (50ml), extracted with ethyl acetate (50ml×3), washed with brine (50ml×2), dried over Na₂SO₄ and evaporated to get oil (1.2 g). The oil was purified by chromatogram gel column (petroleum ether/EtOAc: 50:1 100 ml, petroleum ether/EtOAc : 25:1 100ml) to get the pure title compound (313 mg, two step yields 77%) as white solid (Ono et al., 2003, J Org Chem 68: 7407-7415). ¹H NMR (400 MHz, CDCl₃): δ 0.05-0.08(m, 12H), 0.88-0.9(m, 18H), 2.122-2.184(m, 3H), 2.391-2.434(dd, 1H, J = 12.8, 4.8 Hz), 4.24(dd, 1H, J = 6.8 Hz), 4.34-4.4(m, 3H), 4.93(d, 2H, J = 14.4 Hz), 5.42(s, 1H), 7.61-7.67(m, 2H), 8.01(d, 1H), 8.25(d, 1H).

### EXAMPLE 65

This example describes the synthesis of 3-(2-hydroxypropan-2-yl)phenol, which is an intermediate of a compound of Formula (I), in an embodiment of the invention.

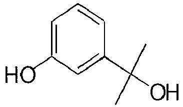

To a solution of 1-(3-hydroxyphenyl)ethanone (7.4 g, 54 mmol) in anhydrous THF (300 mL) at 0 °C was added a solution of methyl magnesium chloride (40 mL, 119 mmol) in THF slowly through a syringe under nitrogen. The reaction mixture was stirred at room temperature for 18 h. Thin layer chromatography (TLC) indicated about 30% of starting material remained. The reaction mixture was refluxed for 2 h and TLC indicated most of starting material was consumed. The reaction mixture was cooled to 0 °C and quenched with saturated aqueous NH₄Cl (20mL), followed by 1M HCl (120mL). The aqueous solution was extracted with EtOAc (50mL×3). The combined organic phase was washed with brine (200mLx2), dried over Na₂SO₄, concentrated under reduced pressure, and purified by silica gel column chromatography using ethyl acetate/petrol ether (10% to about 25%) as eluant to obtain (3.6 g, 43.9%) as a yellow solid. ¹H NMR: δ 1.59(s, 3H), 6.74-6.76(m, J=8Hz, 1H), 6.99-7.01(d, J=8Hz, 1H), 7.08(d, J=2Hz, 1H), 7.20-7.24(m, J=16Hz, 1H).

### EXAMPLE 66

This example describes the synthesis of (*1R,3αR,7αR*)-1-((*S*)-1-hydroxypropan-2-yl)-7α-methyloctahydro-1H-inden-4-ol, which is an intermediate of a compound of Formula (I), in an embodiment of the invention.

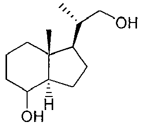

A flame-dried 250 mL three-necked flask was charged sequentially with 300 mg (3.6 mmol) of sodium bicarbonate, 50.0 mL of anhydrous methanol, 50.0 mL of anhydrous CH₂Cl₂, and ergocalciferol ((+) vitamin D₂, 3.0 g, 7.6 mmol). The solution was treated with O₃ (O₂ 5 g/h) and stirred constantly at room temperature for 4-5 h. Solid sodium borohydride (2.5 g, 64 mmol) was then added portion-wise over a period of 10 min in a water bath until complete disappearance of the starting material was observed by TLC. The resulting reaction mixture was quenched with 4 *N* hydrochloric acid, extracted with EtOAc (3×30 mL), dried over MgSO₄ filtered, and *concentrated in vacuo.* Purification by silica gel chromatography (30% EtOAc/petroleum ether) yielded 8.10 g (3.8 mmol) of the title compound in 50% yield. ¹H-NMR (400 MHz,CDCl₃): δ 0.98 (s, 3H), 1.05 (d, 3H, J = 6.4 Hz), 1.2 (d, 2H, J = 10.4 Hz), 1.36-1.82 (m,12H), 1.85(d, 1H, J = 3.6 Hz), 3.38-3.42 (dd, 1H, J = 10.4, 6.8 Hz), 3.64-3.67(dd, 1H, J = 10.4,3.6Hz), 4.10 (d, 1H, J = 2.4 Hz).

### EXAMPLE 67

This example describes the synthesis of (2*S*)-2-((*1R,3αR,7αR*)-4-hydroxy-7α-methyloctahydro-1H-inden-1-yl)propanal, which is an intermediate of a compound of Formula (I), in an embodiment of the invention.

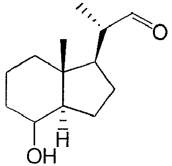

Under nitrogen, the mixture of *p*-toluenesulfonyl chloride (446 mg, 2.33 mmol), 4-(dimethylamino)-pyridine (DMAP, 25.86 mg, 0.21 mmol) in a 40% Et₃N solution (0.6 mL), and CH₂Cl₂ (2 mL) was added the product of Example 66 (450 mg, 2.12 mmol in THF (2 mL)). The reaction mixture was stirred at room temperature for 4-5 h. The reaction mixture was quenched with saturated sodium bicarbonate (10 mL), and the product was extracted with CH₂Cl₂ (15 mL). The organic phase was washed with saturated NH₄Cl solution (10 mL), dried over MgSO₄, and *concentrated in vacuo.* The resulting residue was dissolved in DMSO (10 mL) and NaHCO₃ (0.89 g, 10.6 mmol) was added, the mixture was heated at 150 °C under nitrogen for 30 min then cooled to room temperature rapidly. Water (50 mL) followed by CH₂Cl₂ (50 mL) were added. The aqueous phase was extracted with CH₂Cl₂ (3×30mL), and the combined organic phase was washed with water (20 mL), dried over MgSO₄, filtered, and concentrated *in vacuo.* The residue was chromatographed on a silica gel column (20% EtOAc/petroleum ether) to obtain the title compound (0.31 g) in 70% yield.

### EXAMPLE 68

This example describes the synthesis of (1*R,3αR,7αR*)-1-((*R*)-1-hydroxypropan-2-yl)-7α-methyloctahydro-1H-inden-4-ol, which is an intermediate of a compound of Formula (I), in an embodiment of the invention.

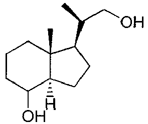

A solution of the product of Example 67 (0.28 g, 1.34 mmol) in CH₂Cl₂ (10 mL) was added tetra-*n*-butylammonium hydroxide (0.43 g, 0.67 mmol), and the resulting solution was stirred at 25 °C for 16 h and then quenched with water (10 mL). The organic phase was washed with water (10 mL), dried over MgSO₄, and concentrated *in vacuo.* The resulting residue was dissolved in methanol (10 mL), then solid sodium borohydride (0.10 g, 2.63 mmol) was added over a period of 10 min. The mixture was quenched with saturated ammonium chloride solution (10 mL), and the product was extracted with EtOAc (50 mL). The organic phase was washed with saturated ammonium chloride solution (20 mL), dried over MgSO₄ filtered, and *concentrated in vacuo.* The residue was purified by chromatography on a silica gel column (9.1 % EtOAc/petroleum ether) to obtain the title compound (96.59 mg) in 34% yield. ¹H-NMR(400 MHz,CDCl₃): δ 0.97-0.988(m, 6H), 1.23-1.87(m, 16H), 3.49(d, 1H, J = 7.2 Hz), 3.72(d, 1H, J = 3.6 Hz), 4.11(d, 1H, J = 2.4 Hz)

### EXAMPLE 69

This example describes the synthesis of ((*1R,3αR,7αR*)-7α-methyl-1-((*R*)-1-(triethylsilyloxy)propan-2-yl)hexahydro-1H-inden-4(2H)-one), which is an intermediate of a compound of Formula (I), in an embodiment of the invention.

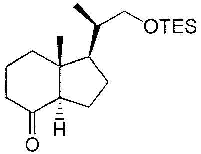

To a solution of the product of Example 68 (0.2 g, 0.94 mmol) in 10 mL of anhydrous CH₂Cl₂ at room temperature was added a 40% Et₃N solution (0.26 mL), DMAP (18 mg, 0.15 mmol), followed by triethylsilyl chloride (TESCl, 0.22 g, 1.42 mmol). The solution was stirred at room temperature for 1 h and then quenched with saturated ammonium chloride solution (15 mL). The mixture was extracted with CH₂Cl₂ (3×20 mL), and the combined organic phase was washed with saturated ammonium chloride solution (15 mL), dried over MgSO₄, filtered, and concentrated to provide a colorless oil 0.37g. The oil was used for the next reaction without further purification.

The residue oil was dissolved in CH₂Cl₂ (10 mL), then Dess-Martin periodinane (0.60 g, 1.41 mmol) was added. After stirring for 2 h at room temperature under nitrogen, the reaction mixture was quenched with saturated ammonium chloride solution (20 mL), and the mixture extracted with EtOAc (3×20 mL). The combined organic phase was washed with a saturated sodium chloride solution (20 mL), dried over MgSO₄, filtered, and concentrated. The resulting residue was chromatographed on a silica gel column (4.8% EtOAc/petroleum ether) to obtain the title compound (268 mg) in 87% yield. ¹H-NMR(400 MHz, CDCl₃) of CD5-TES: δ 0.600 (q, 6H, J = 4.4 Hz), 0.661(s, 3H), 0.930(d, 3H, J = 2.4 Hz), 0.982(t, 9H, J = 2.8 Hz), 1.25-1.70(m, 5H), 1.80-2.10(m, 6H), 2.20-2.30(m, 2H), 2.40-2.50(m, 1H), 3.35-3.45(m, 1H), 3.60-3.70(m, 1H).

### EXAMPLE 70

This example describes the synthesis of (4*R*,8*R*)-6-((*E*)-2-(1-((*R*)-1-(3-(2-hydroxypropan-2-yl)phenylthio)propan-2-yl)-7α-methyldihydro-1H-inden-4(*2H,5H,6H,7H,7αH*)-ylidene)ethylidene)spiro[2.5]octane-4,8-diol (Vida-58) in an embodiment of the invention.

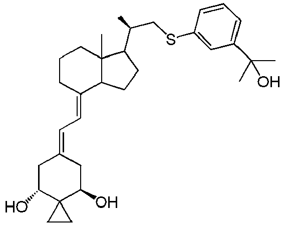

### EXAMPLE 70-A

This example describes the synthesis of intermediate compound 2-(3-((2R)-2-((*1R,3αS,7aR,E*)-4-(2-((4*R*,8*R*)-4,8-bis(*tert*-butyldimethylsilyloxy)spiro[2.5]octan-6-ylidene)ethylidene)-7*α*-methyloctahydro-1H-inden-1-yl)propylthio)phenyl)propan-2-ol in an embodiment of the invention.

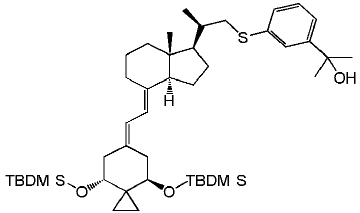

To a suspension of NaH (60%, 13 mg, 0.32 mmol) in DMFis added 2-(3-mercaptophenyl)propan-2-ol (60 mg, 0.3 mmol) in a nitrogen atmosphere. The mixture is stirred at room temperature for 1h. To the solution is added (2*R*)-2-((*1R,3αS,7αR,E*)-4-(2-((4*R*,8*R*)-4,8-bis(*tert*-butyldimethylsilyloxy)spiro[2.5]octan-6-ylidene)ethylidene)-7*α-*methyloctahydro-1H-inden-1-yl)propyl 4-methylbenzenesulfonate (Example 57-H, 52 mg, 0.07 mmol). The mixture is stirred at room temperature overnight and then quenched with water and extracted with DCM. The organic phases are combined and washed with brine, dried with Na₂SO₄, and *concentrated in vacuo.* Purification on a short column gives the title compound (46 mg) as a colorless oil.

### EXAMPLE 70-B

This example describes the synthesis of the title compound that is prepared from the product of Example 15B and 3-(2-methyl-2-hydroxy-ethyl)-phenylmercaptan, according to the procedure described in Example 56.

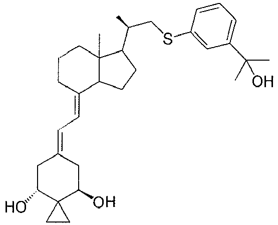

To a solution of 2-(3-((2*R*)-2-((*1R,3αS,7αR,E*)-4-(2-((4*R*,8*R*)-4,8-bis(*tert-*butyldimethylsilyloxy)spiro[2.5]octan-6-ylidene)ethylidene)-7α-methyloctahydro-1H-inden-1-yl)propylthio)phenyl)propan-2-ol (Example 70-A, 46 mg, 0.06 mmol) in MeOH is added CSA (800 mg), and the mixture is stirred at room temperature for 8 hours. It is quenched with water, extracted with DCM, and then *concentrated in vacuo.* Purification provides the title compound (21 mg) as a colorless oil. ¹H NMR(400 MHz, CDCl₃): δδ0.63 (s, 3 H), 1.08 (d, 3 H, *J* = 6.4Hz), 1.25-1.26 (m, 4 H), 1.58 (s, 6 H), 1.92-2.72 (m, 16 H), 2.78 (d, 1 H, *J* = 4 Hz), 3.24 (d, 1 H, *J* = 8 Hz), 4.47 (d, 2 H, *J* = 3.6 Hz), 5.09 (d, 1 H, *J* = 2.8 Hz), 6.17 (d, 1 H, *J* = 7.2 Hz), 6.50 (d, 1 H, *J* = 11.2 Hz), 7.22-7.19 (m, 3 H), 7.50 (s, 1 H). ¹³C-NMR (400 MHz, CDCl₃): δ13.29 (C18), 18.87 (C21), 24.02 (C15,11), 26.17(C16), 27.11 (C9), 31.71 (C26,27), 35.15 (C20), 36.84 (C10), 39.13 (C12), 40.49 (C22), 41.15(C4),46.48-46.53 (C13), 54.15 (C14), 55.78 (C17), 71.16 (C3), 71.48 (C1), 72.51 (C25), 107.81 (C19), 119.82 (C7), 121.96(C28), 125.07 (C29), 125.30(C31),127.29 (C6), 128.69 (C30), 131.37 (C8), 137.46(C23),141.70 (C5), 149.82 (C24), 151.96 (C2).

In Table 1, the following compounds in Examples 71-81 were prepared from the corresponding various intermediates described herein.

**Table 1.**

| **Example** | **Name** | **Structure** |
|---|---|---|
| 71 | (4*R*,8*R*)-6-((*E*)-2-((1*S*,7*S*)-1-((*R*)-1*-*(3-methylbutoxy)ethyl)-7a-methyldihydro-1H-inden-4(*2H*,*5H,6H,7H,7αH*)-ylidene)ethylidene)spiro[2.5]octane-4,8-diol (Vida-81) | 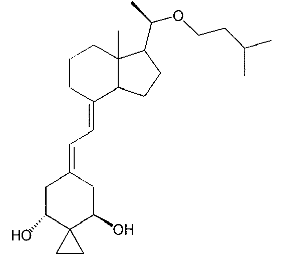 |
| 72 | (4*R*,8*R*)-6-((*E*)-2-((3*αS*,7*αS*)-1-((*R*)*-*1-((S)-2,3-dimethylbutoxy)ethyl)-7α-methyldihydro-1H-inden-4(*2H,5H,6H,7H,7αH*)-ylidene)ethylidene)spiro[2.5]octane-4,8-diol (Vida-84) | 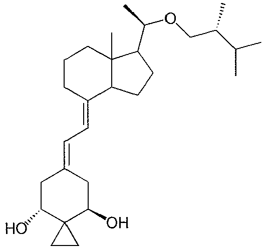 |
| 73 | (4*R*,8*R*)-6-((*E*)-2-((1*S*,7*αS*)-1-((*R*)-1-(3-ethyl-pentyloxy)ethyl)-7α-methyldihydro-1 H-inden-4(*2H,5H,6H, 7H, 7αH*)-ylidene)ethylidene)spiro[2.5]octane-4,8-diol (Vida-90) | 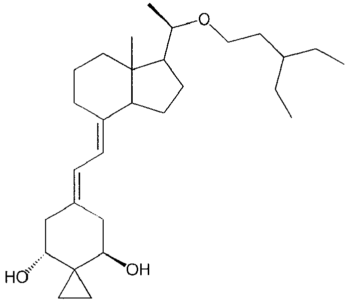 |
| 74 | (4*R,*8*R*)-6-((*E*)-2-(1-((*R*)-1-(3-isopropylphenoxy)propan-2-yl)-7α-methyldihydro-1 H-inden-4(*2H,5H,6H,7H, 7αH*)-ylidene)ethylidene)spiro[2.5]octane-4,8-diol (Vida-137) | 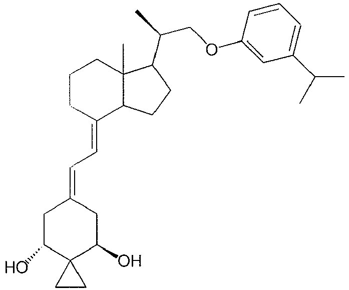 |
| 75 | (4*R*,8*R*)-6-((*E*)-2-(*1*-((*R*)-1-(3-isopropylphenylthio)propan-2-yl)-7α-methyldihydro-1 H-inden-4(*2H,5H,6H,7H, 7αH*)-ylidene)ethylidene)spiro[2.5]octane-4,8-diol (Vida-138) | 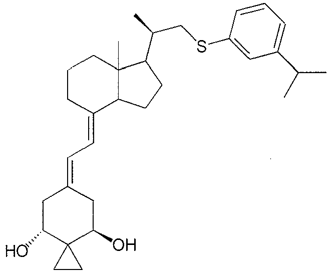 |
| 76 | (*1R,3R*)-5-((*E*)-2-(1-(1-(2,(*S*)*-*3-dimethylbutoxy)ethyl)-7α-methyldihydro-*1H* inden-4(*2H,5H,6H,7H,7αH*)-ylidene)ethylidene)-2-methylenecyclohexane-1,3-diol (Vida-85) | 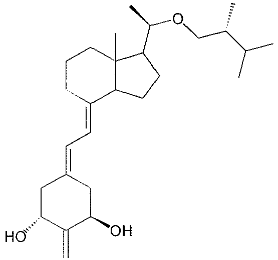 |
| 77 | (*1R,3R*)-5-((*E*)-2-((*1S,3αS,7αS*)-1-((*R*)-1-(3-ethyl-pentyloxy)ethyl)-7α-methyldihydro-*1H* inden-4(*2H,5H,6H,7H,7αH*)-ylidene)ethylidene)-2-methylenecyclohexane-1,3-diol (Vida-91) | 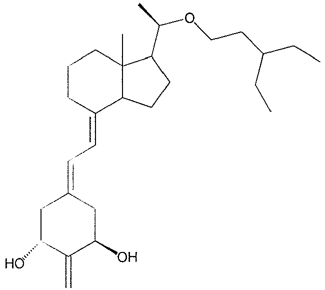 |
| 78 | (*1R,3R*)-5-((*E*)-2-((*1S,7αS*)-1-((*R*)-1-(4-ethyl-hexyloxy)ethyl)-7α-methyldihydro-*1H*-inden-4(*2H,5H,6H,7H,7αH*)-ylidene)ethylidene)-2-methylenecyclohexane-1,3-diol (Vida-101) | 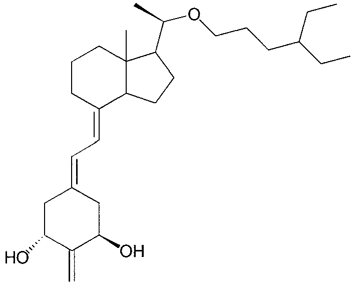 |
| 79 | (1*R*,3*R*)-5-((*E*)-2-(1-((*R*)-1-(3-isopropylphenoxy)propan-2-yl)-7α-methyldihydro-1H-inden-4(*2H,5H,6H,7H,7αH*)-ylidene)ethylidene)-2-methylenecyclohexane-1,3-diol (Vida-117) | 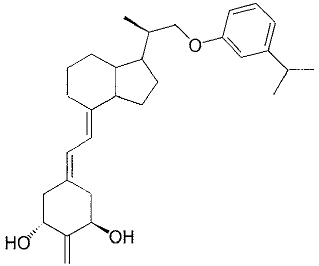 |
| 80 | (1*R*,3*R*)-5-((*E*)-2-(1-((*R*)-1-(3-isopropylphenylthio)propan-2-yl)-7α-methyldihydro-1H-inden-4(*2H,5H,6H,7H,7αH*)-ylidene)ethylidene)-2-methylenecyclohexane-1,3-diol (Vida-123) | 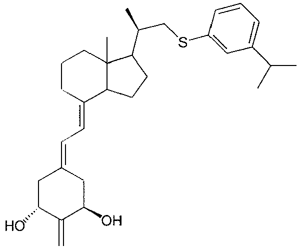 |
| 81 | (*4R,8*)-6-((*E*)-2-((*1S,7αS*)-1-((R)-1-(4-ethyl-hexyloxy)ethyl)-7α-methyldihydro-*1H*-inden-4(*2H,5H,6H,7H,7αH*)-ylidene)ethylidene)spiro[2.5]octane-4,8-diol (Vida-100) | 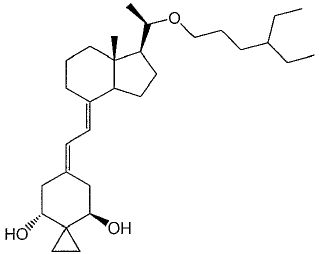 |

### EXAMPLE 82

This example demonstrates the determination of the maximal absorbance wavelength and extinction coefficient of various compound of formula (I) in an embodiment of the invention.

To determine the maximal absorbance wavelength (ODₘₐₓ) and the extinction coefficients for the compounds, each compound is diluted in a 50:50 solution (by volume) of de-ionized water and ethanol at 100 µM and scanned by a spectrophotometer.

Fig. 1 shows the result from a typical absorbance profile from a spectrophotometer scan. The results show that the maximal absorbance wavelength (OD_{maX}) for Vida-5 is at 253 nm and the the extinction coefficient is 48360. Table 2 shows a summary of the ODₘₐₓ and the the extinction coefficient for selected compounds.

**Table 2.**

| Compound # | ODmax, nM | Coefficient |
|---|---|---|
| Vida-1 | 253 | 29550 |
| Vida-4 | 253 | 23610 |
| Vida-5 | 253 | 48360 |
| Vida-10 | 253 | 46990 |
| Vida-11 | 254 | 60100 |
| Vida-20 | 253 | 23340 |
| Vida-21 | 253 | 58710 |
| Vida-36 * | 254 | 26850 |
| Vida-37 | 254 | 25720 |
| Vida-43 | 254 | 43580 |
| Vida-57 | 253 | 49670 |

| | | |
|---|---|---|
| * Reference Example | | |

### BIOLOGICAL ASSAYS

The biological activity of the compounds was assessed using both cultured cells and animal models.

Currently no *in vitro* assays have been shown to be able to reliably predict a compound's hypercalcemic side effect profile *in vivo.* Therefore, compounds were evaluated using normal mice to determine their potencies in raising serum calcium. PTH suppression also was determined in normal mice.

Some compounds were tested in HL-60 promyelocytic leukemia cells and a primary culture of human coronary artery smooth muscle cells (HCASMC) for CYP24A1 induction. It is well documented that CYP24A1 has a VDRE in its promoter region and is a specific target gene of VDR (Meyer et al., 2007, J Biol Chem 282: 22344-22352). A compound that induces CYP24A1 demonstrates its activity in evoking the VDR signaling pathway. Compounds also were tested in HCASMC for their effects in inducing VDR. To assess the suitability of a compound for modulating immune responses, compounds were tested in HL-60 for their effects in inducing CD14 expression. To assess the suitability of a compound for treating thrombosis, compounds also were tested in HCASMC for their effects in modulating thrombomodulin and thrombospondin-1.

Compounds also were evaluated for their effects on serum calcium and PTH suppression in the 5/6 nephrectomized uremic rats, a well-established animal model for renal insufficiency. From the published literature about calcitriol, paricalcitol and doxercalciferol for the treatment of secondary hyperparathyroidism in CKD, the 5/6 nephrectomy rat model was shown to be highly predictive of clinical utility regarding the PTH suppression doses and hypercalcemic effects of these drugs. For example, paricalcitol shows a therapeutic index of ∼3-fold over calcitriol in the uremic rats (Slatopolsky et al., 1998, Am J Kidney Dis 32(2 Suppl 2): S40-7). Similar to the uremic rat data, clinical studies demonstrate that paricalcitol is about 3-fold less potent in suppressing PTH and 10-fold less potent in raising serum calcium than calcitriol, and therefore paricalcitol is considered to have a 3-4-fold wider therapeutic index (efficacy vs. hypercalcemic side effects) than calcitriol (Martin et al., 2001, Am JKidney Dis, 38(5 Suppl 5): S34-40).

Similar to CKD patients who have proteinuria, endothelial dysfunction (Ochodnicky et al., 2006, J Nephrol 19: 246-258) and left ventricular hypertrophy (Curtis et al., 2005, Cardiol Clin 23: 275-284), the 5/6 nephrectomy rats develop proteinuria, severe endothelial dysfunction, and left ventricular hypertrophy. Thus, the rats are useful for evaluating the efficacy compounds on proteinuria and cardiovascular parameters.

Each of these activity assessments is described in detail below.

### EXAMPLE 83

This example demonstrates the ability of compounds of formula (I) to induce the expression of CYP24A1 and CD14 in human HL-60 promyelocytic leukemia cells in an embodiment of the invention.

Compounds are tested in human HL-60 promyelocytic leukemia cells to assess their potency in inducing the expression of CYP24A1 and CD14. HL-60 cells are cultured in IMDM supplemented with 20% heat-inactivated fetal bovine serum at 37 °C in a humidified 5% CO₂-95% air atmosphere. Cells are plated into 6 well plates and treated with test agent for 24 h. Cells are lysed with the addition of 1 mL of Trizol and RNA prepared according to the manufacturer's protocol.

Real-time reverse transcription-PCR is performed with the forward and reverse PCR primers and 250 nM of the TaqMan™ probe specifically designed for CYP24A1 or CD14. After the data acquisition, the CYP24A1 or CD14 mRNA expression level is normalized to the GAPDH mRNA level. Figs. 2A and 2B show the results from a typical CYP24A1 (Fig. 2A) and CD14 (Fig. 2B) expression study. The results show that Vida-5 is potent in inducing the expression of CYP24A1 and CD14 with EC₅₀ (the half maximal effective concentration) values at 5.6 and 4.1 nM, respectively.

Table 3 shows a summary of the EC₅₀ values for selected compounds on inducing the expression of CYP24A1 and CD14 in HL-60 cells. The results show that the test compounds activate VDR, leading to the modulation of VDR target genes such as CYP24A1.

**Table 3.**

| | CYP24A1 | CD14 |
|---|---|---|
| Compound # | EC₅₀, nM | EC₅₀, nM |
| Vida-1 | 69 | 22 |
| Vida-4 | 190 | 31 |
| Vida-5 | 7.3 | 3.7 |
| Vida-10 | 6.8 | 0.4 |
| Vida-11 | 0.8 | 0.9 |
| Vida-20 | 6.7 | 1.6 |
| Vida-21 | 0.8 | 1.1 |
| Vida-36 * | 249200 | 34000 |
| Vida-37 | 6.1 | 12 |
| Vida-43 | 6.9 | 63 |
| Vida-57 | 36 | 21 |
| Vida-58 | 49 | 125 |

| | | |
|---|---|---|
| Some values such as Vida-5 were from an average of 3 or more determinations. * Reference Example | | |

### EXAMPLE 84

This example demonstrates a hypercalcemic side effect profile in normal mice by compounds in accordance with an embodiment of the invention. The normal mice model is a convenient and quick way to assess the hypercalcemic side effect profile of compounds. At the same time, data on serum PTH can be collected.

Male C57/BL mice at 18 -20 g are dosed with the test agent at 100 µl/mouse (concentrations as indicated) i.p. once daily for 3 consecutive days or at 10 ml/kg, p.o. gavage, once daily for 5 days. The animals were harvested 24 h after the last dose by anesthetization with ketamine/xylazine. Blood was collected from the aorta. Serum total calcium was measured using the QuantiChrom Calcium Assay kit and/or the Roche Hitachi 912 Chemistry Analyzer. Serum PTH was measured using a PTH Immunoassay. Vehicle control was 100 µl/mouse of 5% EtOH/95% propylene glycol for i.p. dosing or 10 ml/kg of 20% Hydroxypropyl-β-Cyclodextrin for p.o. gavage dosing.

For serum chemistry data, group mean ± SEM are presented. Differences between vehicle and compound-treated groups are assessed using a one-way ANOVA followed by a Dunnett's post-hoc test. Figs. 3A and 3B show the results from a typical p.o. gavage dosing study (n=4-9 per group). Fig. 4 shows the results from a typical i.p. dosing study.

This model is a convenient way to determine the effect of a compound in inducing a significant increase in serum calcium. It also indicates the effect of a compound on decreasing the serum PTH level. Vida-5 starts to raise serum calcium at 5 µg/kg as shown in Fig. 3A, but starts to suppress PTH at 0.05 µg/kg as shown in Fig. 3B. Vida-1 starts to raise serum Ca at 0.01 µg/kg (i.p. dosing). Vida-4 is at least 100-fold less potent in raising serum Ca than Vida-1 as shown in Fig. 4. Vida-11 and Vida-21 start to raise serum Ca at 0.1 µg/kg (i.p. dosing). Vida-5, Vida-10 and Vida-20 start to raise serum Ca at 1 ug/kg (i.p. dosing). Vida-37 starts to raise serum Ca at 10 µg/kg (i.p. dosing), while Vida-36, Vida-43, Vida-57 and Vida-58 do not raise serum Ca at 10 ug/kg (i.p. dosing).

### EXAMPLE 85

This example demonstrates the use of human coronary artery smooth muscle cells to test selected compounds for the purpose of investigating the effects of compounds in accordance with an embodiment of the invention on human vascular cells.

Compounds were tested in a primary culture of HCASMC to assess their potency in inducing the expression of CYP24A1 and VDR.

Primary culture of HCASMC was grown in smooth muscle growth medium SmGM-2 containing 5.5 mM glucose, 5% FBS, 50 µg/ml gentamicin, 50 ng/ml amphotericin-B, 5 µg/ml insulin, 2 ng/ml hFGF, and 0.5 ng/ml hEGF at 37 °C in a humidified 5% CO₂-95% air. Cells were grown to >80% confluence and used within five passages.

Cells were plated into 6 well plates and treated with test agent for 24 h. Cells were lysed with the addition of 1 mL of Trizol and RNA prepared according to the manufacturer's protocol.

Real-time reverse transcription-PCR was performed with the forward and reverse PCR primers and 0.1 mM of the TaqMan™ probe specifically designed for CYP24A1 or VDR. After the data acquisition, the CYP24A1 or VDR mRNA expression level was normalized to the GAPDH mRNA level. Fig. 5A shows the result from a typical CYP24A1 expression study. Group mean ± STDEV (standard deviation) are presented. The results show that Vida-5 is potent in inducing CYP24A1 expression with an EC₅₀ at 2.9 nM. Fig. 5B shows the result from a typical VDR expression study. The results show that Vida-5 is potent in inducing VDR expression with an EC₅₀ at 1.3 nM.

Table 4 shows a summary of the EC₅₀ values for selected compounds in inducing CYP24A1 and VDR.

**Table 4.**

| Compound | EC₅₀ for CYP24A1 induction, nM | EC₅₀ for VDR induction, nM |
|---|---|---|
| Vida-5 | 2.0 | 3.2 |
| Vida-10 | 0.39 | 0.61 |
| Vida-11 | 0.39 | 1.4 |
| Vida-21 | 1.8 | 2.5 |

| | | |
|---|---|---|
| Some values such as Vida-5 were from an average of 2 or more determinations. | | |

### EXAMPLE 86

This example demonstrates the effects of selected compounds in accordance with an embodiment of the invention on serum creatinine, BUN, PTH, phosphorus, and calcium in 5/6 nephrectomized uremic rats.

The nephrectomy of male, Sprague Dawley, 5/6 rats was performed using a standard two-step surgical ablation procedure (starting body weight ∼200 gm) as previously described (Slatopolsky et al., 1998, Am J Kidney Dis 32: S40-47). Sham operated rats were used as control. The rats were on a diet containing 1.13% calcium and 0.94% phosphorus. Six weeks after the second surgery, treatment was initiated with vehicle (5% ethanol + 95% propylene glycol, 0.4 ml/kg) or test agent by intraperitoneal injection (i.p.), 3 times/week, for two weeks (n ≥ 12 per group). Twenty-four hours after the last dose, retro-orbital venous blood was collected for measurement of PTH and other endpoints.

Calcium, serum phosphorus, creatinine, and blood urea nitrogen (BUN) concentrations were measured by a chemistry analyzer (LX-20, Beckman). Serum PTH was measured using a rat intact parathyroid hormone (i-PTH) ELISA kit. For serum chemistry data, group mean ± SEM are presented. A t-test was used to analyze differences between baseline 6W (6 weeks after surgery before treatment) and 8W (8 weeks after surgery after two weeks of compound treatment).

Figs. 6A and 6B show the effects of Vida-5 on serum creatinine and blood urea nitrogen (BUN) results. Serum creatinine and BUN levels were significantly and uniformly elevated in all 5/6 nephrectomized (NX) rats compared to Sham rats 6 weeks after surgery. Treatment with Vida-5 at the 4 lower doses, had no significant effect on serum creatinine, but the highest dose reduced serum creatinine. Vida-5 also significantly reduced BUN at the two higher doses (0.16 and 0.64 µg/kg).

Figs. 7A and 7B show that treatment with Vida-11 at 3 different doses for 2 weeks had no dose-dependent effect on serum creatinine or BUN.

Figs. 8A and 8B show that Vida-5 at the 5 tested doses did not have a significant effect on raising serum phosphorus (P) and calcium (Ca).

Figs. 9A and 9B show that Vida-11 elevated serum Ca in a dose-dependent manner, but did not show a significant effect on serum P.

Figs. 10A and 10B show the serum PTH results. Vida-5 (Fig. 10A) and Vida-11 (Fig. 10B) at the tested doses significantly suppress PTH levels in a dose-dependent manner.

Vida-10 also was tested in the 5/6 NX uremic rats. Vida-10 did not significantly affect serum creatinine, BUN, or phosphorus. Vida-10 and Vida-11 have different calcemic profiles from one another. More specifically, Vida-10 is at least 64-fold less potent in raising serum Ca than Vida-11 (Fig. 11A), but about 16-fold less potent in suppressing PTH (Fig. 11B).

In the 5/6 NX uremic rats, Vida-5 starts to suppress serum PTH at 0.004 µg/kg, but does not raise serum Ca even at 0.64 µg/kg. Vida-10 starts to suppress serum PTH at 0.16 µg/kg, but does not raise serum Ca, even at 0.64 µg/kg. Vida-11 at 0.01 µg/kg suppresses serum PTH, but also raises serum Ca. From the literature, calcitriol starts to suppress serum PTH at 8 ng/rat (∼0.02 µg/kg) and raises serum Ca at 4 ng/rat (∼0.01 µg/kg) (Slatopolsky et al., 1998, Am J Kidney Dis 32(2 Suppl 2): S40-7). Paricalcitol, considered the best in the class for the treatment of secondary parathyrodisim in CKD patients, starts to suppress serum PTH at 8 ng/rat (∼0.02 µg/kg) and raises serum Ca at 25 ng/rat (∼0.0625 µg/kg) 0.083 µg/kg in the uremic rats (Slatopolsky et al., 1998, Am J Kidney Dis 32(2 Suppl 2): S40-7). In a different study, paricalcitol starts to suppress serum PTH and raises serum Ca at 0.083 µg/kg in the uremic rats (Noonan et al., 2008, Nephrol Dial Transplant 23:3824-3830). The hypercalcemic side effect is a critical limitation for this class of compounds to be applied to wider therapeutical indications.

### EXAMPLE 87

This example demonstrates a biological assay to test the endothelial function in 5/6 nephrectomized uremic rats.

The experimental conditions using the 5/6 (subtotal) nephrectomized rats were as described above. Twenty-four hours after the last dose, the animals were sacrificed and the aorta tissues were harvested for the determination of endothelial function.

Rats were anesthetized with pentobarbital sodium (i.p., 35 mg/kg). Thoracic aortas were excised in a cold modified Krebs solution (see below) and a 3 mm aortic ring was suspended in 10 mL tissue baths under 0.5 grams of resting tension in a modified Krebs solution containing (g/L): NaCl 6.9169, KCl 0.3499, NaHCO₃ 2.0998, MgSO₄ 0.2901, KH₂ PO₄ 0.1604, CaCl₂ 0.2663, glucose 1.9994, EDTA 0.026, equilibrated with 5% CO₂-95% O₂ (pH 7.4 at 37 °C). Aortas were sensitized by the addition of phenylephrine (PE, 3 µM) with 10 min washouts between intervals. Aortas were precontracted with PE (3 µM), and the endothelium-dependent vasodilator acetylcholine (ACh) was added in half-log increments (10⁻⁹ mol/L - 10^{-4.5} mol/L) at 3 - 5 min intervals, allowing time for the effect of ACh to plateau. After a 60 min washout, aortas were precontracted with PE (3 µM) and subsequently treated with endothelial-independent vasodilator sodium nitroprusside (SNP; 10⁻⁹ mol/L - 10⁻⁶ mol/L) at 3 - 5 min intervals, allowing time for the effect to plateau. Data were recorded with the BL-420F Data Acquisition & Analysis System.

ACh and SNP-induced relaxation were calculated as the % relaxation of the PE-induced precontraction. Differences in vascular function were determined using a two-way ANOVA, followed by a Bonferonni post-hoc test. Fig. 12A shows that a 2-week treatment with Vida-5 produced a dose-dependent improvement in acetylcholine-induced endothelial-dependent relaxation. As a comparison, Fig. 12B shows that Vida-5 at the tested doses had no significant effect on sodium nitroprusside (SNP)-induced endothelial-independent relaxation. SNP serves as a source of nitric oxide and induces relaxation via an endothelial-independent pathway.

Figs. 13A and 13B show that Vida-11 also produced a dose-dependent improvement in acetylcholine-induced endothelial-dependent relaxation. Vida-11 at the tested doses also had no significant effect on SNP-induced relaxation.

Fig. 14 shows that Vida-10 produced a dose-dependent improvement in acetylcholine-induced endothelial-dependent relaxation. Treatment with 0.16 and 0.64 µg/kg of Vida-10 improved acetylcholine-induced endothelial-dependent relaxation back to the Sham level. Vida-10 at the tested doses also had no significant effect on SNP-induced relaxation.

Vida-11 is more potent in suppressing PTH, but Vida-10 is more potent in improving endothelial function. These results show that Vida-5, Vida-10 and Vida-11 are potent in improving endothelial function. Since Vida-5, Vida-10, and Vida-11 exhibit different serum Ca and PTH profiles, the results also demonstrate that a compound's effect on improving endothelial function is independent of its effect on serum Ca and PTH.

### EXAMPLE 88

This example demonstrates a biological assay to test left ventricular hypertrophy in 5/6 nephrectomized uremic rats.

The experimental conditions using the 5/6 (subtotal) nephrectomized rats were as described above. Twenty-four hours after the last dose, the rats were anesthetized with pentobarbital sodium (35 mg/kg, i.p.). The animals were sacrificed by cervical dislocation. The hearts were taken and washed with cold saline. After removing the residual saline with filter paper, the weight of the freshly dissected hearts and left ventricles were measured, and the ratio of left ventricle weight to body weight (LVW/BW) and/or left ventricle weight to heart weight (LVW/HW) were calculated. Thereafter, the left ventricles were rapidly put into liquid nitrogen until ready for histological and immunohistochemical analysis.

To examine the cardiomyocyte morphology, the left ventricular tissue was fixed in a 4% formaldehyde-phosphate-buffered saline (pH 7.4) solution overnight. The samples were embedded in wax and cut into 4 µm sections. The sections were stained with hematoxylin-eosin, and examined under a microscope.

To determine the diameter of cardiomyocytes, a previously published method was followed (Xiang et al., 2005, Am J Physiol Endocrinol Metab 288: E125-132). The sections of left ventricles were stained with FITC-labeled wheat germ agglutinin (1:5 dilution) for 2 h at room temperature and then examined under a fluorescence microscope to visualize the myocyte membrane. The relative size of the cardiomyocytes was quantified by measuring the diameter of the myocytes, which was the distance between the two plasma membranes of a cell in longitudinal section. The measurement was done using ImageJ software. Data were obtained from 30 cells randomly selected from 5-10 microscopic fields of left ventricle slides.

Group mean ± SEM are presented. Differences between SHAM, vehicle, and treated animals were assessed using a t-test, a one-way ANOVA followed by a Dunnett's post-hoc test, or a two-way ANOVA, followed by a Bonferonni post-hoc test. Similar to the human CKD condition, the 5/6 (subtotal) nephrectomized rats are known to develop left ventricular hypertrophy (Wolf et al., 2000, Journal of Cardiovascular Pharmacology 36: S348-350). Some animals were sacrificed before treatment to evaluate the development of left ventricular hypertrophy.

Figs. 15A and 15B show that, at 8 weeks after the renal ablation surgery, Vida-5 at the tested doses produced a dose-dependent effect on reducing the LVW/BW and LVW/HW.

Figs. 16A and 16B show that Vida-11 exhibited mixed results that the compound reduced LVW/BW at the two lower doses, but not at 0.16 µg/kg. All the other parameters such as serum Ca, P, and PTH also were collected from these studies to serve as controls, and the values were similar to those described above. Since Vida-11 is much more hypercalcemic than Vida-5, there is a likelihood that the effect of Vida-11 on reducing LVH might be partially compromised by hypercalcemia.

Fig. 17 shows that Vida-10 produced a dose-dependent effect on reducing the LVW/BW. Vida-10 at 0.16 and 0.64 µg/kg had no effect on serum Ca, but significantly reduced the LVW/BW. In this study, Vida-5 at 0.04 g/kg plus losartan (0.025 mg/ml in drinking water) was compared with Vida-5 at 0.04 g/kg alone, and the result showed that Vida-5 at 0.04 g/kg completely normalized LVW/BW with or without the addition of losartan, an angiotensin II receptor antagonist.

Figs. 18A, 18B, 18C, and 18D show that cardiomyocytes were markedly hypertrophic in the NX-vehicle treated animals as demonstrated by the cardiomyocyte morphology. Vida-5 at 0.01 µg/kg improved the morphology of the cardiomyocytes, and Vida-5 at 0.16 µg/kg nearly restored the morphology of the cardiomyocytes back to the Sham level. These results confirm the LVW/BW and LVW/HW results that treatment with Vida-5 improves the left ventricular hypertrophy condition in the NX uremic rats.

The sections of left ventricles were stained with FITC-labeled wheat germ agglutinin to determine the diameter of cardiomyocytes, and the results are shown in Fig. 19. Vida-5 at doses 0.01-0.64 µg/kg significantly reduced the cardiomyocyte diameter.

### EXAMPLE 89

This example demonstrates the treatment of proteinuria in 5/6 nephrectomized uremic rats comprising a compound or salt therof of Formula (I).

The experimental conditions using the 5/6 (subtotal) nephrectomized rats were as described above. Urinary protein excretion was determined by urinary collection over a period of 24 h in metabolic cages at two time points: 24 h before the first dose and 24 hr after the last dose. Each animal was placed in a metabolic cage and urine was collected during a period of 24 h. Urinary protein concentration was determined by the biuret method (Morozova and Baryshnikova, 1991, Lab Delo 2:23-5). Total protein excretion per day was calculated by multiplying urinary protein concentration by total urine volume produced during the 24 h period.

Group mean ± SEM are presented. Differences before versus after treatment were assessed using a t-test. Differences between SHAM, vehicle, and treated animals were assessed using a one-way ANOVA followed by a Dunnett's post-hoc test.

Figs. 20A and 20B show that the protein concentration (mg/ml) and the total protein excretion per day were significantly elevated in the NX vehicle group. Vida-5 and Vida-11 at the tested doses significantly reduced proteinuria when comparing before treatment versus after treatment. The results demonstrate that compounds of Formula (I) are useful for the treatment of proteinuria. In addition, since Vida-5 and Vida-11 exhibit different serum Ca profiles, the results also show that a compound's effect on improving proteinuria is independent of its effect on serum Ca.

### EXAMPLE 90

This example demonstrates the modulation of thrombosis markers such as thrombospondin-1 and thrombomodulin in human smooth muscle cells comprising a compound or salt therof of Formula (I).

Human coronary artery smooth muscle cells (HCASM) were incubated with Vida-5 or Vida-11 at different concentrations for 48 h and then were solubilized in 50 µl of SDS-PAGE sample buffer. The protein content in each sample was determined by the bicinchoninic acid protein assay. Samples were resolved by SDS-PAGE using a 4-12% NuPAGE gel, and proteins were electrophoretically transferred to polyvinylidene fluoride membrane for Western blotting. The membrane was blocked for 1 h at 25 °C with 5% nonfat dry milk in phosphate buffered saline Tween-20 (PBS-T) and then incubated with a mouse anti-thrombospondin-1 monoclonal antibody (2,000-fold dilution), or a mouse anti-TM monoclonal antibody (2,000-fold dilution) in PBS-T overnight at 4 °C.

The membrane was washed with PBS-T and incubated with a horseradish peroxidase-labeled anti-mouse antibody for 1 h at 25 °C. The membrane was then incubated with Amersham ECL Plus Western Blotting Detection Reagents. Specific bands were visualized by the Multiimage II of Alpha Innotech imaging system. Band intensity was quantified using Spot Denso.

Thrombospondin-1 (THBS1) is an adhesive glycoprotein that mediates cell-to-cell and cell-to-matrix interactions. THBS1 can bind to fibrinogen, fibronectin, laminin, type V collagen, and integrins alpha-V/beta-1, and has been shown to play roles in platelet aggregation, angiogenesis, and tumorigenesis. THBS1 expression in the vascular wall is significantly increased in injured vessels and in stent-induced neointima (Sajid et al., 2001, J. Investig. Med. 49:398-406; Zohlnhofer et al., 2001, Circulation 103:1396-1402). Thrombomodulin (TM) is a monomeric transmembrane protein that serves as a cell surface receptor for thrombin. When thrombin binds to TM, it undergoes a conformational change, resulting in an altered substrate specificity to activate protein C. Under normal conditions, many of these factors involved in thrombosis, such as THBS1 and TM, are predominantly localized on endothelial cells. However, vascular injury often results in altered expression patterns for these factors. For example, in both human and mouse in atherosclerotic vessels, TM has been shown to be markedly down-regulated in endothelial cells and SMC may become a relevant source of TM under pathological conditions such as in advanced atherosclerosis (Yoshii et al., 2003, Med. Electron. Microsc. 36:165-172; Tohda et al., 1998, Arterioscler. Thromb. Vasc. Biol. 18:1861-1869).

Fig. 21A shows that the thrombospondin-1 protein was significantly reduced in smooth muscle cells by Vida-5 and Vida-21. Fig. 21B shows that the thrombomodulin protein was increased in smooth muscle cells by Vida-5 and Vida-21.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description.

## Claims

1. A compound of Formula (I) 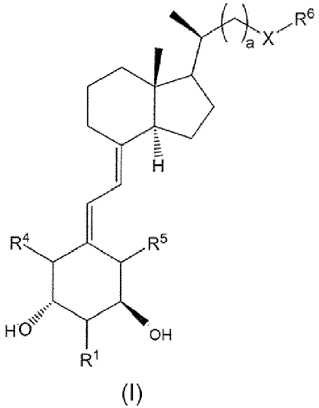 wherein
R¹ is =CH₂ ; or
R¹ and the carbon to which it is bonded together form a cyclopropyl group;
R⁴ and R⁵ are the same or different and each is selected from the group consisting of H, C₁₋₁₂ alkyl, hydroxyl, C₁₋₁₂ alkoxy, and halo;
wherein said C₁₋C₁₂ alkyl and said C₁₋C₁₂ alkoxy are optionally and independently substituted with one or more substituents selected from the group consisting of alkyl, alkenyl, alkynyl, cycloalkyl, aroyl, halo, monohaloalkyl, dihaloalkyl, trihaloalkyl, monohaloalkoxy, dihaloalkoxy, trihaloalkoxy, hydroxy, alkoxy, alkoxycarbonyl, cycloalkyloxy, heterocyclooxy, oxo, alkanoyl, aryl, arylalkyl, alkylaryl, heteroaryl, heteroarylalkyl, alkylheteroaryl, heterocyclyl, aryloxy, alkanoyloxy, amino, monoalkylamino, dialkylamino, trialkylamino, arylamino, arylalkylamino, cycloalkylamino, heterocycloamino, alkanoylamino, aroylamino, aralkanoylamino, mercapto, alkylthio, arylthio, arylalkylthio, cycloalkylthio, heterocyclothio, alkylthiono, arylthiono, arylalkylthiono, alkylsulfonyl, arylsulfonyl, arylalkylsulfonyl, sulfamato NH₂SO₂O-, alkylsulfamato, sulfonamido-SO₂NH₂, alkyl sulfonamido, nitro, cyano, carboxy, carbamyl NH₂CO-, monosubstituted carbamyl selected from alkyl-NH-CO, aryl-NH-CO and arylalkyl-NH-CO and disubstituted carbamyl selected from dialkyl-N-CO, diaryl-N-CO and alkyl-aryl-N-CO;
R⁶ is C₁₋₁₂ alkyl or aryl;
wherein said C₁₋C₁₂ alkyl is optionally and independently substituted with one or more substituents selected from the group consisting of alkyl, alkenyl, alkynyl, cycloalkyl, aroyl, halo, monohaloalkyl, dihaloalkyl, trihaloalkyl, monohaloalkoxy, dihaloalkoxy, trihaloalkoxy, hydroxy, alkoxy, alkoxycarbonyl, cycloalkyloxy, heterocyclooxy, oxo, alkanoyl, aryl, arylalkyl, alkylaryl, heteroaryl, heteroarylalkyl, alkylheteroaryl, heterocyclyl, aryloxy, alkanoyloxy, amino, monoalkylamino, dialkylamino, trialkylamino, arylamino, arylalkylamino, cycloalkylamino, heterocycloamino, alkanoylamino, aroylamino, aralkanoylamino, mercapto, alkylthio, arylthio, arylalkylthio, cycloalkylthio, heterocyclothio, alkylthiono, arylthiono, arylalkylthiono, alkylsulfonyl, arylsulfonyl, arylalkylsulfonyl, sulfamato NH₂SO₂O-, alkylsulfamato, sulfonamido -SO₂NH₂, alkyl sulfonamido, nitro, cyano, carboxy, carbamyl NH₂CO-, monosubstituted carbamyl selected from alkyl-NH-CO, aryl-NH-CO and arylalkyl-NH-CO, and disubstituted carbamyl selected from dialkyl-N-CO, diaryl-N-CO and alkyl-aryl-N-CO; and
wherein said aryl is optionally independently substituted with one or more substituents selected from the group consisting of alkyl, alkenyl, alkynyl, cycloalkyl, aroyl, halo, monohaloalkyl, dihaloalkyl or trihaloalkyl, monohaloalkoxy, dihaloalkoxy or trihaloalkoxy, hydroxy, alkoxy, alkoxycarbonyl, cycloalkyloxy, heterocyclooxy, oxo, alkanoyl, arylalkyl, alkylaryl, heteroaryl, heteroarylalkyl, alkylheteroaryl, heterocyclyl, aryloxy, alkanoyloxy, amino, monoalkylamino, dialkylamino or trialkylamino, arylamino, arylalkylamino, cycloalkylamino, heterocycloamino, alkanoylamino, aroylamino, aralkanoylamino, mercapto, alkylthio, arylthio, arylalkylthio, cycloalkylthio, heterocyclothio, alkylthiono, arylthiono, arylalkylthiono, alkylsulfonyl, arylsulfonyl, arylalkylsulfonyl, sulfamato NH₂SO₂O-, alkylsulfamato, sulfonamido-SO₂NH₂, alkyl sulfonamido, nitro, cyano, carboxy, carbamyl NH₂CO--, and monosubstituted carbamyl selected from alkyl-NH-CO, aryl-NH-CO and arylalkyl-NH-CO, and disubstituted carbamyl selected from dialkyl-N-CO, diaryl-N-CO and alkyl-aryl-N-CO;
X is oxygen or sulfur; and
a is 0-5;
provided that when a is 1-5, then R⁶ is optionally substituted aryl, the optional substituents of aryl being as defined above;
and when a is 0, R⁶ is optionally substituted C₁₋₁₂ alkyl, the optional substituents of C₁₋₁₂ alkyl being as defined above;
or a pharmaceutically acceptable salt thereof.

2. The compound or a pharmaceutically acceptable salt thereof of claim 1, wherein X is oxygen.

3. The compound or a pharmaceutically acceptable salt thereof of claim 1, wherein X is sulfur.

4. The compound or a pharmaceutically acceptable salt thereof of any one of claims 1-3, wherein R¹ is =CH₂.

5. The compound or a pharmaceutically acceptable salt thereof of any one of claims 1-3, wherein R¹ is cyclopropyl.

6. The compound or a pharmaceutically acceptable salt thereof of any one of claims 1-5, wherein R⁴ and R⁵ are each H.

7. The compound or a pharmaceutically acceptable salt thereof of any one of claims 1-6, wherein a is 0 and R⁶ is selected from one or more of (i) optionally substituted C₁₋₁₂ alkyl; and (ii) C₁₋₁₂ alkyl or hydroxy C₁₋₁₂ alkyl, the optional substituents as defined above.

8. The compound or a pharmaceutically acceptable salt thereof of claim 7, wherein R⁶ is isopentyl, 3-hydroxy-3-methylbutyl, 2,3-dimethylbutyl, 2,3-dimethyl-3-hydroxybutyl, 3-ethylpentyl, 3-ethyl-3-hydroxypentyl, 4-ethylhexyl, or 4-ethyl-4-hydroxyhexyl.

9. The compound or a pharmaceutically acceptable salt thereof of any one of claims 1-6, wherein a is 1 to 5 and R⁶ is selected from one or more of (i) optionally substituted aryl; and (ii) aryl substituted with a C₁₋₁₂ alkyl or a C₁₋₁₂ hydroxyalkyl, the optional substituents as defined above.

10. The compound or a pharmaceutically acceptable salt thereof of claim 9, wherein R⁶ is aryl substituted with isopropyl or 2-hydroxypropan-2-yl.

11. The compound or a pharmaceutically acceptable salt thereof of any one of claims 1-6, 9 and 10, wherein a is 1.

12. The compound or a pharmaceutically acceptable salt thereof of claim 1 wherein the compound is selected from the group consisting of
(1 R, 3R)-5-((E)-2-((3αS, 7αS)-1-((R)- 1 -((S)-3 -hydroxy-2,3 -dimethylbutoxy)ethyl)-7α-methyldihydro-1H-inden-4(2H, 5H, 6H, 7H, 7αH)-ylidene)ethylidene)-2-methylenecyclohexane-1 ,3-diol (Vida-5);
(4R,8R)-6-((E)-2-((1S,7αS)-1-((R)-1-(3-ethyl-3-hydroxypentyloxy)ethyl)-7α-methyldihydro-1H-inden-4(2H, 5H,6H, 7H, 7αH)-ylidene)ethylidene)spiro[2.5]octane-4,8-diol (Vida-10);
(4R, 8R)-6-((E)-2-((1S, 7αS)-1-((R)-1-(4-ethyl-4-hydroxyhexyloxy)ethyl)-7α-methyldihydro-1 H-inden-4(2H,5H,6H, 7H, 7αH)-ylidene)ethylidene)spiro[2.5]octane-4,8-diol (Vida-20);
(1R, 3R)-5-((E)-2-((1S, 3αS, 7αS)-1-((R)-1-(3-ethyl-3-hydroxypentyloxy)ethyl)-7α-methyldihydro-1 H-inden-4(2H, 5H, 6H, 7H, 7αH)-ylidene)ethylidene)-2-methylenecyclohexane-1,3-diol (Vida-11);
(1R, 3R)-5-((E)-2-((1S, 7αS)-1-((R)-1-(4-ethyl-4-hydroxyhexyloxy)ethyl)-7α-methyldihydro-1H-inden-4(2H, 5H, 6H, 7H, 7α/H)-ylidene)ethylidene)-2-methylenecyclohexane- 1,3-diol (Vida-21);
4R,8R)-6-((E)-2-((1S, 7αS)-1-((R)-1-(3-hydroxy-3-methylbutoxy)ethyl)-7α-methyldihydro-1 H-inden-4(2H,5H,6H, 7H, 7αH)-ylidene)ethylidene)spiro[2.5]octane-4,8-diol (Vida-1);
(4R,8R)-6-((E)-2-((3αS, 7aS)-1-((R)-1-((S)-3 -hydroxy-2,3 -dimethylbutoxy)ethyl)-7α-methyldihydro-1H-inden-4(2H, 5H, 6H, 7 H, 7H)-lidene)ethylidene)spiro[2.5]octane-4,8-diol (Vida-4);
(4R,8R)-6-((E)-2-(1-((R)-1-(3-(2-hydroxypropan-2-yl)phenoxy)propan-2-yl)-7α-methyldihydro-1 H-inden-4(2H,5H, 6H,7H,7αH)-ylidene)ethylidene)spiro[2.5]octane-4,8-diol (Vida-57);
(4R, 8R)-6-((E)-2-(1 -((R)-1-(3-(2-hydroxypropan-2-yl)phenylthio)propan-2-yl)-7a-methyldihydro-1 H-inden-4(2H, 5H, 6H, 7H, 7αH)-ylidene)ethylidene)spiro[2.5]octane-4,8-diol (Vida-58);
(1 R,3R)-5-((E)-2-(1-((R)-1-(3-(2-hydroxypropan-2-yl)phenoxy)propan-2-yl)-7a-methyldihydro-1 H-inden-4(2H, 5H, 6H, 7H, 7αH)-ylidene)ethylidene)-2-methylenecyclohexane-1 ,3-diol (Vida-37);
(1 R,3R)-5-((E)-2-(1-((R)-1-(3-(2-hydroxypropan-2-yl)phenylthio)propan-2-yl)-7a-methyldihydro-IH-inden-4(2H,5H, 6H, 7H, 7αH)-ylidene)ethylidene)-2-methy lenecyclohexane-1,3 -diol (Vida-43) ;
(4R, 8R)-6-((E)-2-((1S, 7αS)-1-((R)-1-(3-methylbutoxy)ethyl)-7α-methyldihydro-1H-inden-4(2H,5H,6H, 7H, 7αH)-ylidene)ethylidene)spiro[2.5]octane-4,8-diol (Vida-81);
(4R, 8R)-6-((E)-2-((3α S, 7α S)-1-((R)-1-((S)-2,3 -dimethylbutoxy)ethyl)-7α-methyldihydro-1 H-inden-4(2H,5H,6H, 7H, 7αH)-ylidene)ethylidene)spiro[2.5]octane-4,8-diol (Vida-84);
(1 R,3R)-5-((E)-2-((3αS, 7αS)-1-((R)-1-((S)-2,3-dimethylbutoxy)ethyl)-7α-methyldihydro-1H-inden-4(2H, 5H, 6H, 7H, 7αH)-ylidene)ethylidene)-2-methylenecyclohexane-1,3 -diol (Vida-85);
(4R, 8R)-6-((E)-2-((1S, 7αS)-1-((R)-1-(3-ethyl-pentyloxy)ethyl)-7α-methyldihydro-1H-inden-4(2H,5H,6H, 7H, 7αH)-ylidene)ethylidene)spiro[2.5]octane-4,8-diol (Vida-90);
(1R, 3R)-5-((E)-2-((1 S, 3αS, 7αS)-1-((R)-1-(3 -ethyl-pentyloxy)ethyl)-7a-methyldihydro-1 H-inden-4(2H,5H,6H, 7H, 7αH)-ylidene)ethylidene)-2-methylenecyclohexane-1,3 -diol (Vida-91);
(4R, 8R)-6-((E)-2-((1S, 7αS)-1-((R)-1-(4-ethyl-hexyloxy)ethyl)-7α-methyldihydro-1H-inden-4(2H,5H,6H, 7H, 7αH)-ylidene)ethylidene)spiro[2.5]octane-4,8-diol (Vida-100);
(1R,3R)-5-((E)-2-((1S, 7αS)-1-((R)-1-(4-ethyl-hexyloxy)ethyl)-7α-methyldihydro-1H-inden-4(2H, 5H, 6H, 7H, 7αH)-ylidene)ethylidene)-2-methylenecyclohexane-1,3-diol (Vida-101);
(1R,3R)-5-((E)-2-(1-((R)-1-(3-isopropylphenoxy)propan-2-yl)-7a-methyldihydro-IH-inden-4(2H,5H,6H, 7H, 7αH)-ylidene)ethylidene)-2-methylenecyclohexane-l,3-diol (Vida-117);
(1 R, 3R)-5-((E)-2-(1-((R)-1-(3 -isopropylphenylthio)propan-2-yl)-7α-methyldihydro-1 H-inden-4(2H,5H,6H, 7H, 7αH)-ylidene)ethylidene)-2-methylenecyclohexane-1,3-diol (Vida-123)
(4R, 8R)-6-((E)-2-(1-((R)-1-(3-isopropylphenoxy)propan-2-yl)-7α-methyldihydro-1H-inden-4(2H,5H,6H, 7H, 7αH)-ylidene)ethylidene)spiro[2.5]octane-4,8-diol (Vida-137); and
(4R,8R)-6-((E)-2-(1-((R)-1-(3-isopropylphenylthio)propan-2-yl)-7a-methyldihydro-1 H-inden-4(2H,5H,6H, 7H, 7αH)-ylidene)ethylidene)spiro[2.5]octane-4,8-diol (Vida-138).

13. A pharmaceutical composition comprising (i) a compound or a pharmaceutically acceptable salt thereof of any one of claims 1-12 and (ii) a pharmaceutically acceptable carrier.

14. A compound of formula (I) or a pharmaceutically acceptable salt thereof as claimed in any one of claims 1 to 12 for use in a method of treating a disease which benefits from a modulation of the vitamin D receptor.

15. A compound of formula (I) or a pharmaceutically acceptable salt thereof as claimed in any one of claims 1 to 12 for use in a method of medical treatment for a disease which is selected from the group consisting of hypocalcemia, cancer, psoriasis, hyperparathyroidism, osteoporosis, secondary hyperparathyroidism, proteinuria/renal disease progression, endothelial dysfunction, left ventricular hypertrophy, aging, metabolic syndrome, insulin resistance, obesity, viral infection, bacterial infection, musculoskeletal disorders, high blood pressure, hypertriglyceridemia, immune disorders, multiple sclerosis, myelodysplastic syndrome, proximal myopathy, seasonal affective disorder, senile warts, skin pigmentation disorders, and thrombosis.

## Patentansprüche

1. Verbindung der Formel (I): 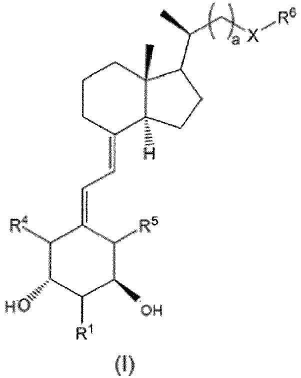 worin
R¹ =CH₂ ist; oder
R¹ und das Kohlenstoffatom, an den es gebunden ist, zusammen eine Cyclopropylgruppe bilden;
R⁴ und R⁵ gleich oder unterschiedlich und jeweils aus der aus H, C₁₋₁₂-Alkyl, Hydroxyl, C₁₋₁₂-Alkoxy und Halogen bestehenden Gruppe ausgewählt sind;
worin das C₁₋₁₂-Alkyl und das C₁₋₁₂-Alkoxy gegebenenfalls und jeweils unabhängig mit einem oder mehreren aus der aus Alkyl, Alkenyl, Alkinyl, Cycloalkyl, Aroyl, Halogen, Monohalogenalkyl, Dihalogenalkyl, Trihalogenalkyl, Monohalogenalkoxy, Dihalogenalkoxy, Trihalogenalkoxy, Hydroxy, Alkoxy, Alkoxycarbonyl, Cycloalkyloxy, Heterocyclooxy, Oxo, Alkanoyl, Aryl, Arylalkyl, Alkylaryl, Heteroaryl, Heteroarylalkyl, Alkylheteroaryl, Heterocyclyl, Aryloxy, Alkanoyloxy, Amino, Monoalkylamino, Dialkylamino, Trialkylamino, Arylamino, Arylalkylamino, Cycloalkylamino, Heterocycloamino, Alkanoylamino, Aroylamino, Aralkanoylamino, Mercapto, Alkylthio, Arylthio, Arylalkylthio, Cycloalkylthio, Heterocyclothio, Alkylthio, Arylthiono, Arylalkylthiono, Alkylsulfonyl, Arylsulfonyl, Arylalkylsulfonyl, Sulfamato-NH₂SO₂O, Alkylsulfamato, Sulfonamido-SO₂NH₂, Alkylsulfonamido, Nitro, Cyano, Carboxy, Carbamyl NH₂CO-, aus Alkyl-NH-CO-, Aryl-NH-CO- und Arylalkyl-NH-CO- ausgewähltem monosubstituiertem Carbamyl und aus Dialkyl-N-CO-, Diaryl-N-CO- und Alkyl-aryl-N-CO- ausgewähltem disubstituiertem Carbamyl bestehenden Gruppe ausgewählten Substituenten substituiert sind;
R⁶ C₁₋₁₂-Alkyl oder Aryl ist;
worin das C₁₋₁₂-Alkyl gegebenenfalls und jeweils unabhängig mit einem oder mehreren aus der aus Alkyl, Alkenyl, Alkinyl, Cycloalkyl, Aroyl, Halogen, Monohalogenalkyl, Dihalogenalkyl, Trihalogenalkyl, Monohalogenalkoxy, Dihalogenalkoxy, Trihalogenalkoxy, Hydroxy, Alkoxy, Alkoxycarbonyl, Cycloalkyloxy, Heterocyclooxy, Oxo, Alkanoyl, Aryl, Arylalkyl, Alkylaryl, Heteroaryl, Heteroarylalkyl, Alkylheteroaryl, Heterocyclyl, Aryloxy, Alkanoyloxy, Amino, Monoalkylamino, Dialkylamino, Trialkylamino, Arylamino, Arylalkylamino, Cycloalkylamino, Heterocycloamino, Alkanoylamino, Aroylamino, Aralkanoylamino, Mercapto, Alkylthio, Arylthio, Arylalkylthio, Cycloalkylthio, Heterocyclothio, Alkylthio, Arylthiono, Arylalkylthiono, Alkylsulfonyl, Arylsulfonyl, Arylalkylsulfonyl, Sulfamato-NH₂SO₂O, Alkylsulfamato, Sulfonamido-SO₂NH₂, Alkylsulfonamido, Nitro, Cyano, Carboxy, Carbamyl NH₂CO-, aus Alkyl-NH-CO-, Aryl-NH-CO- und Arylalkyl-NH-CO- ausgewähltem monosubstituiertem Carbamyl und aus Dialkyl-N-CO-, Diaryl-N-CO- und Alkyl-aryl-N-CO- ausgewähltem disubstituiertem Carbamyl bestehenden Gruppe ausgewählten Substituenten substituiert ist; und
worin das Aryl gegebenenfalls jeweils unabhängig mit einem oder mehreren aus der aus Alkyl, Alkenyl, Alkinyl, Cycloalkyl, Aroyl, Halogen, Monohalogenalkyl, Dihalogenalkyl, Trihalogenalkyl, Monohalogenalkoxy, Dihalogenalkoxy, Trihalogenalkoxy, Hydroxy, Alkoxy, Alkoxycarbonyl, Cycloalkyloxy, Heterocyclooxy, Oxo, Alkanoyl, Aryl, Arylalkyl, Alkylaryl, Heteroaryl, Heteroarylalkyl, Alkylheteroaryl, Heterocyclyl, Aryloxy, Alkanoyloxy, Amino, Monoalkylamino, Dialkylamino, Trialkylamino, Arylamino, Arylalkylamino, Cycloalkylamino, Heterocycloamino, Alkanoylamino, Aroylamino, Aralkanoylamino, Mercapto, Alkylthio, Arylthio, Arylalkylthio, Cycloalkylthio, Heterocyclothio, Alkylthio, Arylthiono, Arylalkylthiono, Alkylsulfonyl, Arylsulfonyl, Arylalkylsulfonyl, Sulfamato NH₂SO₂O-, Alkylsulfamato, Sulfonamido -SO₂NH₂, Alkylsulfonamido, Nitro, Cyano, Carboxy, Carbamyl NH₂CO-, aus Alkyl-NH-CO-, Aryl-NH-CO- und Arylalkyl-NH-CO- ausgewähltem monosubstituiertem Carbamyl und aus Dialkyl-N-CO-, Diaryl-N-CO- und Alkyl-aryl-N-CO- ausgewähltem disubstituiertem Carbamyl bestehenden Gruppe ausgewählten Substituenten substituiert ist;
X Sauerstoff oder Schwefel ist; und
a = 0 bis 5 ist;
mit der Maßgabe, dass, wenn a = 1 bis 5 ist, R⁶ gegebenenfalls substituiertes Aryl ist, wobei die optionalen Substituenten von Aryl wie oben definiert sind;
und dass, wenn a = 0 ist, R⁶ gegebenenfalls substituiertes C₁₋₁₂-Alkyl ist, wobei die optionalen Substituenten von C₁₋₁₂-Alkyl wie oben definiert sind;
oder ein pharmazeutisch annehmbares Salz davon.

2. Verbindung oder pharmazeutisch annehmbares Salz davon nach Anspruch 1, worin X Sauerstoff ist.

3. Verbindung oder pharmazeutisch annehmbares Salz davon nach Anspruch 1, worin X Schwefel ist.

4. Verbindung oder pharmazeutisch annehmbares Salz davon nach einem der Ansprüche 1 bis 3, worin R¹ =CH₂ ist.

5. Verbindung oder pharmazeutisch annehmbares Salz davon nach einem der Ansprüche 1 bis 3, worin R¹ Cyclopropyl ist.

6. Verbindung oder pharmazeutisch annehmbares Salz davon nach einem der Ansprüche 1 bis 5, worin R⁴ und R⁵ jeweils H sind.

7. Verbindung oder pharmazeutisch annehmbares Salz davon nach einem der Ansprüche 1 bis 6, worin a = 0 ist und R⁶ aus einem oder mehreren von (i) gegebenenfalls substituiertem C₁₋₁₂-Alkyl, und (ii) C₁₋₁₂-Alkyl oder Hydroxy-C₁₋₁₂-alkyl ausgewählt ist, wobei die optionalen Substituenten wie oben definiert sind.

8. Verbindung oder pharmazeutisch annehmbares Salz davon nach Anspruch 7, worin R⁶ Isopentyl, 3-Hydroxy-3-methylbutyl, 2,3-Dimethylbutyl, 2,3-Dimethyl-3-hydroxybutyl, 3-Ethylpentyl, 3-Ethyl-3-hydroxypentyl, 4-Ethylhexyl oder 4-Ethyl-4-hydroxyhexyl ist.

9. Verbindung oder pharmazeutisch annehmbares Salz davon nach einem der Ansprüche 1 bis 6, worin a = 1 bis 5 ist und R⁶ aus einem oder mehreren von (i) gegebenenfalls substituiertem Aryl; und (ii) mit einem C₁₋₁₂-Alkyl oder einem C₁₋₁₂-Hydroxyalkyl substituiertem Aryl ausgewählt ist, wobei die optionalen Substituenten wie oben definiert sind.

10. Verbindung oder pharmazeutisch annehmbares Salz davon nach Anspruch 9, worin R⁶ mit Isopropyl oder 2-Hydroxypropan-2-yl substituiertes Aryl ist.

11. Verbindung oder pharmazeutisch annehmbares Salz davon nach einem der Ansprüche 1 bis 6, 9 und 10, worin a = 1 ist.

12. Verbindung oder pharmazeutisch annehmbares Salz davon nach Anspruch 1, worin die Verbindung aus der aus den folgenden bestehenden Gruppe ausgewählt ist:
(1R,3R)-5-((E)-2-((3αS,7αS)-1-((R)-1-((S)-3-Hydroxy-2,3-dimethylbutoxy)ethyl)-7α-methyldihydro-1H-inden-4-(2H,5H,6H,7H,7αH)-yliden)ethyliden)-2-methylen-cyclohexan-1,3-diol (Vida-5);
(4R,8R)-6-((E)-2-((1 S,7αS)-1-((R)-1-(3-Ethyl-3-hydroxypentyloxy)ethyl)-7α-methyldihydro-1H-inden-4-(2H,5H,6H,7H,7αH)-yliden)ethyliden)spiro[2.5]octan-4,8-diol (Vida-10);
(4R,8R)-6-((E)-2-((1 S,7αS)-1-((R)-1-(4-Ethyl-4-hydroxyhexyloxy)ethyl)-7α-methyldihydro-1H-inden-4-(2H,5H,6H,7H,7αH)-yliden)ethyliden)spiro[2.5]octan-4,8-diol (Vida-20);
(1 R,3R)-5-((E)-2-((1S,3αS,7αS)-1-((R)-1-(3-Ethyl-3-hydroxypentyloxy)ethyl)-7α-methyldihydro-1H-inden-4-(2H,5H,6H,7H,7αH)-yliden)ethyliden)-2-methylencyclohexan-1,3-diol (Vida-11);
(1 R,3R)-5-((E)-2-((1S,7αS)-1-((R)-1-(4-Ethyl-4-hydroxypentyloxy)ethyl)-7α-methyldihydro-1H-inden-4-(2H,5H,6H,7H,7aH)-yliden)ethyliden)-2-methylencyclohexan-1,3-diol (Vida-21);
(4R,8R)-6-((E)-2-((1S,7αS)-1-((R)-1-(3-Hydroxy-3-methylbutoxy)ethyl)-7α-methyldihydro-1H-inden-4-(2H,5H,6H,7H,7αH)-yliden)ethyliden)spiro[2.5]octan-4,8-diol (Vida-1);
(4R,8R)-6-((E)-2-((3αS,7αS)-1-((R)-1-((S)-3-Hydroxy-2,3-dimethylbutoxy)ethyl)-7α-methyldihydro-1H-inden-4-(2H,5H,6H,7H,7αH)-yliden)ethyliden)spiro[2.5]octan-4,8-diol (Vida-4);
(4R,8R)-6-((E)-2-(1-((R)-1-(3-(2-Hydroxypropan-2-yl)phenoxy)propan-2-yl)-7a-methyldihydro-1H-inden-4-(2H,5H,6H,7H,7αH)-yliden)ethyliden)spiro[2.5]octan-4,8-diol (Vida-57);
(4R,8R)-6-((E)-2-(1-((R)-1-(3-(2-Hydroxypropan-2-yl)phenylthio)propan-2-yl)-7α-methyldihydro-1H-inden-4-(2H,5H,6H,7H,7αH)-yliden)ethyliden)spiro[2.5]octan-4,8-diol (Vida-58);
(1 R,3R)-5-((E)-2-(1-((R)-1-(3-(2-Hydroxypropan-2-yl)phenoxy)propan-2-yl)-7α-methyldihydro-1H-inden-4-(2H,5H,6H,7H,7αH)-yliden)ethyliden)-2-methylencyclohexan-1,3-diol (Vida-37);
(1R,3R)-5-((E)-2-(1-((R)-1-(3-(2-Hydroxypropan-2-yl)phenylthio)propan-2-yl)-7α-methyldihydro-1H-inden-4-(2H,5H,6H,7H,7αH)-yliden)ethyliden)-2-methylencyclohexan-1,3-diol (Vida-43);
(4R,8R)-6-((E)-2-(1S,7αS)-1-((R)-1-(3-Methylbutoxy)ethyl)-7α-methyldihydro-1H-inden-4-(2H,5H,6H,7H,7aH)-yliden)ethyliden)spiro[2.5]octan-4,8-diol (Vida-81);
(4R,8R)-6-((E)-2-(3αS,7αS)-1-((R)-1-((S)-2,3-Dimethylbutoxy)ethyl)-7α-methyldihydro-1H-inden-4-(2H,5H,6H,7H,7αH)-yliden)ethyliden)spiro[2.5]octan-4,8-diol (Vida-84);
(1R,3R)-5-((E)-2-((3αS,7αS)-1-((R)-1-((S)-2,3-Dimethylbutoxy)ethyl)-7α-methyldihydro-1H-inden-4-(2H,5H,6H,7H,7aH)-yliden)ethyliden)-2-methylencyclohexan-1,3-diol (Vida-85);
(4R,8R)-6-((E)-2-(1S,7αS)-1-((R)-1-(3-Ethylpentyloxy)ethyl)-7α-methyldihydro-1H-inden-4-(2H,5H,6H,7H,7aH)-yliden)ethyliden)spiro[2.5]octan-4,8-diol (Vida-90);
(1 R,3R)-5-((E)-2-(1S,3αS,7αS)-1-((R)-1-(3-Ethylpentyloxy)ethyl)-7α-methyldihydro-1H-inden-4-(2H,5H,6H,7H,7aH)-yliden)ethyliden)-2-methylencyclohexan-1,3-diol (Vida-91);
(4R,8R)-6-((E)-2-(1S,7αS)-1-((R)-1-(4-Ethylhexyloxy)ethyl)-7α-methyldihydro-1H-inden-4-(2H,5H,6H,7H,7αH)-yliden)ethyliden)spiro[2.5]octan-4,8-diol (Vida-100);
(1 R,3R)-5-((E)-2-(1S,7αS)-1-((R)-1-(4-Ethylhexyloxy)ethyl)-7α-methyldihydro-1H-inden-4-(2H,5H,6H,7H,7αH)-yliden)ethyliden)-2-methylencyclohexan-1,3-diol (Vida-101);
(1R,3R)-5-((E)-2-(1-((R)-1-(3-Isopropylphenoxy)propan-2-yl)-7α-methyldihydro-1H-inden-4-(2H,5H,6H,7H,7αH)-yliden)ethyliden)-2-methylencyclohexan-1,3-diol (Vida-117);
(1R,3R)-5-((E)-2-(1-((R)-1-(3-Isopropylphenylthio)propan-2-yl)-7α-methyldihydro-1H-inden-4-(2H,5H,6H,7H,7αH)-yliden)ethyliden)-2-methylencyclohexan-1,3-diol (Vida-123);
(4R,8R)-6-((E)-2-(1-((R)-1-(3-Isopropylphenoxy)propan-2-yl)-7α-methyldihydro-1H-inden-4-(2H,5H,6H,7H,7αH)-yliden)ethyliden)spiro[2.5]octan-4,8-diol (Vida-137); und
(4R,8R)-6-((E)-2-(1-((R)-1-(3-Isopropylphenylthio)propan-2-yl)-7α-methyldihydro-1H-inden-4-(2H,5H,6H,7H,7αH)-yliden)ethyliden)spiro[2.5]octan-4,8-diol (Vida-138).

13. Pharmazeutische Zusammensetzung, die Folgendes umfasst: (i) eine Verbindung oder ein pharmazeutisch annehmbares Salz davon nach einem der Ansprüche 1 bis 12 und (ii) einen pharmazeutisch annehmbaren Träger.

14. Verbindung der Formel (I) oder ein pharmazeutisch annehmbares Salz davon nach einem der Ansprüche 1 bis 12 zur Verwendung in einem Verfahren zur Behandlung einer Erkrankung, die sich durch eine Modulierung des Vitamin-D-Rezeptors verbessert.

15. Verbindung der Formel (I) oder ein pharmazeutisch annehmbares Salz davon nach einem der Ansprüche 1 bis 12 zur Verwendung in einem medizinischen Behandlungsverfahren für eine Erkrankung, die aus der aus Hypokalzämie, Krebs, Psoriasis, Hyperparathyreoidismus, Osteoporose, sekundärem Hyperparathyreoidismus, Proteinurie/Niederkrankheitsprogression, Endothelfehlfunktion, linksventrikulärer Hypertrophie, Alterung, metabolischem Syndrom, Insulinresistenz, Adipositas, Vireninfektion, Bakterieninfektion, Muskel-Skelett-Störungen, hoher Blutdruck, Hypertriglyceridämie, Immunstörungen, multipler Sklerose, myelodysplastischem Syndrom, proximaler Myopathie, Winterdepression, Alterswarzen, Hauptpigmentstörungen und Thrombose bestehenden Gruppe ausgewählt ist.

## Revendications

1. Composé de formule (I) 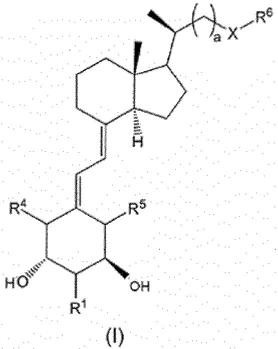 dans laquelle
R¹ est =CH₂ ; ou
R¹ et le carbone auquel il est lié forment ensemble un groupe cyclopropyle ;
R⁴ et R⁵ sont identiques ou différents, et chacun est choisi dans le groupe consistant en H, les groupes alkyle en C₁₋₁₂, hydroxyle, alcoxy en C₁₋₁₂ et halogéno ;
où lesdits groupes alkyle en C₁-C₁₂ et alcoxy en C₁-C₁₂ sont en option et indépendamment substitués par un ou plusieurs substituants choisis dans le groupe consistant en les groupes alkyle, alcényle, alcynyle, cycloalkyle, aroyle, halogéno, monohalogènalkyle, dihalogènalkyle, trihalogènalkyle, monohalogènalcoxy, dihalogènalcoxy, trihalogènalcoxy, hydroxy, alcoxy, alcoxycarbonyle, cycloalkyloxy, hétérocyclooxy, oxo, alcanoyle, aryle, arylalkyle, alkylaryle, hétéroaryle, hétéroarylalkyle, alkylhétéroaryle, hétérocyclyle, aryloxy, alcanoyloxy, amino, monoalkylamino, dialkylamino, trialkylamino, arylamino, arylalkylamino, cycloalkylamino, hétérocycloamino, alcanoylamino, aroylamino, aralcanoylamino, mercapto, alkylthio, arylthio, arylalkylthio, cycloalkylthio, hétérocyclothio, alkylthiono, arylthiono, arylalkylthiono, alkylsulfonyle, arylsulfonyle, arylalkylsulfonyle, sulfamato, NH₂SO₂O-, alkylsulfamato, sulfonamido -SO₂NH₂, alkylsulfonamido, nitro, cyano, carboxy, carbamyle NH₂CO-, carbamyle monosubstitué choisi parmi les groupes alkyl-NH-CO, aryl-NH-CO et arylalkyl-NH-CO et les groupes carbamyle disubstitués choisis parmi les groupes dialkyl-N-CO, diaryl-N-CO et alkyl-aryl-N-CO ;
R⁶ est un groupe alkyle en C₁₋₁₂ ou aryle :
où ledit groupe alkyle en C₁-C₁₂ est en option et indépendamment substitué par un ou plusieurs substituants choisis dans le groupe consistant en les groupes alkyle, alcényle, alcynyle, cycloalkyle, aroyle, halogéno, monohalogènalkyle, dihalogènalkyle, trihalogènalkyle, monohalogènalcoxy, dihalogènalcoxy, trihalogènalcoxy, hydroxy, alcoxy, alcoxycarbonyle, cycloalkyloxy, hétérocyclooxy, oxo, alcanoyle, aryle, arylalkyle, alkylaryle, hétéroaryle, hétéroarylalkyle, alkylhétéroaryle, hétérocyclyle, aryloxy, alcanoyloxy, amino, monoalkylamino, dialkylamino, trialkylamino, arylamino, arylalkylamino, cycloalkylamino, hétérocycloamino, alcanoylamino, aroylamino, aralcanoylamino, mercapto, alkylthio, arylthio, arylalkylthio, cycloalkylthio, hétérocyclothio, alkylthiono, arylthiono, arylalkylthiono, alkylsulfonyle, arylsulfonyle, arylalkylsulfonyle, sulfamato, NH₂SO₂O-, alkylsulfamato, sulfonamido -SO₂NH₂, alkylsulfonamido, nitro, cyano, carboxy, carbamyle NH₂CO-, carbamyle monosubstitué choisi parmi les groupes alkyl-NH-CO, aryl-NH-CO et arylalkyl-NH-CO et les groupes carbamyle disubstitués choisis parmi les groupes dialkyl-N-CO, diaryl-N-CO et alkyl-aryl-N-CO ; et
où ledit groupe aryle est en option indépendamment substitué par un ou plusieurs substituants choisis dans le groupe consistant en les groupes alkyle, alcényle, alcynyle, cycloalkyle, aroyle, halogéno, monohalogènalkyle, dihalogènalkyle ou trihalogènalkyle, monohalogènalcoxy, dihalogènalcoxy ou trihalogènalcoxy, hydroxy, alcoxy, alcoxycarbonyle, cycloalkyloxy, hétérocyclooxy, oxo, alcanoyle, arylalkyle, alkylaryle, hétéroaryle, hétéroarylalkyle, alkylhétéroaryle, hétérocyclyle, aryloxy, alcanoyloxy, amino, monoalkylamino, dialkylamino ou trialkylamino, arylamino, arylalkylamino, cycloalkylamino, hétérocycloamino, alcanoylamino, aroylamino, aralcanoylamino, mercapto, alkylthio, arylthio, arylalkylthio, cycloalkylthio, hétérocyclothio, alkylthiono, arylthiono, arylalkylthiono, alkylsulfonyle, arylsulfonyle, arylalkylsulfonyle, sulfamato, NH₂SO₂O-, alkylsulfamato, sulfonamido -SO₂NH₂, alkylsulfonamido, nitro, cyano, carboxy, carbamyle NH₂CO-, et carbamyle monosubstitué choisi parmi les groupes alkyl-NH-CO, aryl-NH-CO et arylalkyl-NH-CO et les groupes carbamyle disubstitués choisis parmi les groupes dialkyl-N-CO, diaryl-N-CO et alkyl-aryl-N-CO ;
X est un atome d'oxygène ou de soufre ; et
a vaut 0-5 ;
à la condition que, quand a vaut 1-5, alors R⁶ soit un groupe aryle en option substitué, les substituants en option du groupe aryle étant tels que définis ci-dessus ; et que, quand a vaut 0, R⁶ soit un groupe alkyle en C₁₋₁₂ en option substitué, les substituants en option du groupe alkyle en C₁₋₁₂ étant tels que définis ci-dessus ;
ou un sel pharmaceutiquement acceptable de ce composé.

2. Composé ou sel pharmaceutiquement acceptable de celui-ci selon la revendication 1, dans lequel X est un atome d'oxygène.

3. Composé ou sel pharmaceutiquement acceptable de celui-ci selon la revendication 1, dans lequel X est un atome de soufre.

4. Composé ou sel pharmaceutiquement acceptable de ce dernier selon l'une quelconque des revendications 1 à 3, dans lequel R¹ est =CH₂.

5. Composé ou sel pharmaceutiquement acceptable de celui-ci selon l'une quelconque des revendications 1 à 3, dans lequel R¹ est le groupe cyclopropyle.

6. Composé ou sel pharmaceutiquement acceptable de celui-ci selon l'une quelconque des revendications 1 à 5, dans lequel R⁴ et R⁵ sont chacun H.

7. Composé ou sel pharmaceutiquement acceptable de celui-ci selon l'une quelconque des revendications 1 à 6, dans lequel a vaut 0, et R⁶ est choisi parmi un ou plusieurs de (i) groupes alkyle en C₁₋₁₂ en option substitués, et (ii) groupes alkyle en C₁₋₁₂ ou hydroxyalkyle en C₁₋₁₂, les substituants en option étant tels que définis ci-dessus.

8. Composé ou sel pharmaceutiquement acceptable de celui-ci selon la revendication 7, dans lequel R⁶ est un groupe isopentyle, 3-hydroxy-3-méthylbutyle, 2,3-diméthylbutyle, 2,3-diméthyl-3-hydroxybutyle, 3-éthylpentyle, 3-éthyl-3-hydroxypentyle, 4-éthylhexyle ou 4-éthyl-4-hydroxyhexyle.

9. Composé ou sel pharmaceutiquement acceptable de celui-ci selon l'une quelconque des revendications 1 à 6, dans lequel a vaut 1 à 5, et R⁶ est choisi parmi un ou plusieurs de (i) groupes aryle en option substitués ; et (ii) groupes aryle substitués par un groupe alkyle en C₁₋₁₂ ou hydroxyalkyle en C₁₋₁₂, les substituants en option étant tels que définis ci-dessus.

10. Composé ou sel pharmaceutiquement acceptable de celui-ci selon la revendication 9, dans lequel R⁶ est un groupe aryle substitué par un groupe isopropyle ou 2-hydroxypropan-2-yle.

11. Composé ou sel pharmaceutiquement acceptable de celui-ci selon l'une quelconque des revendications 1 à 6, 9 et 10, dans lequel a vaut 1.

12. Composé ou sel pharmaceutiquement acceptable de celui-ci selon la revendication 1, le composé étant choisi dans le groupe consistant en
(1R,3R)-5-((E)-2-((3aS,7αS)-1-((R)-1-((S)-3-hydroxy-2,33-diméthylbutoxy)éthyl)-7α-méthyldihydro-1H-indène-4(2H,5H,6H,7H,7αH)-ylidène)éthylidène)-2-méthylènecyclohexane-1,3-diol (Vida-5) ;
(4R,8R)-6-((E)-2-((1S,7αS)-1-((R)-1-(3-éthyl-3-hydroxypentyloxy)éthyl)-7α-méthyldihydro-1H-indène-4(2H,5H,6H,7H,7αH)-ylidène)éthylidène)spiro[2.5]octane-4,8-diol (Vida-10) ;
(4R,8R)-6-((E)-2-((1S,7αS)-1-((R)-1-(4-éthyl-4-hydroxyhexyloxy)éthyl)-7α-méthyldihydro-1H-indène-4(2H,5H,6H,7H,7αH)-ylidène)éthylidène)spiro[2.5]octane-4,8-diol (Vida-20) ;
(1R,3R)-5-((E)-2-((1S,3aS,7αS)-1-((R)-1-(3-éthyl-3-hydroxypentyloxy)éthyl)-7α-méthyldihydro-1H-indène-4(2H,5H,6H,7H,7αH)-ylidène)éthylidène)-2-méthylènecyclohexane-1,3-diol (Vida-11) ;
(1R,3R)-5-((E)-2-((1S,7αS)-1-((R)-1-(4-éthyl-4-hydroxyhexyloxy)éthyl)-7α-méthyldihydro-1H-indène-4(2H,5H,6H,7H,7α/H)-ylidène)éthylidène)-2-méthylènecyclohexane-1,3-diol (Vida-21) ;
(4R,8R)-6-((E)-2-((1S,7αS)-1-((R)-1-(3-hydroxy-3-méthylbutoxy)éthyl)-7α-méthyldihydro-1H-indène-4(2H,5H,6H,7H,7αH)-ylidène)éthylidène)spiro[2.5]octane-4,8-diol (Vida-1) ;
(4R,8R)-6-((E)-2-((3aS,7αS)-1-((R)-1-((S)-3-hydroxy-2,3-diméthylbutoxy)éthyl)-7α-méthyldihydro-1H-indène-4(2H,5H,6H,7H,7αH)-ylidène)éthylidène)spiro[2.5]octane-4,8-diol (Vida-4) ;
(4R,8R)-6-((E)-2-(1-((R)-1-(3-(2-hydroxypropan-2-yl)phénoxy)propan-2-yl)-7α-méthyldihydro-1H-indène-4(2H,5H,6H,7H,7αH)-ylidène)éthylidène)spiro[2.5]octane-4,8-diol (Vida-57) ;
(4R,8R)-6-((E)-2-(1-((R)-1-(3-(2-hydroxypropan-2-yl)phénylthio)propan-2-yl)-7α-méthyldihydro-1H-indène-4(2H,5H,6H,7H,7αH)-ylidène)éthylidène)spiro[2.5]octane-4,8-diol (Vida-58) ;
(1R,3R)-5-((E)-2-(1-((R)-1-(3-(2-hydroxypropan-2-yl)-7α-méthyldihydro-1H-indène-4(2H,5H,6H,7H,7αH)-ylidène)éthylidène)-2-méthylènecyclohexane-1,3-diol (Vida-37) ;
(1R,3R)-5-((E)-2-(1-((R)-1-(3-(2-hydroxypropan-2-yl)phénylthio)propan-2-yl)-7α-méthyldihydro-1H-indène-4(2H,5H,6H,7H,7αH)-ylidène)éthylidène)-2-méthylènecyclohexane-1,3-diol (Vida-43) ;
(4R,8R)-6-((E)-2-((1S,7αS)-1-((R)-1-(3-méthylbutoxy)éthyl)-7α-méthyldihydro-1H-indène-4(2H,5H,6H,7H,7αH)-ylidène)éthylidène)spiro[2.5]octane-4,8-diol (Vida-81) ;
(4R,8R)-6-((E)-2-((3aS,7αS)-1-((R)-1-((S)-2,3-diméthylbutoxy)éthyl)-7α-méthyldihydro-1H-indène-4(2H,5H,6H,7H,7αH)-ylidène)éthylidène)spiro[2.5]octane-4,8-diol (Vida-84) ;
(1R,3R)-5-((E)-2-((3aS,7αS)-1-((R)-1-((S)-2,3-diméthylbutoxy)éthyl)-7α-méthyldihydro-1H-indène-4(2H,5H,6H,7H,7αH)-ylidène)éthylidène)-2-méthylènecyclohexane-1,3-diol (Vida-85) ;
(4R,8R)-6-((E)-2-((1S,7αS)-1-((R)-1-(3-éthylpentyloxy)éthyl)-7α-méthyldihydro-1H-indène-4(2H,5H,6H,7H,7αH)-ylidène)éthylidène)spiro[2.5]octane-4,8-diol (Vida-90) ;
(1R,3R)-5-((E)-2-((1S,3aS,7αS)-1-((R)-1-(3-éthylpentyloxy)éthyl)-7α-méthyldihydro-1H-indène-4(2H,5H,6H,7H,7αH)-ylidène)éthylidène)-2-méthylènecyclohexane-1,3-diol (Vida-91) ;
(4R,8R)-6-((E)-2-((1S,7αS)-1-((R)-1-(4-éthylhexyloxy)éthyl)-7α-méthyldihydro-1H-indène-4(2H,5H,6H,7H,7αH)-ylidène)éthylidène)spiro[2.5]octane-4,8-diol (Vida-100) ;
(1R,3R)-5-((E)-2-((1S,7αS)-1-((R)-1-(4-éthylhexyloxy)éthyl)-7α-méthyldihydro-1H-indène-4(2H,5H,6H,7H,7αH)-ylidène)éthylidène)-2-méthylènecyclohexane-1,3-diol (Vida-101) ;
(1R,3R)-5-((E)-2-(1-((R)-1-(3-isopropylphénoxy)propan-2-yl)-7α-méthyldihydro-1H-indène-4(2H,5H,6H,7H,7αH)-ylidène)éthylidène)-2-méthylènecyclohexane-1,3-diol (Vida-117) ;
(1R,3R)-5-((E)-2-(1-((R)-1-(3-isopropylphénylthio)propan-2-yl)-7α-méthyldihydro-1H-indène-4(2H,5H,6H,7H,7αH)-ylidène)éthylidène)-2-méthylènecyclohexane-1,3-diol (Vida-123) ;
(4R,8R)-6-((E)-2-(1-((R)-1-(3-isopropylphénoxy)propan-2-yl)-7α-méthyldihydro-1H-indène-4(2H,5H,6H,7H,7αH)-ylidène)éthylidène)spiro[2.5]octane-4,8-diol (Vida-137) ; et
(4R,8R)-6-((E)-2-(1-((R)-1-(3-isopropylphénylthio)propan-2-yl))-7α-méthyldihydro-1H-indène-4(2H,5H,6H,7H,7αH)-ylidène)éthylidène)spiro[2.5]octane-4,8-diol (Vida-138).

13. Composition pharmaceutique comprenant (i) un composé ou un sel pharmaceutiquement acceptable de celui-ci selon l'une quelconque des revendications 1 à 12, et (ii) un support pharmaceutiquement acceptable.

14. Composé de formule (I) ou sel pharmaceutiquement acceptable de celui-ci tel que revendiqué dans l'une quelconque des revendications 1 à 12, pour une utilisation dans un procédé de traitement d'une maladie qui tire avantage d'une modulation du récepteur de la vitamine D.

15. Composé de formule (I) ou sel pharmaceutiquement acceptable de celui-ci tel que revendiqué dans l'une quelconque des revendications 1 à 12 pour une utilisation dans un procédé de traitement médical d'une maladie choisie dans le groupe consistant en l'hypercalcémie, le cancer, le psoriasis, l'hyperparathyroïdie, l'ostéoporose, l'hyperparathyroïdie secondaire, la protéinurie/la progression de la maladie rénale, le dysfonctionnement endothélial, l'hypertrophie ventriculaire gauche, le vieillissement, le syndrome métabolique, la résistance à l'insuline, l'obésité, une infection virale, une infection bactérienne, les troubles musculo-squelettiques, l'hypertension, l'hypertriglycéridémie, les troubles immuns, la sclérose en plaques, le syndrome myélodysplasique, la myopathie proximale, le trouble affectif saisonnier, les verrues séniles, des troubles de la pigmentation de la peau, et la thrombose.
